# EUROPEAN PATENT APPLICATION

(11) **EP 2 014 772 A2**
(43) Date of publication of application: **14.01.2009**
(21) Application number: 08009585.4
(22) Date of filing: 03.04.1997
(51) Int. Cl.: C12Q 1/00, G01N 33/53, C07K 1/00

(54) **Method for the diagnosis and prognosis of cancer**

(30) Priority: 03.03.1997 US 39532 P; 21.06.1996 US 20196 P; 05.06.1996 US 19372 P; 05.04.1996 US 14943 P
(62) Divisional of application: 97920115.9
(71) Applicant: Giordano, Antonio, Philadelphia, Pennsylvania 19128 (US)
(72) Inventor: Giordano, Antonio, Philadelphia, Pennsylvania 19128 (US); Baldi, Alfonso, 80128 Naples (IT)
(74) Representative: Thomas, Dean

(57) **Abstract**

The invention provides diagnostic and prognostic methods which comprise determining the level of expression of the tumor suppressor gene pRb2/p130, because the relative level of pRb2/p130 expression correlates with the presence of cancer, tumor grade and patient prognosis. These methods may be used to detect cancer, to make treatment decisions, to predict patient outcome, and to predict the risk of cancer in disease-free individuals. The invention further provides methods for the detection of mutations and polymorphisms in the pRb2/p130 gene, which may be used to characterize genetic events associated with tumor formation, to trace the parental origin of mutations, to identify carriers of germline mutations and to identify individuals with a predisposition to cancer.

## Description

### Reference to Government Grant

The invention described herein was supported in part by National Institutes of Health grant RO1 CA60999-01A1. The U.S. government has certain rights in the invention.

### Cross-Reference to Related Applications

This application claims priority from U.S. provisional patent application No. 60/039,532 filed March 3, 1997, U.S. Provisional Application No. 60/020,196 filed June 21, 1996, U.S. Provisional Application No. 60/019,372 filed June 5, 1996 and U.S. Provisional Application No. 60/014,943 filed April 5, 1996.

### Field of the Invention

The invention relates to methods for the identification of individuals at risk for cancer, and for the detection and evaluation of cancers.

### Background of the Invention

### A. The Rb Family of Tumor Suppressors

Many types of human cancer are believed to be caused by an imbalance of growth regulators within a cell. A decrease in negative control growth regulators and/or their deactivation can cause a cancerous condition. Alternatively, an increase in positive control growth regulators can also cause a cancerous condition.

Since the identification of the first tumor suppressor gene, much effort in cancer research has been focused on the identification of new tumor suppressor genes and their involvement in human cancer. Many types of human cancers are thought to develop by a loss of heterozygosity of putative tumor suppressor genes not yet identified (Lasko et al., Annu. Rev. Genetics, 25. 281-296 (1991)) according to Knudson's "two-hit" hypothesis (Knudson, Proc. Natl. Acad. Sci. USA, 68, 820-823 (1971)).

One of the most studied tumor suppressor genes is the retinoblastoma susceptibility gene (Rb), whose gene product (pRb, p105, or pRb/p105) has been shown to play a key role in the regulation of cell division. In interphasic cells, pRb contributes to maintaining the quiescent state of the cell by repressing transcription of genes required for the cell cycle through interaction with transcription factors, such as E2F (Wagner et al., Nature, 352, 189-190 (1991); Nevins, Science, 258, 424-429 (1992); and Hiebert et al., Genes Develop., 6, 177-185 (1992)). The loss of this activity can induce cell transformation as evidenced by the reversion of the transformed phenotype in pRb cells after replacement of a functional pRb (Huang et al., Science 242 1563-1565 (1988); Bookstein et al., Science, 247: 712-715 (1990); and Sumegi et al., Cell Growth Differ., 1 247-250 (1990)).

Upon entrance into the cell cycle, pRb seems to be phosphorylated by cell cycle-dependent kinases (Lees et al., EMBO J. 10:4279-4290 (1991); Hu et al., Mol. Cell. Biol., 12:971-980 (1992); Hinds et al., Cell, 70:993-1006 (1992); and Matsushime et al., Nature, 35:295-300)) which is thought to permit its dissociation from transcription factors and, hence, the expression of genes required for progression through the cell cycle.

It has been found that the retinoblastoma protein family includes at least three members. Two other proteins, p107, and the recently cloned pRb2/p130. share regions of homology with pRb/p105, especially in two discontinuous domains which make up the "pocket region". Ewen et al., Cell 66: 1155-1164 (1993); Mayol et al., Oncogene 8: 1561-2566 (1993): Li et al., Genes Dev. 7: 2366-2377 (1993); and Hannon et al., Genes Dev. 7: 2378-2391 (1993). The pocket domain is required for binding with several viral transforming oncoproteins (Moran, Curr. Opin. Genet. Dev. 3: 63-70 (1993)).

The pRb2/p130 cDNA and putative amino acid sequence are set forth by Li *et al*. The p107 cDNA and putative amino acid sequence are set forth by Ewen *et al*. The entire disclosures of Li *et al*. and Ewen *et al*. are incorporated herein by reference.

It has been found that pRb2/p130, as well as p107 and pRb, act as negative regulators of cell cycle progression, blocking the cells in the G1 phase (Goodrich et al., Cell 67: 293-302 (1991); Zhu et al., Genes Dev. 7:1111-1125 (1993); Claudio et al., Cancer Res. 54:5556-5560 (1994); and Zhu et al., EMBO J. 14:1904-1913 (1995)). However, the three proteins exhibit different growth suppressive properties in selected cell lines, suggesting that although the different members of the retinoblastoma protein family may complement each other, they are not fully functionally redundant (Claudio *et al*., *supra*).

The mechanisms by which these three proteins exert their control on cell cycle progression are not fully understood but likely include complex formation and modulation of the activity of several transcription factors (Sang et al., Mol. Cell. Differ. 3:1-29 (1995)). The most studied of these complexes is the one with the E2F family of transcription factors. E2F's are heterodimeric transcription factors composed of E2F-like and DP-like subunits that regulate the expression of genes required for progression through G₀/G₁ S phase of the cell cycle (Lan Thangue, N.B., Trends Biochem. Sci. 19:108-114 (1994)).

The three proteins bind and modulate the activity of distinct E2F/DP1 complexes in different phases of the cell cycle (Sang *et al*., *supra*; Chellapan et al., Cell 65:1053-1061 (1991); Shirodkar et at., Cell 66:157-166 (1992); Cobrinik et al., Genes Dev. 7:2392-2404 (1993), Hijmans et al., Mol. Cell. Biol. 15:3082-3089 (1995); and Vairo et al., Genes Dev. 9:869-881 (1995)). This suggests distinct roles for these related proteins in the regulation of the cell cycle.

It has been demonstrated that the growth suppressive properties of pRb2/p130 are specific for the G1 phase. D-type cyclins, as well as transcription factor E2F-1 and E1A viral oncoproteins, were able to rescue pRb2/p130-mediated G1-growth arrest in tumor cells. This suggests that, like other Rb family proteins, the phosphorylation of pRb2/p 130 is controlled by the cell cycle machinery, and that pRb2/p130 may indeed be another key G1-S phase regulator. Claudio et al., Cancer Res. 56, 2003-2008 (1996).

The association of pRb with transcription factors, such as E2F. has been shown to occur by interactions at a region known as the "pocket region" (Raychaudhuri et al., Genes Develop., 5 1200-1207 (1991)). Recently, p 107 has also been shown to exert such a binding profile (Cao et al., Nature, 355 176-179 (1992)). Domains A and B, along with a spacer, are believed to correspond with the "pocket region" in the pRb2/p130 gene described herein. Moreover, mutations have been found in the pocket region for several human cancers where a lack of function for the pRb protein is thought to be involved in the acquisition of the transformed phenotype (Hu et al., EMBO J., 9 1147-1153 (1990); Huang et al., Mol. Cell. Biol., 10: 3761-3769 (1990)).

The Rb, p107, and pRb2/p130 proteins may play a key role in cell cycle regulation in that all three proteins interact with several cyclin/cdk complexes. pRb can be regulated by cyclin/cdk complexes, such as cyclin A/cdk2, cyclin E/cdk2 and cyclin D/cdk4, even if stable interaction between pRb and cyclin A/cdk2 or cyclin A/cdk2 has not been found *in vivo* (MacLachlan et al., Eukaryotic Gene Exp. 5: 127-156 (1995)). On the other hand, both p107 and pRb2/p130 stably interact *in vivo* with cyclin E/cdk2 and cyclin A/cdk2 complexes (Li *et al*., *supra*; Ewen et al., Science 255:85-87 (1992); and Faha et al., Science 255:87-90 (1992)). These complexes may be responsible for the existence of different phosphorylated forms of pRb. p107 and pRb2/p130 in the various phases of the cell cycle (Chen et al., Cell 58:1193-1198 (1989); De Caprio et al., Proc., Natl. Acad. Sci. USA 89: 1795-1798 (1992): and Beijersbergen et al., Genes Dev. 9:1340-1353 (1993)). In that pRb's functional activities are enhanced by these phosphorylations, it is likely that pRb2/p130 is also affected in the same manner by this post-translational modification. Since pRb2/p130 demonstrates similar, even if not redundant. functional properties to pRb, it is proposed that pRb2/p130 acts, like pRb, as a tumor suppressor gene. It has also been found that pRb2/p130 maps on the long arm of chromosome 16. This finding reinforces the notion of pRb2/p130 as a tumor suppressor gene. Chromosome 16 is a region frequently reported to show loss of heterozygosity (LOH) in several human neoplasias, such as breast, ovarian, hepatocellular and prostatic carcinomas (Yeung et al., Oncogene 8:3465-3468 (1993)). Chromosome 16, and specifically pRb2/p130, has also been implicated in a rare human skin disease known as hereditary cylindromatosis (HR). HR has been reported as mapping to loci on chromosome 16q12-q13. In that the pRb2/p130 gene maps to chromosome 16q12-q13, it has been put forth as a likely candidate for the tumor suppressor gene involved with the onset of this disease. Biggs et al., Nature Genetics 11:441-443 (December 1995).

There is a need for improved methods for identification of individuals at risk for cancer, and for the detection and evaluation of cancers.

Because the pRb2/p130 gene is a tumor suppressor gene and because it maps to a chromosomal region known to be associated with various carcinomas, there is a need for a method to screen individuals for mutations in this gene. There is also a need to identify sequence polymorphisms in this gene. It is believed that mutations, both within the exon coding sequences and the exon-intron junctions, can occur that will affect pRb2/p130's function. Direct DNA sequence analysis of individual exons taken from genomic DNA extracted from tumors has been used successfully to identify mutations of the p53 gene in ovarian carcinomas and the Rb gene in retinoblastoma rumors. Milner et al., Cancer Research 53: 2128-2132 (1993); Yandell et al., N.E.J. Medicine 321:1689-1695 (1989). However, direct sequencing of exons is an undesirable approach because it is a time intensive process. An understanding of the genomic structure of the pRb2/p130 gene will enable those skilled in the art to screen a patient's DNA for polymorphisms and sequence mutations in the pRb2/p130 gene. Identification of sequence mutations will also enable the diagnosis of carriers of germline mutations of the pRb2/p130 gene and enable prenatal screening in these cases.

### B. Gynecologic Cancers

Gynecologic cancers include cancers of the uterus, ovary, cervix, vagina, vulva, and fallopian tube as well as gestational trophoblastic disease. Cancers of the uterus include endometrial carcinomas and uterine sarcomas.

Endometrial cancer is the most common malignancy of the female genital tract. Although this neoplasm is frequently diagnosed at an early stage (75 percent in stage I), approximately 20 percent of the patients will die of the disease, half of which were diagnosed at stage I (Pettersson, Annual Report On The Results Of Treatment In Gynecological Cancer, Radiumhemmet, Stockholm, vol. 22: 65-82; Braly, Gynecol Oncol 58: 145-7 (1995)). The ability to identify patients with a more aggressive disease is crucial to planning an adequate treatment for each case. With this purpose in mind, several pathologic tumor features have been considered so far, including histologic type, grade of differentiation, depth of myometrial invasion, lymph nodal metastases and extra-uterine spread (MacMahon, Gynecol Oncol 2: 122 (1974); Chambers et al., Gynecol Oncol 27: 180-8 (1987)). Unfortunately, none of these factors allows a sufficiently accurate stratification of the patients. Such parameters have also questionable reproducibility.

There is great need for a simple laboratory test which is a consistent predictor of clinical outcome in endometrial cancer. What is needed is a prognostic method which can, at an early disease stage, identify the aggressiveness of an individual patient's disease, before initiation of therapy.

Ovarian cancer is the leading cause of gynecologic cancer death in the United States. Most ovarian malignancies are epithelial carcinomas, with a minority of tumors arising from the germ or stromal cells. In ovarian cancers, the degree of cellular differentiation (histologic grade) is an important independent predictor of both response to treatment and overall survival. Ovarian cancers frequently exhibit chromosomal alterations. The pRb2/p130 gene maps to human chromosome 16q12.2, which is one region that is frequently altered in human ovarian cancers. There is a need for improved methods of grading ovarian tumors. The improved methods would be useful in the diagnosis of disease, in selection of treatment, and as prognostic indicators.

### C. Lung Cancer

Lung cancer is the greatest single cause of cancer-related deaths in Western countries. Selecting an appropriate course of therapy for lung cancer requires an accurate determination of the cancer's malignant potential. This determination is typically made by "grading" the tumor. The grading of tumors is typically carried out by examination of the character and appearance of tumor sections by skilled pathologists. A significant problem in the use of histologic criteria when determining the prognosis and types of treatment for lung cancer is the degree of interobserver and intraobserver variability in reading the same specimens. Determinations are necessarily subjective. In addition, there is heterogeneity within the tumor itself in both primary and metastatic sites. It may become necessary to obtain the opinion of several pathologists to reach a consensus on individual tumor grade.

There is a need for a simple laboratory test which is more consistently predicative of the malignant potential of an individual patient's lung tumor than the present subjective pathological analysis of tumor samples.

Detection of latent cancers before the appearance of lung lesions would allow therapeutic intervention at the earliest stages of the disease, thereby maximizing the prospects for a positive therapeutic outcome. It would also be desirable, through a simple genetic test, to identify disease free individuals who are at risk of lung cancer. Such a screening test would be most advantageous for those individuals who, through environmental exposure to carcinogens or through family history of cancer, may be at risk for developing lung cancer.

There is a need for a simple laboratory test which can be used to augment other forms of lung cancer diagnosis and to identify individuals with latent lung cancers. There is also need for a test to screen individuals for a predisposition to lung cancer.

### Summary of the Invention

The present invention relates to the human pRb2/p130 gene and pRb2/p130 protein, and their use as molecular markers in methods for the diagnosis and prognosis of cancer and for prediction of a predisposition to cancer. According to a preferred embodiment of the invention, the cancer is a gynecologic cancer or a non-small cell lung cancer. According to a most preferred embodiment of the invention, the cancer is endometrial carcinoma, ovarian cancer, a squamous cell carcinoma of the lung, or adenocarcinoma of the lung.

It is an object of the invention to provide a method for determining a prognosis in a patient afflicted with cancer comprising determining the expression level of the pRb2/p130 gene in a sample from the patient. A decreased level of pRb2/p130 gene expression in the sample is indicative of an unfavorable prognosis.

Another object of the invention is to provide a method for detecting or identifying a cancerous disease state in a tissue comprising determining the expression level of the pRb2/p130 gene in a sample of the tissue. Evaluation is advantageously conducted by determining the level of pRb2/p130 expression in the sample, and comparing the expression level in the sampled tissue with the pRb2/p130 expression level in normal, non-cancerous tissue. A decreased pRb2/p130 expression level is indicative of the presence of cancer. This method may be used to detect cancer in an individual not otherwise displaying a visible lesion.

A further object of the invention is to provide a method for identifying disease free individuals at risk for cancer, or individuals at risk for the recurrence of cancer following treatment, comprising determining the level of expression of the pRb2/p130 gene in tissue sampled from an individual and comparing the pRb2/p130 expression level in the sampled tissue with a normal pRb2/p130 expression level. A decreased level of pRb2/p130 expression is indicative of the likelihood of disease or disease recurrence. In the case of endometrial cancer, a method is provided for identifying the risk of recurrence following hysterectomy, and for evaluating the need for further treatment such as radiation therapy or chemotherapy.

Another object of the invention is to provide a method for grading a cancer comprising determining the level of expression of the pRb2/p130 gene in a sample of tissue from a patient suffering from cancer. The expression level in the sampled tissue is compared with the expression level in normal tissue. The degree of the decrement in expression level in the cancer sampled tissue as compared to the normal tissue is indicative of the pathological grade of the cancer. A larger decrement indicates a more aggressive disease state.

It is an object of the invention to provide a DNA segment consisting essentially of an intron of the pRb2/p130 gene, or an at least 15 nucleotide segment thereof.

Another object of the invention is to provide an amplification primer of at least 15 nucleotides consisting essentially of a DNA segment having a nucleotide sequence substantially complementary to a segment of a pRb2/p130 intron exclusive of the splice signal dinucleotides of said intron.

A further object of the invention is to provide methods for identifying polymorphisms and mutations in an exon of a human pRb2/p130 gene.

One embodiment of the invention includes a method for amplifying and identifying polymorphisms and mutations in an exon of a human pRb2/p130 gene, which method comprises:
(a) treating, under amplification conditions, a sample of genomic DNA containing the exon with a primer pair comprising a first primer which hybridizes to the promoter region or to an intron upstream of said exon and a second primer which hybridizes to an intron or to the 3'-noncoding region, said treatment producing an amplification product containing said exon;
(b) determining the nucleotide sequence of said amplification product to provide the nucleotide sequence of said exon; and
(c) comparing the sequence of said exon obtained in step b to a sequence for the sequence of a corresponding wild type exon.

Each primer of the PCR primer pair consists of an amplification primer of at least 15 nucleotides consisting essentially of a DNA segment from the promoter region, from a pRb2/p130 intron exclusive of the splice signal dinucleotides, or from the 3'-noncoding region.

The amplification primer described above has a nucleotide sequence substantially complementary to the 3'-noncoding region, the promoter region given as SEQ ID NO: 113, or an intron having a nucleotide sequence selected from the group consisting of SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62. SEQ ID NO:63, SEQ ID NO:64, SEQ ID NO:65. SEQ ID NO:66. SEQ ID NO:67, and SEQ ID NO:68.

In a preferred embodiment, the amplification primer as described above has a nucleotide sequence selected from the group consisting of SEQ ID NO:69, SEQ ID NO:70. SEQ ID NO:71, SEQ ID NO:72. SEQ ID NO:73. SEQ ID NO:74, SEQ ID NO:75, SEQ ID NO:76. SEQ ID NO:77. SEQ ID NO:78, SEQ ID NO:79, SEQ ID NO:80, SEQ ID NO:81. SEQ ID NO:82, SEQ ID NO:83, SEQ ID NO:84, SEQ ID NO:85, SEQ ID NO:86. SEQ ID NO:87, SEQ ID NO:88, SEQ ID NO:89, SEQ ID NO:90, SEQ ID NO:91, SEQ ID NO:92, SEQ ID NO:93, SEQ ID NO:94, SEQ ID NO:95. SEQ ID NO:96, SEQ ID NO:97, SEQ ID NO:98, SEQ ID NO:99, SEQ ID NO:100, SEQ ID NO: 101, SEQ ID NO:102, SEQ ID NO: 103, SEQ ID NO:104, SEQ ID NO:105, SEQ ID NO:106, SEQ ID NO: 107, SEQ ID NO:108, SEQ ID NO: 109, SEQ ID NO: 110, SEQ ID NO: 111, and SEQ ID NO:112.

Another embodiment of the invention includes a method for identifying polymorphisms and mutations in an exon of a human pRb2/p130 gene, which method comprises:
(a) forming a polymerase chain reaction admixture by combining in a polymerase chain reaction buffer, a sample of genomic DNA containing said exon, a primer pair comprising a first primer which hybridizes to the promoter region or to an intron upstream of said exon and a second primer which hybridizes to the 3'-noncoding region or to an intron downstream of said exon, a mixture of one or more deoxynucleotide triphosphates, and a compound capable of radioactively labeling said primer pair, and a DNA polymerase;
(b) subjecting said admixture to a plurality of polymerase chain reaction thermocycles to produce a pRb2/p130 amplification product;
(c) denaturing said pRb2/p130 amplification product:
(d) electrophoretically separating said denatured pRb2/p130 amplification product:
(e) exposing the electrophoretically separated product of step d to a film to produce a photographic image; and
(e) comparing the mobility of the bands in said photographic image of said pRb2/p130 amplification product to a electrophoretically separated amplification product for a corresponding wild type exon.

In another embodiment, the invention includes a method for identifying mutations in a human chromosomal sample containing an exon of a human pRb2/p130 gene, which method comprises:
(a) forming an admixture by combining in a buffer, a chromosomal sample containing said exon, a primer pair comprising a first primer which hybridizes to the promoter region or to an intron upstream of said exon and a second primer which hybridizes to the 3'-noncoding region or to an intron downstream of said exon, a mixture of one or more deoxynucleotide triphosphates including at least one deoxynucleotide triphosphate that is labeled, and a DNA polymerase;
(b) subjecting said admixture to a temperature and time sufficient to produce a pRb2/p130 amplification product; and
(c) visualizing said pRb2/p130 amplification product with a fluorochrome conjugate specific to said label; and
(d) comparing the visualized pRb2/p130 amplification product obtained in step a to a visualized amplification product for a corresponding wild type exon.

These and other objects will be apparent to those skilled in the art from the following discussion.

### Description of the Figures

Figure 1 is a plot of the probability of survival of 100 patients with endometrial carcinoma (all stages) who were characterized as having either pRb2/p130-positive or pRb2/p130-negative tumors.
Figure 2 is a plot of the probability of survival in the same 100 patients with endometrial carcinoma, as stratified by stage and pRb2/p130 expression.
Figure 3A is a schematic representation of the human pRb2/p130 gene. Exons are represented by open rectangles, while the introns are represented by hatched vertical bars. Exons 10-13, 14-16, and 17-20 represent domain A, a spacer, and domain B, respectively.
Figure 3B is a schematic representation of the human pRb2/p130 genomic clones derived from the P1 and X phage libraries.

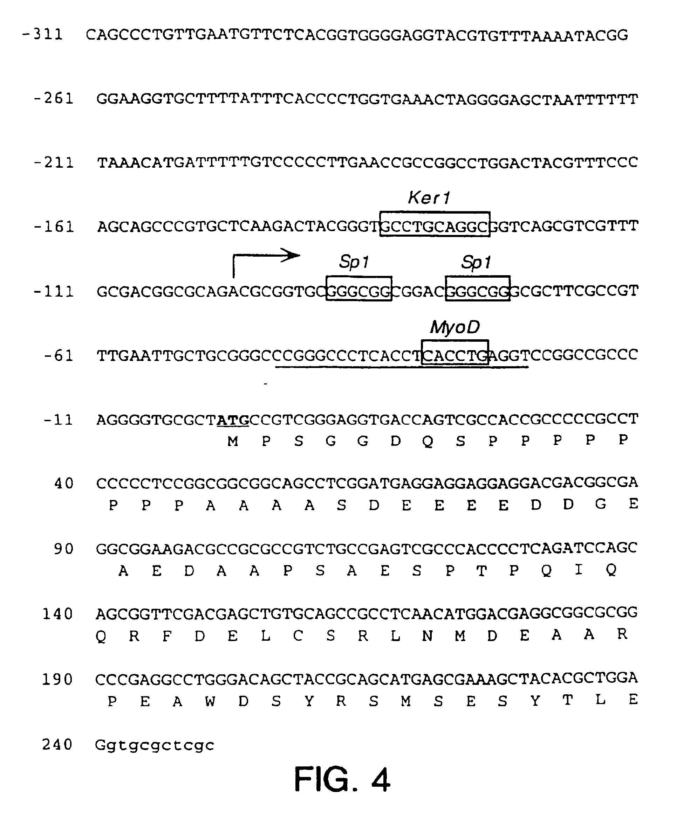

Figure 4 is the nucleotide sequence (SEQ ID NO:4) of the 5' end and 5' upstream region of the human pRb2/p130 gene showing the transcription start site (→) and the sequence complementary to a primer utilized for a primer extension analysis (underlined). Position + 1 is assigned to the A of the ATG translation start codon (bold and underlined). The sequences corresponding to the Sp1 factor recognition motif are boxed. Also boxed are the sequence motifs corresponding to the MyoD and Ker1 transcription factors. The nucleotides beginning at position I through position 240 correspond to exon 1 of pRb2/p130. The lowercase letters beginning at position 241 represent the first ten nucleotides of intron 1.

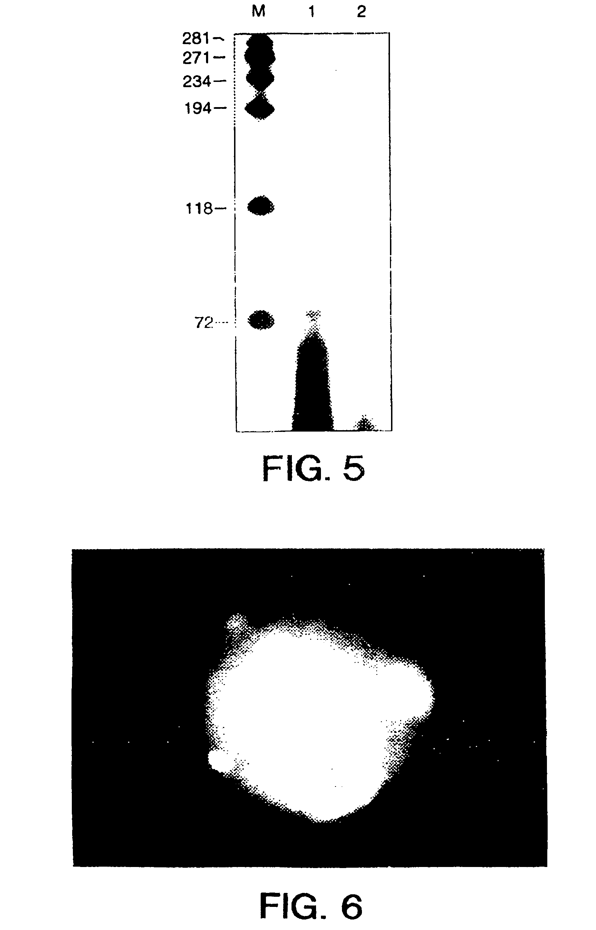

Figure 5 shows the products of a primer extension experiment done to identify the transcription start site for the human pRb2/p130 gene. Cytoplasmatic RNA was hybridized overnight to an oligonucleotide complementary to the twenty four nucleotides beginning at position -22 of Figure 4 (SEQ ID NO:4). Lane M contains molecular-weight markers (φχ174 DNA/Hae III. Promega). Lanes 1 and 2 contain the primer-extended product of pRb2/p130 from HeLa cells and tRNA as template, respectively. Figure 6 illustrates two alleles containing exon 20 of the pRb2/p130 gene in the nucleus of a peripheral blood lymphocyte visualized through the use of the PRINS technique.

### Detailed Description of the Invention

### A. Abbreviations and Definitions

### 1. Abbreviations

- bp: base pairs
- BSA: Bovine Serum Albumin
- dATP: deoxyadenine triphosphate
- dCTP: deoxycytosine triphosphate
- dGTP: deoxyquenosine triphosphate
- DIG DNA: Digoxigenin-labeled DNA
- DIG-dUTP: Digoxigenin-deoxyuridine triphosphate
- DNA: deoxyribonucleic acid
- dTTP: deoxythymine triphosphate
- EDTA: ethylene dinitrolotetraacetic acid
- FITC: fluorescein isothiocyanate
- PCR: polymerase chain reaction
- PHA: phytohemagglutinin
- PRINS: oligonucleotide-PRimed *IN Situ* synthesis
- RNA: ribonucleic acid
- SDS: sodium dodecyl sulfate
- SSC: standard saline citrate
- SSCP: single-strand conformation polymorphism
- TBE: buffer mixture of 0.09 M tris. 0.09 M boric acid, and 2.5 mM EDTA

### 2. Definitions

**"Allele"** refers to one or more alternative forms of a gene occupying a given locus on a chromosome.

**"Affected tissue"** means tissue which, through visual or other examination, is believed to contain a purported cancerous or precancerous lesion.

**"Amplification product"** refers to a nucleic acid segment produced by amplification procedures such as PCR, SSCP, and PRINS, which product is complementary to the template segment amplified.

**"Downstream"** identifies sequences which are located in the direction of expression, i.e., on the 3'-side of a given site in a nucleic acid.

**"Endometrial cancer"** or **"endometrial carcinoma"** means a polypoid growth arising in the endometrium.

**"Expression",** with respect to the pRb2/p130 gene, means the realization of genetic information encoded in the gene to produce a functional RNA or protein. The term is thus used in its broadest sense, unless indicated to the contrary, to include either transcription or translation.

**"Expression level",** with respect to the pRb2/p130 gene, means not only an absolute expression level, but also a relative expression level as determined by comparison with a standard level of pRb2/p130 expression.

**"Genomic DNA"** refers to all of the DNA sequences composing the genome of a cell or organism. In the invention described herein it includes the exons, introns, and regulatory elements for the pRb2/p130 gene.

**"Grading",** with respect to a tumor sample, means a classification of the perceived degree of malignancy. In grading tumor samples, a pathologist or other observer evaluates the degree of differentiation (e. g. grade 1, well differentiated, grade 2, moderately differentiated, grade 3, poorly differentiated) of the tissue.

**"Gynecologic cancer"** means a tumor arising in the uterus, ovary, cervix, vagina, vulva, or fallopian tube, as well as gestational trophoblastic disease.

**"Hybridization"** means the Watson-Crick base-pairing of essentially complementary nucleotide sequences (polymers of nucleic acids) to form a double-stranded molecule.

**"3'-noncoding region"** means those nucleic acid sequences downstream of the termination codon.

**"Non-small cell lung cancer"** (NSCLC) means all forms of lung cancer except small cell lung cancer (SCLC). In particular, by non-small cell lung cancer is meant the group of lung cancers including squamous cell carcinomas, adenocarcinomas, bronchiolo-alveolar carcinomas and large cell carcinomas.

**"Polymorphic"** refers to the simultaneous occurrence in the population of genomes showing allelic variations. As used herein the term encompasses alleles producing different phenotypes, as well as proteins for which amino acid variants exist in a population, but for which the variants do not destroy the protein's function.

**"Primer"** refers to an oligonucleotide which contains a free 3' hydroxyl group that forms base pairs with a complementary template strand and is capable of acting as the starting point for nucleic acid synthesis by a polymerase. The primer can be single-stranded or double-stranded, however, if in double-stranded form, the primer is first treated in such a way so as to separate it from its complementary strand.

**"pRb2/p130 gene"** means the gene which encodes the pRb2/p130 protein, the cDNA of which is set forth as SEQ ID NO:1, and all allelic variations and mutants thereof.

**"pRb2/p130 intron"** as used herein means a wild type intron segment of the pRb2/p130 gene, as well as any allelic variations thereof.

**"pRb2/p130 protein"** means the translation product of the pRb2/p130 gene, including all allelic variations and mutants thereof. The pRb2/p130 amino acid sequence is set forth as SEQ ID NO:2.

**"Prognosis"** is used according to its ordinary medical meaning, that is, the prospect of recovery from a disease.

**"Splice junction"** or **"exon-intron junction"** refers to the nucleotide sequence immediately surrounding an exon-intron boundary of a nuclear gene. As used herein the term includes the sites of breakage and reunion in the mechanism of RNA splicing.

**"Splice signal dinucleotide"** refers to the first two nucleotides (5'-terminal) or the last two nucleotides (3'-terminal) of an intron. In highly conserved genes the 5'-terminal dinucleotide is GT and the 3'-terminal dinucleotide is AG. Alternatively, the 5'-terminal dinucleotide and the 3'-terminal dinucleotide are referred to as the **"donor"** and **"acceptor"** sites, respectively.

**"Substantially complementary nucleotide sequence"** means, as between two nucleotide sequences, a relationship such that the sequences demonstrate sufficient Watson-Crick base-pair matching to enable formation of a hybrid duplex under hybridization conditions. It is not required, however, that the base-pair matchings be exact.
**Downstream"** identifies sequences which are located in the direction of expression, i.e., on the 3'-side of

**"Upstream"** identifies sequences which are located in the direction opposite from expression, i.e. on the 5'-side of a given site in a nucleic acid.

The present invention provides methods for the identification of individuals at risk for cancer, and for the detection and evaluation of cancers. These methods are of two basic types: methods based on determination of pRb2/p130 expression levels, and methods based on determination of the gnomic structure of pRB2/p130.

### B. Methods Based on Determination of pRb2/p130 Expression Level

The present invention provides improved methods, based on pRb2/p130 expression levels, for the diagnosis and prognosis of cancers including but not limited to gynecologic cancers and non-small cell lung cancers. Among the gynecologic cancers to which these methods may be applied are ovarian cancer and endometrial cancer.

### 1. Gynecologic Cancers

Early ovarian cancer is frequently asymptomatic, or produces only mild symptoms which might be ignored by the patient. The majority of ovarian tumors have spread beyond the ovary, and frequently beyond the pelvis, at the time of diagnosis. Improved methods for the diagnosis and prognosis of ovarian cancer will be useful in treatment selection, and should have a favorable effect on patient outcomes. The present invention rests on the discovery that in ovarian cancer tissue, there is a correlation between the expression of pRb2/p130 and tumor grade.

Endometrial cancer often follows a favorable course, however a considerable proportion of these cases behave poorly and ultimately die of the disease. Currently used surgical-pathologic parameters do not always allow the identification of this subset of patients.

According to the F.I.G.O. criteria for staging in endometrial cancer, surgical procedure should always include peritoneal washing, abdominal hysterectomy, bilateral salpingo-oophorectomy and systematic pelvic and paraaortic lymphadenectomy. Indeed, this operation is often unnecessarily "radical" and potentially dangerous to patients with tumors limited to the uterine corpus. This observation becomes more relevant if it is considered that patients with endometrial cancer very often present also cardiovascular disease, diabetes mellitus, hypertension and severe obesity (Wingo et al., Am J Obstet Gynecol 152:803-8 (1985), which are known risk factors for morbidity from abdominal surgery (DiSaia et al., "Adenocarcinoma Of The Uterus" In: Clinical Gynecologic Oncology, St. Louis: Mosby-Year Book. p. 156-93 (1993). On the other hand. in the obese or patients at high surgical risk total hysterectomy can be easily and safely performed by the vaginal technique (Massi et al., Am J Obstet Gynecol 174:1320-6 (1996); Pitkin, Obstet Gynecol 49:567-9 (1977); Peters et al., Am J Obstet Gynecol 146:285-90 (1983)). With this in view, the relative pRb2/p130 expression, assayed according to the present invention may be used in the selection of candidates for a less aggressive surgical treatment, without decreasing their chance of cure, as well as being helpful for the identification of high risk patients, to whom every surgical effort should be attempted and post-surgical treatment given.

Normal cells of the endometrium express a relatively high level of pRb2/p130 protein. The present invention rests on the discovery of a highly statistical inverse correlation between the expression of pRb2/p130 in tissues from endometrial cancer patients and the eventual clinical outcome following treatment. Decreased levels of pRb2/p130 are significantly associated with a poor survival. The study results reported herein indicate that the risk of dying of endometrial carcinoma is increased almost fivefold in patients whose tumors were pRb2/p130 negative, regardless of the tumor stage or grade of differentiation.

Tissue with the greatest malignant potential expresses little or no pRb2/p130. Accordingly, a sample is contacted with an antibody specific for pRb2/p130 protein. In the case of endometrial cancer, the sample may typically comprise endometrial tissue, and may specifically comprise an endometrial tumor. The amount of antibody bound by the sample may be determined relative to the amount of antibody bound by a sample of normal endometrial tissue. The difference in the amount of antibody bound by the normal and test samples is indicative of the patient's prognosis. The endometrial carcinoma study described in Example I concurrently tested a known molecular prognostic indicator, i.e., DNA index, various classic clinical-pathologic parameters and pRb2/p130 expression. Decreased levels of pRb2/p130 were significantly associated with a poorer survival. The expression of pRb2/p130 thus represents an independent predictor of clinical outcome in endometrial carcinoma. Well known risk factors, such as F.I.G.O. stage and tumor ploidy were also confirmed as independent prognosticators, although of minor strength. The pRb2/p130 expression was significantly correlated with tumor ploidy and patient age, in that pRb2/p130 negativity was associated with aneuploidy (P=0.001) and with age > 65 years (P=0.008), in accordance with the known negative impact of such features on survival in endometrial cancer (DiSaia et al., Am J Obstet Gynecol 151:1009-15 (1985); Susini et al., Am J Obstet Gynecol 170:527-34 (1994); Massi et al., Am J Obstet Gynecol 174:1320-6 (1996)). However, it is noteworthy that tumor ploidy resisted as an independent prognostic variable by multivariate analysis. Stratification by pRb2/p130 status and ploidy allowed identification of patient subgroups with significant differences in survival (data not shown). A trend toward correlation was also found between pRb2/p130 status and another major prognostic indicator such as grade of differentiation, where pRb2/p130 negativity was more frequent among moderately and poorly differentiated tumors (P=0.06). Furthermore, concerning grade of differentiation, stratification by pRb2/p130 status revealed significant differences in survival within each grade group (data not shown). Expression of pRb2/p130 was not correlated with tumor stage; pRb2/p130 negative tumors were equally distributed among different tumor stages, thus indicating that this feature is typical of certain tumors, from their onset in early stages.

Thus, the pRb2/p130 expression level may serve as a convenient molecular marker to replace or augment conventional prognostic techniques. An important advantage of the use of pRb2/p130 expression over classical surgical pathologic parameters as a prognostic factor is that the former can be determined at the time of the initial diagnosis, before any therapy is initiated. For patients not previously treated by radiotherapy or chemotherapy, low levels of pRb2/p130 can be used to identify tumors with a tendency to behave aggressively.

An early accurate determination of the aggressiveness of disease in an individual patient is a necessary part of designing a course of treatment. In cases where the test method of the invention identifies a poor prognosis, adjuvant therapy, such as radiation therapy or chemotherapy, may be initiated. This more aggressive treatment should increase the patient's chance of survival. The pRb2/p130 expression level, even in early stages of the disease, is reflective of the malignant potential of the patient's carcinoma and the aggressiveness of the ensuing disease course. This form of "molecular based" prognosis can be evaluated more consistently than conventional prognostic factors which are based upon subjective evaluations of histological type, grade of differentiation, depth of myometrial invasion, degree of lymph nodal metastases, extra-uterine spread, and the other factors upon which endometrial carcinoma prognoses are presently based.

### 2. Lung Cancer

In the case of lung cancer, a sample of lung tissue is removed from an individual by conventional biopsy techniques which are well-known to those skilled in the art. The sample is generally collected by needle biopsy. See, for example, Cancer: Principles & Practice of Oncology, V. T. DeVita, Jr. et al., eds. 3rd edition (1989), J. B. Lippincott Co., Philadelphia, PA, p. 616-619, incorporated herein by reference (transcarinal needle biopsy and transthoracic percutaneous fine-needle aspiration biopsy). For identification of lung lesions as comprising NSCLC, the sample is taken from the disease lesion. The disease lesion is first located by x-ray or other conventional lung lesion imaging techniques known to those skilled in the art. For testing for latent NSCLC or NSCLC predisposition, the tissue sample may be taken from any site in the lung. Tissue with the greatest malignant potential expresses little or no pRb2/p130. Normal lung tissue cells express a high level of pRb2/p130 protein. The pRb2/p130 expression level in the cells of the patient lung tumor tissue can be compared with the level in normal lung tissue of the same patient, or with the level in the lung tissue of a normal control group.

Non-small cell lung cancer (NSCLC) includes squamous cell carcinomas, adenocarcinomas, bronchiolo-alveolar carcinomas and large cell carcinomas. A highly significant statistical inverse correlation has been established between the expression of pRb2/p130 in tissues from non-small cell lung cancers and the tissues' pathological grading by skilled pathologists.

Thus, the pRb2/p130 expression level may serve as a convenient molecular marker to replace or augment conventional tumor grading. Accurate tumor grading is a necessary part of designing a course of treatment for the individual patient. Grading is reflective of the malignant potential of the tumor in question and thus the aggressiveness of the ensuing disease course. The generation of vital tumor grade information is made easier, by relying on pRb2/p130 as a molecular surrogate for more subjective observations concerning tumor histology. This form of "molecular-based" grading can be performed more consistently than conventional pathological grading which is based upon subjective evaluations by expert pathologists, pRb2/p130 expression levels may also serve as a convenient molecular marker for the presence of active or latent NSCLC, or predisposition to NSCLC.

Lung lesions may be identified as non-small cell lung carcinomas (NSCLCs) by showing a decrement in the expression of pRb2/p130 in the lesion compared to the level of pRb2/p130 in normal, non-cancerous control lung tissue. Similarly, the level of pRb2/p130 expression in lung tissue of individuals with no apparent lung lesion but other symptoms of lung cancer, or in disease-free individuals, indicates latent NSCLC or risk of NSCLC, respectively. Early diagnosis of NSCLC, even before the appearance of visible lung lesions, will permit earlier initiation of treatment and increased survival.

According to the practice of the invention, an at least about one-third decrement in pRb2/p130 expression level in an affected lung tissue sample, in comparison with normal controls, indicates that the lesion is an NSCLC. Similarly, a pRb2/p130 expression decrement of about one-third or greater in lung tissue of patients who are free of lung lesions but manifest other potential lung cancer symptoms such as sputum cytology irregularities, coughing or bronchitis, is indicative of pre-lesion NSCLC. An about one-third or greater pRb2/p130 expression decrement in lung tissue of otherwise healthy individuals manifesting no symptoms of lung cancer is believed indicative of a risk of future NSCLC. Decrements in pRb2/p130 expression of about one-half or greater are even more indicative of NSCLC disease or NSCLC predisposition.

According to one aspect of the invention, individuals who are disease free are evaluated for risk in contracting NSCLC. The test method may be used to identify individuals at risk of developing NSCLC from among populations exposed to environmental carcinogens, e.g. asbestos workers, miners, textile workers, tobacco smokers and the like, and from among families having a history of NSCLC or other forms of cancer.

### 3. Methods for Determining Expression Levels

According to the practice of the present invention, a sample of affected tissue is removed from a cancer patient by conventional biopsy techniques which are well-known to those skilled in the art. The sample is preferably obtained from the patient prior to initiation of radiotherapy or chemotherapy. The sample is then prepared for a determination of pRb2/p130 expression level.

Determining the relative level of expression of the pRb2/p130 gene in the tissue sample comprises determining the relative number of pRb2/p130 RNA transcripts, particularly mRNA transcripts in the sample tissue, or determining the relative level of the corresponding pRb2/p130 protein in the sample tissue. Preferably, the relative level of pRb2/p130 protein in the sample tissue is determined by an immunoassay whereby an antibody which binds pRb2/p130 protein is contacted with the sample tissue. The relative pRb2/p130 expression level in cells of the sampled tumor is conveniently determined with respect to one or more standards. The standards may comprise, for example, a zero expression level on the one hand and the expression level of the gene in normal tissue of the same patient, or the expression level in the tissue of a normal control group on the other hand. The standard may also comprise the pRb2/p130 expression level in a standard cell line. The size of the decrement in pRb2/p130 expression in comparison to normal expression levels is indicative of the future clinical outcome following treatment.

Methods of determining the level of mRNA transcripts of a particular gene in cells of a tissue of interest are well-known to those skilled in the art. According to one such method, total cellular RNA is purified from the effected cells by homogenization in the presence of nucleic acid extraction buffer, followed by centrifugation. Nucleic acids are precipitated, and DNA is removed by treatment with DNase and precipitation. The RNA molecules are then separated by gel electrophoresis on agarose gels according to standard techniques, and transferred to nitrocellulose filters by, *e.g.,* the so-called "Northern" blotting technique. The RNA is immobilized on the filters by heating. Detection and quantification of specific RNA is accomplished using appropriately labelled DNA or RNA probes complementary to the RNA in question. See Molecular Cloning: A Laboratory Manual, J. Sambrook et al., eds., 2nd edition, Cold Spring Harbor Laboratory Press, 1989, Chapter 7, the disclosure of which is incorporated by reference.

In addition to blotting techniques, the mRNA assay test may be carried out according to the technique of *in situ* hybridization. The latter technique requires fewer tumor cells than the Northern blotting technique. Also known as "cytological hybridization", the *in situ* technique involves depositing whole cells onto a microscope cover slip and probing the nucleic acid content of the cell with a solution containing radioactive or otherwise labelled cDNA or cRNA probes. The practice of the *in situ* hybridization technique is described in more detail in U.S. Patent 5,427,916, the entire disclosure of which is incorporated herein by reference.

The nucleic acid probes for the above RNA hybridization methods can be designed based upon the published pRb2/p130 cDNA sequence of Li et al., Genes Dev. 7: 2366-2377 (1993), the entire disclosure of which is incorporated herein by reference. The nucleotide sequence is reproduced herein as SEQ ID NO: 1. The translation initiation codon comprises nucleotides 70-72 of SEQ ID NO:1. The translation termination codon comprises nucleotides 3487-3489.

Either method of RNA hybridization, blot hybridization or *in situ* hybridization, can provide a quantitative result for the presence of the target RNA transcript in the RNA donor cells. Methods for preparation of labeled DNA and RNA probes, and the conditions for hybridization thereof to target nucleotide sequences, are described in *Molecular Cloning, supra*, Chapters 10 and 11, incorporated herein by reference.

The nucleic acid probe may be labeled with, e.g., a radionuclide such as ³²P, ¹⁴C, or ³⁵S; a heavy metal; or a ligand capable of functioning as a specific binding pair member for a labelled ligand, such as a labelled antibody, a fluorescent molecule, a chemolescent molecule, an enzyme or the like.

Probes may be labelled to high specific activity by either the nick translation method or Rigby et al., J. Mol. Biol. 113: 237-251 (1977) or by the random priming method, Fienberg et al., Anal. Biochem. 132: 6-13 (1983). The latter is the method of choice for synthesizing ³²P-Iabelled probes of high specific activity from single-stranded DNA or from RNA templates. Both methods are well-known to those skilled in the art and will not be repeated herein. By replacing preexisting nucleotides with highly radioactive nucleotides, it is possible to prepare ³²P-labelled DNA probes with a specific activity well in excess of 10⁸ cpm/microgram according to the nick translation method. Autoradiographic detection of hybridization may then be performed by exposing filters on photographic film. Densitometric scanning of the filters provides an accurate measurement of mRNA transcripts.

Where radionuclide labelling is not practical, the random-primer method may be used to incorporate the dTTP analogue 5-(N-(N-biotinyl-epsilon-aminocaproyl)-3-aminoallyl)deoxyuridine triphosphate into the probe molecule. The thus biotinylated probe oligonucleotide can be detected by reaction with biotin binding proteins such as avidin, streptavidin, or anti-biotin antibodies coupled with fluorescent dyes or enzymes producing color reactions.

The relative number of pRb2/p130 transcripts may also be determined by reverse transcription of mRNA followed by amplification in a polymerase chain reaction (RT-PCR), and comparison with a standard. The methods for RT-PCR and variations thereon are well known to those of ordinary skill in the art.

According to another embodiment of the invention, the level of pRb2/p130 expression in cells of the patient tissue is determined by assaying the amount of the corresponding pRb2/p130 protein. A variety of methods for measuring expression of the pRb2/p130 protein exist, including Western blotting and immunohistochemical staining. Western blots are run by spreading a protein sample on a gel, using an SDS gel, blotting the gel with a cellulose nitrate filter, and probing the filters with labeled antibodies. With immunohistochemical staining techniques, a cell sample is prepared, typically by dehydration and fixation, followed by reaction with labeled antibodies specific for the gene product coupled, where the labels are usually visually detectable, such as enzymatic labels, florescent labels, luminescent labels, and the like.

According to one embodiment of the invention, tissue samples are obtained from patients and the samples are embedded then cut to e.g. 3-5 µm, fixed, mounted and dried according to conventional tissue mounting techniques. The fixing agent may advantageously comprise formalin. The embedding agent for mounting the specimen may comprise, e. g. , paraffin. The samples may be stored in this condition. Following deparaffinization and rehydration, the samples are contacted with an immunoreagent comprising an antibody specific for pRb2/p130. The antibody may comprise a polyclonal or monoclonal antibody. The antibody may comprise an intact antibody, or fragments thereof capable of specifically binding pRb2/p130 protein. Such fragments include, but are not limited to, Fab and F(ab')₂ fragments. As used herein, the term "antibody" includes both polyclonal and monoclonal antibodies. The term "antibody" means not only intact antibody molecules, but also includes fragments thereof which retain antigen binding ability.

Appropriate polyclonal antisera may be prepared by immunizing appropriate host animals with pRb2/p130 protein and collecting and purifying the antisera according to conventional techniques known to those skilled in the an. Monoclonal antibody may be prepared by following the classical technique of Kohler and Milstein, Nature 254:493497 (1975), as further elaborated in later works such as Monoclonal Antibodies, Hybridomas: A New Dimension in Biological Analysis, R. H. Kennet et al., eds., Plenum Press, New York and London (1980).

Substantially pure pRb2/p130 for use as an immunogen for raising polyclonal or monoclonal antibodies may be conveniently prepared by recombinant DNA methods. According to one such method, pRb2/p130 is prepared in the form of a bacterially expressed glutathione S-transferase (GST) fusion protein. Such fusion proteins may be prepared using commercially available expression systems, following standard expression protocols, e.g., "Expression and Purification of Glutathione-S-Transferase Fusion Proteins", Supplement 10, unit 16.7, in Current Protocols in Molecular Biology (1990). Also see Smith and Johnson, Gene 67: 34-40 (1988); Frangioni and Neel, Anal. Biochem. 210: 179-187 (1993). Briefly, DNA encoding for pRb2/p130 is subcloned into a pGEX2T vector in the correct reading frame and introduced into *E*. *coli* cells. Transformants are selected on LB/ampicillin plates; the plates are incubated 12 to 15 hours at 37°C. Transformants are grown in isopropyl-β-D-thiogalactoside to induce expression of pRb2/p130-GST fusion protein. The cells are harvested from the liquid cultures by centrifugation. The bacterial pellet is resuspended and the cell pellet sonicated to lyse the cells. The lysate is then contacted with glutathione-agarose beads. The beads are collected by centrifugation and the fusion protein eluted. The GST carrier is then removed by treatment of the fusion protein with thrombin cleavage buffer. The released pRb2/p130 protein is recovered.

As an alternative to immunization with the complete pRb2/p130 molecule, antibody against pRb2/p130 can be raised by immunizing appropriate hosts with immunogenic fragments of the whole protein, particularly peptides corresponding to the carboxy terminus of the molecule.

The antibody either directly or indirectly bears a detectable label. The detectable label may be attached to the primary anti-pRb2/p130 antibody directly. More conveniently, the detectable label is attached to a secondary antibody, *e.g*., goat anti-rabbit IgG, which binds the primary antibody. The label may advantageously comprise, for example, a radionuclide in the case of a radioimmunoassay; a fluorescent moiety in the case of an immunofluorescent assay; a chemiluminescent moiety in the case of a chemiluminescent assay; or an enzyme which cleaves a chromogenic substrate, in the case of an enzyme-linked immunosorbent assay.

Most preferably, the detectable label comprises an avidin-biotin-peroxidase complex (ABC) which has surplus biotin-binding capacity. The secondary antibody is biotinylated. To locate pRb2/p130 antigen in the tissue section under analysis, the section is treated with primary antiserum against pRb2/p130, washed, and then treated with the secondary antiserum. The subsequent addition of ABC localizes peroxidase at the site of the specific antigen, since the ABC adheres non-specifically to biotin. Peroxidase (and hence antigen) is detected by incubating the section with *e*.*g*. H₂O₂ and diaminobenzidine (which results in the antigenic site being stained brown) or H₂O₂ and 4-chloro-1-naphthol (resulting in a blue stain).

The ABC method can be used for paraffin-embedded sections, frozen sections, and smears. Endogenous (tissue or cell) peroxidase may he quenched *e.g*. with H₂O₂ in methanol.

The level of pRb2/p130 expression in tumor samples may be compared on a relative basis to the expression in normal tissue samples by comparing the stain intensities, or comparing the number of stained cells. The lower the stain intensity with respect to the normal controls, or the lower the stained cell count in a tissue section having approximately the same number of cells as the control section, the lower the expression of the pRb2/p130 gene, and hence the higher the expected malignant potential of the sample.

In the examples which follow, a polyclonal antibody raised against pRb2/p130, designated ADL1 was utilized. The specificity of the antibody has been confirmed by Western blot analysis, (Pertile et al., Cell Growth & Diff 6:1659-64 (1995); Claudio et al., Cancer Res 56:2003-8 (1996)), as well as by immunoprecipitation of the antibody with the *in vitro* translated forms of the cDNAs coding for pRb2/p130 and for the other retinoblastoma related proteins, pRb/p105 and p107. The ADL1 antibody was able to immunoprecipitate only the *in vitro* translated form of the pRb2/p130 protein (Baldi et al., Clin Cancer Res 2:1239-45 (1996).

### C. Methods Based on Determination of the Genomic Structure of pRB2/p130

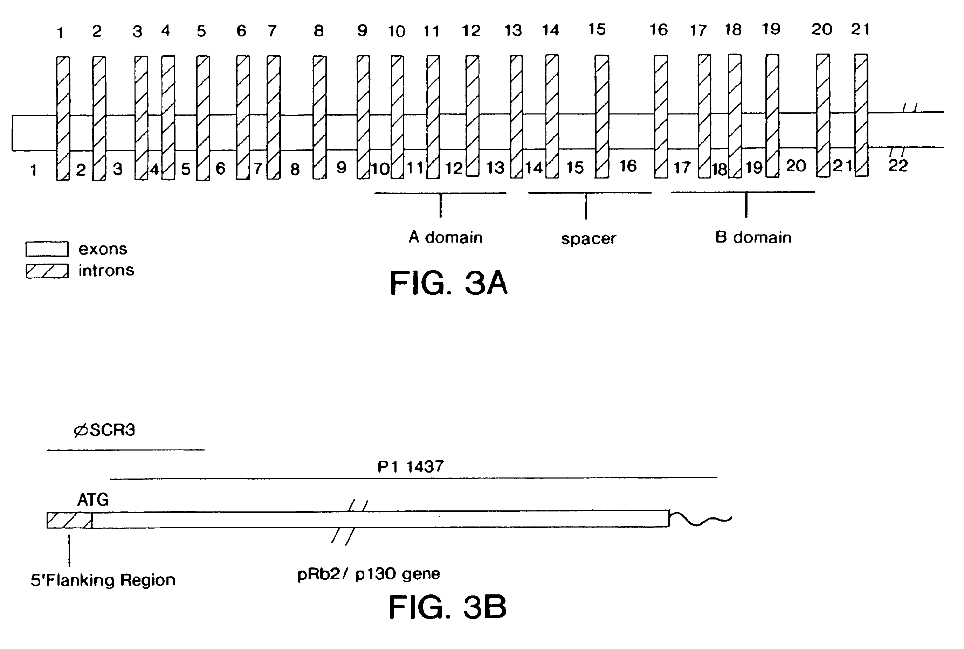

The genomic structure of the human pRb2/p130 gene is described herein. The pRb2/p130 genomic DNA has been cloned and sequenced. The pRb2/p130 gene has been mapped to the long arm of chromosome 16, an area previously reported to show loss of heterozygosity (LOH) for human neoplasias. The putative promoter for pRb2/p130 has been identified, cloned and sequenced. The complete intron-exon organization of the gene has been elucidated. The pRb2/p130 gene contains 22 exons and 21 introns, spanning over 50 kb of genomic DNA. The length of the individual exons ranges from 65 bp to 1517 bp, while the length of individual introns ranges from 82 bp to 9837 bp. The organization of these exons and introns are shown in Figure 3A. The location and size of each exon and intron of pRb2/p130, as well as the nucleotide sequences at the exon-intron junctions are shown below in Table 7. (SEQ ID NOS:6-47). The exon sequences are shown in upper case letters, while the intron sequences are in lower case letters. The superscript numbers correspond to the nucleotide positions of the exon-intron boundaries on SEQ ID NO:1.

All the exons were completely sequenced and no discrepancies were found in comparing the genomic sequence of the exons and the cDNA sequence previously reported. Li, Y. et al., Genes 7:2366-2377 (1993). The exon-intron boundaries were determined by comparing the sequence of the genomic DNA described herein to the published cDNA sequence of Li *et al*., *supra*. The exon-intron boundaries were identified as the positions where the genomic DNA sequence diverged from that of the cDNA.

With the exception of exon 22, the largest of all the exons (1517 bp in length), the exons found were relatively small, with the shortest, exons 4 and 7, comprising only 65 nucleotides each. Exons 10 through 20 code for the region of the pRb2/p130 protein which form the "pocket region". Exons 10 through 13 and 17 through 20 translate to Domain A and Domain B, respectively. Exons 14, 15, and 16 code for the region of the pRb2/p130 protein, known as the "spacer." The spacer lies between Domains A and B.

The introns have been completely sequenced. The shortest intron, intron 16, lying between exons 16 and 17, is only 82 bp in length, whereas the largest intron, intron 21, spans 9837 bp. Intron 21 is located between exons 21 and 22. The complete sequences for the introns are given as SEQ ID NOS: 48-68. All of the intron sequences of pRb2/p130 conform to the GT-AG rule found to be characteristic of other human genes. Breathnach, R. et al., Annu. Rev. Biochem. 50:349-383 (1981). This rule identifies the generic sequence of an intron as GT..... AG. Introns having this generic form are
characterized as conforming to the GT - AG rule. The two dinucleotides, GT and AG, known as the "splice signal dinucleotides," act as signals for splicing out the introns during the processing of the pRb2/p130 mRNA. Point mutations in splice signal dinucleotides have been associated with aberrant splicing in other genes *in vivo* and *in vitro. See generally*, Genes V, B. Lewin. Oxford University Press, pp. 913-916, New York (1994) and Yandell *et al*., *supra* at p. 1694. Thus, it is important to identify any mutations to the splice signal dinucleotides or other sequences that are excluded from the RNA transcript during splicing.

The pRb2/pl30 genomic structure and intron sequences described herein may be used to delineate mutations and rearrangements associated with tumor formation. The genomic structure and intron sequences herein may also be used to screen for naturally occurring polymorphisms at the nucleotide level. Knowledge of a specific single polymorphism can be used to eliminate a mutation in pRb2/p130 as a causative factor in a tumor if the purported mutation displays the same pattern as the polymorphism. Knowledge of polymorphisms in pRb2/p130 can be used to determine the genetic linkage of an identical mutation, and in turn, the tracing of parental origin and family histories without the need for time for time intensive sequencing if mutation is of germline origin. These polymorphisms can then be utilized for the development of diagnostic approaches for human neoplasias. However, it should be noted that not all polymorphisms are of equal utility in these applications. It is preferable to seek out mutations in the exons, as these mutations are most likely to lead to tumor development. Further, because the coding regions of the gene are generally more stable and less likely to mutate over time, it follows that polymorphisms in the exon region are typically less common. The detection of a polymorphism in the exon region of pRb2/p130 would enable screening of both genomic DNA and cDNA.

In the examples that follow, several screening methods are exemplified to identify pRb2/p130 mutations and polymorphisms.

### 1. Transcriptional Control of pRb2/p130

There is evidence that tumor suppressor gene products directly interact with transcription factors, such as MyoD, which regulate not only cell growth, but also cell differentiation. Sang *et al*., *supra* at p. 8. Mutations in the sequence region motifs for these transcription factors would be expected to effect the function of the tumor suppressor genes. Accordingly, in addition to identifying the genomic structure of the pRb2/p130 gene, additional experiments were conducted to define the 5'-flanking promoter sequence of pRb2/p130. Part of the putative promotor sequence for pRb2/p130, along with the entire sequence of the first exon and the beginning of the first intron is shown in Figure 4 (SEQ ID NO:4). The full sequence for the putative promoter region is given in SEQ ID NO:113.

To characterize the pRb2/p130 promoter, a primer extension analysis was performed to locate the transcription initiation site. The protocol for the prime-extension analysis is given in the examples that follow. A twenty four nucleotide segment (SEQ ID NO:114) containing the antisense-strand sequence 26 to 50 nucleotides upstream from the putative ATG codon (*See* Fig. 4) was end-labeled and used as a primer for an extension reaction on cyctoplasmatic RNA from HeLa cells. As shown in Fig. 5, a major extended fragment of 78 bp was detected (lane 1) from the primer extension done with HeLa cells as the template. The additional bands detected by the primer extension analysis could represent additional initiation sites. This finding (lane 1) is consistent with a transcription initiation site 99 nucleotides upstream of the start codon. On the contrary, there was no primer extension product observed when tRNA was used as a template (lane 2). The probable position of the identified transcription initiation site within the promoter sequence is indicated by the arrow in Fig. 4. The primer extension analysis was repeated three times, and similar results were produced in each instance.

The putative transcription factor-binding sites were identified by their similarity to consensus sequences for known transcription factor-binding sites by use of the SIGNAL SCAN program. A description of this program is included in the examples that follow. The most recognizable sequence motifs are for the transcription factors Sp1 (two sites), Ker1 and MyoD. Fig. 4 shows the location of these motifs. Ker1 is involved in keratinocyte-specific transcription, while MyoD is involved in myogenesis. Leask et al., Genes Dev. 4: 1985-1998 (1990); Weintraub, H., Cell 75: 1241-1244 (1993). The presence in the promoter region for pRb2/p130 of these sequence motifs supports a hypothesis of an involvement of this gene in the complex pathways regulating differentiation of specific cell systems.

### 2. Detection of Mutations in pRb2/p130

The present invention provides a method for amplifying the genomic DNA of pRb2/p130 and for screening polymorphisms and mutations therein. The assay methods described herein can be used to diagnose and characterize certain cancers or to identify a heterozygous carrier state. While examples of methods for amplifying and detecting mutations in pRb2/p130 are given, the invention is not limited to the specific methods exemplified. Other means of amplification and identification that rely on the use of the genomic DNA sequence for pRb2/p130 and/or the use of the primers described herein are also contemplated by this invention.

Generally, the methods described herein involve preparing a nucleic acid sample for screening and then assaying the sample for mutations in one or more alleles. The nuclei acid sample is obtained from cells. Cellular sources of genomic DNA include cultured cell lines, or isolated cells or cell types obtained from tissue (or whole organs or entire organisms). Preferably, the cell source is peripheral blood lymphocytes. Methods of DNA extraction from blood and tissue samples are known to those skilled in the art. *See, for example*, Blin et al., Nuc. Acids Res. 3:2303-2308 (1976); and Sambrook et al., Molecular Cloning:A Laboratory Manual, Second Edition, pp. 9.16-9.23, Cold Spring Harbor Laboratory Press, Cold Spring Harbor, NY (1989), the entire disclosure of which is incorporated herein by reference. If the patient sample to be screened is in the form of double-stranded genomic DNA. it is first denatured using methods known to those skilled in the art. Denaturation can be carried out either by melting or subjecting the strands to agents that destabilize the hydrogen bonds, such as alkaline solutions and concentrated solutions of formamide or urea.

In one embodiment of the invention, prior to screening the genomic DNA sample, the pRb2/p130 genomic DNA sample is amplified by use of the polymerase chain reaction (PCR), using a primer pair, a buffer mixture, and an enzyme capable of promoting chain elongation. Methods of conducting PCR are well known to those skilled in the art. *See,* for example, Beutler *et al*., U.S. Patent No. 5,234,811, or Templeton, N.S., Diag. Mol. Path. 1(1):58-72 (1992), which are incorporated herein by reference as if set forth at length. The amplification product produced from PCR can then be used to screen for mutations using the techniques known as Single Strand Conformational Polymorphism (SSCP) or Primed *In-Situ* DNA synthesis (PRINS). Of course, mutations can also be identified through the more laborious task of sequencing the gene isolates of a patient and comparing the sequence to that for the corresponding wild type pRb2/p130 segment.

PCR is carried out by thermocycling, *i*.*e*., repeated cycles of heating and cooling the PCR reaction mixture, within a temperature range whose lower end is 37°C to 55°C and upper end is around 90°C to 100°C. The specific temperature range chosen is dependent upon the enzyme chosen and the specificity or stringency required. Lower end temperatures are typically used for annealing in amplifications in which high specificity is not required and conversely, higher end temperatures are used where greater stringency is necessary. An example of the latter is when the goal is to amplify one specific target DNA from genomic DNA. A higher annealing temperature will produce fewer DNA segments that are not of the desired sequence. Preferably, for the invention described herein, the annealing temperature is between 50°C and 65°C. Most preferably, the annealing temperature is 55°C.

The PCR is generally performed in a buffered aqueous solution, *i*.*e*., a PCR buffer, preferably at a pH of 7-9, most preferably about 8. Typically, a molar excess of the primar is mixed with the buffer containing the template strand. For genomic DNA, this ratio is typically 10°:1 (primer: template). The PCR buffer also contains the deoxynucleotide triphosphates (DATP, dCTP, dGTP, and dTTP) and a polymerase. Polymerases suitable for use in PCR include, but are not limited to, *E. coli* DNA polymerase I, the Klenow fragment of *E. coli* DNA polymerase I, T4 DNA polymerase, T7 DNA polymerase, *Taq* DNA polymerase (*Thermus aquaticus* DNA polymerase I), and other heat-stable enzymes which will facilitate the formation of amplification products.

The primers used herein can be naturally occurring oligonucleotides purified from a nucleic acid restriction digest or produced synthetically using any suitable method, which methods are known to those skilled in the art. The primers used herein can be synthesized using automated methods.

Because a mutation can occur in both the exon itself and the splice junction, it is necessary to design primers that will ensure that the entire exon region to be analyzed is amplified. To amplify the entire exon, the oligonucleotide primer for any given exon must be designed such that it includes a portion of the complementary sequence for the promoter region, for the 3'-noncoding region, or for the introns flanking the exon to be amplified, provided however that the primer sequence should not include the sequence for the splice signal dinucleotides. It is important to exclude the complementary sequence for the splice signal dinucleotides from the primer in order to ensure that the entire region, including the splice signal dinucleotide, is amplified. Including the complementary sequences to the splice signal dinucleotides could result in an amplification product that "plasters over" the splice junction and masks any potential mutation that could occur therein. It should be noted, however, that the introns flanking the exon are not limited to the introns immediately adjacent to the exon to be amplified. The oligonucleotide primer can be designed such that it includes a portion of the complementary sequence for the introns upstream or downstream from the exon to exon to be amplified. In the latter instance, the amplification product produced would include more than one exon. Preferably at least 20 to 25 nucleotides of the sequence for each flanking intron are included in the primer sequence.

The primers used herein are selected to be substantially complementary to each strand of the pRb2/p130 segment to be amplified. There must be sufficient base-pair matching to enable formation of a hybrid duplex under hybridization conditions. It is not required, however, that the base-pair matchings be exact. Therefore, the primer sequence may or may not reflect the exact sequence of the pRb2/p130 segment to be amplified. Non-complementary bases or longer sequences can be interspersed into the primer, provided the primer sequence retains sufficient complementarity with the segment to be amplified and thereby form an amplification product.

The primers must be sufficiently long to prime the synthesis of amplification products in the presence of a polymerizing agent. The exact length of the primer to be used is dependent on many factors including, but not limited to, temperature and the source of the primer. Preferably the primer is comprised of 15 to 30 nucleotides, more preferably 18 to 27 nucleotides, and most preferably 24 to 25 nucleotides. Shorter primers generally require cooler annealing temperatures with which to form a stable hybrid complex with the template.

Primer pairs are usually the same length, however, the length of some primers was altered to obtain primer pairs with identical annealing temperatures. Primers of less than 15 bp are generally considered to generate non-specific amplification products.

According to one embodiment of this invention, SSCP is used to analyze polymorphisms and mutations in the exons of pRb2/p130. SSCP has the advantages over direct sequencing in that it is simple, fast, and efficient. The analysis is performed according to the method of Orita et al., Genomics 5:874-879 (1989), the entire disclosure of which is incorporated herein by reference. The target sequence is amplified and labeled simultaneously by the use of PCR with radioactively labeled primers or deoxynucleotides. Neither *in situ* hybridization nor the use of restriction enzymes is necessary for SSCP.

SSCP detects sequence changes, including single-base substitutions (point mutations), as shifts in the electrophoretic mobility of a molecule within a gel matrix. A single nucleotide difference between two similar sequences is sufficient to alter the folded structure of one relative to the other. This conformational change is detected by the appearance of a band shift in the tumor DNA, when compared with the banding pattern for a corresponding wild type DNA segment. Single base pair mutations can be detected following SSCP analysis of PCR products up to about 400 bp. PCR products larger than this size must first be digested with a restriction enzyme to produce smaller fragments.

In another embodiment of the invention, sequence mutations in pRb2/p130 can be detected utilizing the PRINS technique. The PRINS method represents a versatile technique, which combines the accuracy of molecular and cytogenetic techniques, to provide a physical localization of the genes in nuclei and chromosomes. *See* Cinti et al., Nuc. Acids Res. Vol 21, No. 24: 5799-5800 (1993), the entire disclosure of which is incorporated herein by reference. The PRINS technique is based on the sequence specific annealing of unlabeled oligodeoxynucleotides *in situ*. The oligodeoxynucleotides operate as a primer for *in situ* chain elongation catalyzed by *Taq* I polymerase. Labeled nucleotides, labeled with a substance such as biotin or Digoxigenin, act as substrate for chain elongation. The labeled DNA chain is visualized by exposure to a fluorochrome-conjugated antibody specific for the label substance. Preferably, the label is Digoxigenin and the fluorochrome conjugated antibody is anti-Digoxigenin-FITC. This results in the incorporation of a number of labeled nucleotides far greater than the number of nucleotides in the primer itself. Additionally, the specificity of the hybridization is not vulnerable to the problems that arise when labeled nucleotides are placed in the primer. The bound label will only be found in those places where the primer is annealed and elongated.

Neither the SSCP nor the PRINS technique will characterize the specific nature of the polymorphism or mutation detected. If a band shift is detected through use of SSCP analysis, one must still sequence the sample segment and compare the sequence to that of the corresponding wild type pRb2/p130 segment. Similarly, if the absence of one or both of the alleles for a given exon segment is detected by the PRINS technique, the sequence of the segment must be determined and compared to the nucleotide sequence for the corresponding wild type in order to determine the exact location and nature of the mutation, i.e., point mutation, deletion or insertion. The PRINS technique is not capable of detecting polymorphisms.

Protocols for the use of the SSCP analysis and the PRINS technique are included in the examples that follow.

The PRINS method of detecting mutations in the pRb2/p 130 gene may be practiced in kit form. In such an embodiment, a carrier is compartmentalized to receive one or more containers, such as vials or test tubes, in close confinement. A first container may contain one or more subcontainers, segments or divisions to hold a DNA sample for drying, dehydrating or denaturing. A second container may contain the PRINS reaction mixture, which mixture is comprised of a PCR buffer, a DIG DNA labeling mixture, a polymerase such as Taq I DNA polymerase, and the primers designed in accordance with this invention (see Example 7, Table 8). The DIG DNA labeling mixture is comprised of a mixture of labeled and unlabeled deoxynucleotides. Preferably, the labeled nucleotides are labeled with either biotin or Digoxigenin. More preferably, the label is Digoxigenin. A third container may contain a fluorochrome conjugated antibody specific to the label. The fluorochrome conjugated antibody specific for Digoxigenin is anti-Digoxigenin-FITC. Suitable conjugated fluorochromes for biotin include avidin-FITC or avidin Texas Red. The fourth container may contain a staining compound, preferably Propidium Iodide (PI). The kit may further contain appropriate washing and dilution solutions.

### Examples

The following examples illustrate the invention. These examples are illustrative only, and do not limit the scope of the invention.

### Example 1

### Expression of pRb2/p130 in Endometrial Carcinoma

### A. Patients and Tumors

Between September 1988 and December 1994, 196 patients with previously untreated endometrial carcinoma were seen at the Department of Obstetrics and Gynecology, University of Florence, Italy. To avoid concern for the possibility radiation affecting molecular analyses, the patients who received preoperative irradiation were excluded. In 175 cases surgery was the first treatment. Paraffin-embedded tissue blocks containing the most representative portion of the tumor were available in 104 of these cases; four patients were lost to follow up, leaving a total of 100 patients. Patients' ages ranged from 46 to 84 years with a median age of 64 years. Histologic slides were reviewed to assess histologic type, grade of differentiation and depth of myometrial invasion. The stage was evaluated by microscopic analysis of the surgical specimen according to the 1988 International Federation of Gynecology and Obstetrics (FIGO) classification (*Gynecol Oncol* 35: 125 (**1988**). Table 1 summarizes the clinical and pathological characteristics of the study group.

### B. Surgical Treatment

Surgical treatment included total hysterectomy in 95 cases and extended hysterectomy in five cases. Bilateral salpingo-oophorectomy was always associated. Pelvic and paraaortic lymphadenectomy were performed at the surgeon's discretion, but not systematically. Overall. 43 patients underwent lymphadenectomy. The omentum was removed when appropriate (four cases).

**Table 1. Clinical And Pathological Features Of 100 Patients In Which pRb2/p130 Expression Was Tested.**

| **Feature** | **Number of Patients** |
|---|---|
| Age | |
| < 65 yr | 52 |
| ≥ 65 yr | 48 |

| FIGO stage | |
|---|---|
| I | 68 |
| II | 15 |
| III | 14 |
| IV | 3 |

| Histologic type | |
|---|---|
| Adenocarcinoma | 74 |
| Adenosquamous | 17 |
| Adenoacanthoma | 4 |
| Papillary serous | 4 |
| Clear cell | 1 |

| Grade of differentiation | |
|---|---|
| Well differentiated | 44 |
| Moderately differentiated | 26 |
| Poorly differentiated | 25 |
| Not evaluable | 5 |

| Depth of myometrial invasion | |
|---|---|
| ≤ 50 % | 41 |
| > 50% | 59 |

| Adjuvant treatment | |
|---|---|
| None | 57 |
| Radiotherapy | 37 |
| Chemotherapy | 6 |

### C. Tumor Specimen Collection

For all 100 patients, a tumor specimen was taken fresh from a site regarded to be representative of the lesion immediately after hysterectomy. Each tumor sample was later divided into two parts: one for flow cytometry and the other for histological analysis.

### D. Adjuvant Therapy

Forty-three of the 100 patients received adjuvant treatment. Of the 43 patients receiving adjuvant treatment, 37 received radiotherapy and 6 received chemotherapy Poor grade of differentiation, deep myometrial invasion (> 50 percent) and tumor outside the uterine corpus (stage > I) were the major criteria for receiving adjuvant treatment. The irradiated patients (37 patients) received 56Gy on the whole pelvis. Chemotherapy (six patients) was given, when possible, in cases with more advanced disease (stage III-IV). The chemotherapy regimen included cisplatin (60 mg per square meter of body surface area) in combination with cyclophosphamide (600 mg per square meter of body surface area) and epirubicin (60 mg per square meter of body surface area), every 21 days, for six cycles.

### E. Follow-up And Evaluation Of Results

After completing the treatment, patients were seen every three months for the first two years, every four months during the third and fourth years, and every six months thereafter. Recurrence was considered as any documented relapse of the tumor either in the pelvis or systemic. Disease-free interval was calculated from the date of the operation. Patients with residual disease after surgery or who recurred within three months from the date of the operation were not considered free of disease and therefore excluded from the disease-free analysis, but not from the actuarial survival calculation. Patients with deaths from causes other than endometrial cancer were considered as lost to follow-up and therefore-their survival times were censored at the date of death. Follow-up data were available for all 100 patients, with a median of 48 months (range 20 to 86 months). Disease-free interval and actuarial survival were the end-points of the study.

### F. Flow Cytometric Analysis Of DNA Index

For flow cytometry, a suspension of tumor cells was obtained by mincing the sample with a lancet and scissors in phosphate-buffered saline. The cell suspension was filtered by a 50 micrometer mesh of polyacrylamide, fixed in 70 percent ethanol, and stored at -4°C until assayed. Prior to DNA analysis the ethanol was removed by centriguation (1500 revolutions/min for ten minutes); the pellet was then resuspended and washed twice in phosphate-buffered saline. The RNA was removed by digestion with ribonuclease (Serva, 0.1 mg/ml in phosphate-buffered saline) for 30 minutes at 37°C. the nuclei were washed in phosphate-buffered saline, and DNA was stained with 40 mg propidium iodide (Becton Dickinson) and I gm sodium citrate per liter in distilled water. Human female lymphocytes were added to the samples before enzymatic treatment and staining, and they were used as the DNA diploid standard. The DNA analyses were performed with an Elite flow cytometer (Coulter Corporation, Hialeah, Fla.) provided with a 15 mW Argon laser, at a wavelength of 488 mm Data were expressed as DNA histograms. The DNA ploidy was given by the DNA index, defined as a proportion of the modal DNA values of the tumor G₀ and G₁ cells (peak channel) to the DNA content of the diploid standard. The histograms were based on measurement of more than 10,000 cells and resulted, in general, in a good resolution with a coefficient of variation of three to six percent. Calculation of DNA index was done by processing each histogram in the computer-assisted program Multicycle Autofit, version 2.00 (Phoenix Flow Systems, San Diego, CA).

All cases with DNA index value of 1 (±0.04) were classified as diploid and others as aneuploid.

### G. Antibody

Rabbit polyclonal immune serum, designated ADL1, was prepared against pRb2/p130 according to the procedure of Harlow and Lane, Antibodies: A Laboratory Manual, Cold Spring Laboratory Press (1988), Chapter 5, the disclosure of which is incorporated herein by reference. Rabbits were immunized with a conjugate comprising the peptide Glu-Asn-His-Ser-Ala-Leu-Leu-Arg-Arg-Leu-Gln-Asp-Val-Ala-Asn-Asp-Arg-Gly-Ser-His-Cys (SEQ ID NO:3) coupled to keyhole limpet hemocyanin (KLH).. The peptide corresponds to the carboxy terminus of the pRb2/p130 protein. Briefly, rabbits were immunized with the SEQ ID NO:3-KLH conjugate by subcutaneous injection once every two weeks until a total of three injections were given. The initial injection (primary immunization) comprised 1 mg SEQ ID NO:3-KLH conjugate in 500 µl PBS, plus 500 µl of complete Freund's adjuvant. The second and third injections (boosts) comprised 500 µg of the conjugate in 500 µl PBS, plus 500 µl of complete Freund's adjuvant. The rabbits were bled after the third injection. Subsequent boosts, with the same composition as the second and third injections, were given once a month.

### H. Immunohistochemical Analysis

Sections of each tumor specimen were cut to 5-micrometer, mounted on glass and dried overnight at 37°C. All sections were then deparaffinized in xylene, rehydrated through graded alcohol series and washed in phosphate-buffered saline. This buffer was used for all subsequent washes and for the dilution of the antibodies. Sections were quenched in 0.5 percent hydrogen peroxide and blocked with diluted ten percent normal goat anti-rabbit serum. Slides were then incubated for one hour at room temperature with the ADL1 immune serum at a dilution of 1:1000, then incubated with diluted goat anti-rabbit biotinylated antibody (Vector, Burlingame, Calif.) for 30 minutes at room temperature. After washing in phosphate-buffered saline, the slides were processed by the ABC method (Vector) for 30 minutes at room temperature. Diaminobenzidine (Sigma, St. Louis) was used as the final chromogen, and hematoxylin as the nuclear counterstain. Negative controls for each tissue section consisted of substitution of the primary antibody with the corresponding pre-immune serum. Moreover, preincubation of the antibody with an excess of the corresponding immunizing antigen, blocked the immunocytochemical reaction, thus confirming the specificity of the ADL1 antibody for pRb2/p130 (data not shown).

All the samples were processed under the same conditions. In each experiment, normal uterine tissue was also included as a control. The results of pRb2/p130 immunostaining were independently interpreted by three observers who had no previous knowledge of the clinical outcome of each patient. The level of concordance, expressed as the percentage of agreement between the observers was 90 percent (90 of 100 specimens). In the remaining specimens the score was obtained from the opinions of the two investigators in agreement. The results were expressed as percentage of positive cells. In each tumor sample, at least 20 high power fields were randomly chosen and 2.000 cells were counted. The pRb2/p130 immunostaining was mostly nuclear, but a few specimens also exhibited cytoplasmatic staining. This pattern of immunoreactivity could be referred to microstructural alterations caused by the fixing and embedding procedures, or might reflect differences in the levels of expression and in the localization of this antigen during the various phases of the cell cycle, as has already been shown at the molecular level (Claudio et al., Cancer Res 56: 2003-8 (1996**).**

### I. Cellular Reactivity Cutoff Point

To evaluate the prognostic value of pRb2/p130 expression, the patients' disease-free and actuarial survival durations were compared after dividing them into two groups using different cutoff points of percent pRb2/p130 positivity. The P values were significant for poor disease-free and actuarial survival when a cutoff point of 40 percent or fewer reactive cells was used (P = 0.003 and P <0.001, respectively). The level of significance decreased to P = 0.02 and P = 0.01, respectively, with a cutoff point of 50 percent positivity and became insignificant with a cutoff point of 60 percent or higher positivity. Consequently, subsequent survival analyses were carried out using a 40 percent reactivity cutoff point. A similar approach to identify optimal cutoff points has been used in immunohistochemical studies utilizing p53 expression and bcl-2 expression (Shim et al., J Natl Cancer Inst 88: 519-29 (1996**);** Silvestrini et al., J Clin Oncol 14: 1604-10 (1996**)).**

### J. Statistical Analysis

Fisher's exact test was used to evaluate the association between pRb2/p130 expression and the other prognostic variables (Fienberg, The Analysis Of Cross-Classified Categorical Data, MIT Press, Cambridge, Mass.: Zelterman et al., "Contingency Tables In Medical Studies". NEJM Books 293-310 (1992)). Disease-free interval and actuarial survival were calculated according to the Kaplan-Meier method (Kaplan et al., Am Stat Assoc 53: 457-81 (1958)) and evaluated by the log-rank test (Miller, Survival Analysis. pp. 44-102. John Wiley, New York (1981)). Univariate Cox analysis was used to assess the effect of each prognostic variable on disease-free interval and survival. A multivariate analysis (Cox proportional-hazards regression, with forward selection of variables) (Cox, J R Stat Soc 34: 187-220 (1972)) was performed to estimate which of the possible risk factors yielded independent prognostic information. Data analysis was performed with the SPSS statistical package, release 5.0.1 (SPSS Inc., Chicago, IL).

### K. Results

A brown stain indicated the presence of pRb2/p130 in tumor cells. The specimens were characterized as having no detectable staining, staining in only a few positive cells (about ten percent), staining in more than 40 percent of the cells, or intense staining in the majority of cells. Tumors with immunostaining in more than 40 percent of cells were considered to be positive for pRb2/p130.

In normal uterine samples, strong immunoreactivity was detected for pRb2/p130 in all endometrial and endocervical epithelial cells. Of the 100 endometrial adenocarcinomas examined, five showed immunoreactivity for pRb2/p130 in 20 percent or fewer cells, 15 had reactivity in 30 percent of the cells and nine had staining in 40 percent of the cells. These. 29 tumors (29 percent) were considered pRb2/p130 negative. The remaining 71 tumors were scored as 50 percent positivity in 11 cases, as 60 percent positivity in 49 cases and with staining in over 70 percent of the cells in four cases. These 71 tumors (71 percent) were considered pRb2/p130 positive.

The DNA index values showed a diploid type in 73 cases and an aneuploid type in 27 cases. The DNA index of the aneuploid tumors was hypodiploid in one case, hypertetraploid in four cases; the remaining 22 cases had a modal DNA content in the diploid to tetraploid range (1 < DNA index < 2).

### L. Association Of pRb2/p130 Expression With Clinical And Pathological Features.

The expression of pRb2/p130 was inversely correlated with patients' age: in patients younger than 65 years pRb2/p130 negative tumors were nine of 52 (17.3 percent) in contrast with 20 of 48 in patients aged 65 years or older (41.6 percent) (P = 0.008). Immunostaining for pRb2/p130 was more frequently negative among patients with aneuploid tumors (13 of 27; 48.1 percent) than among those with a diploid pattern (16 of 73; 21.9 percent) (P = 0.001). Tumors negative for pRb2/p130 were more frequent among patients with poorly or moderately differentiated carcinomas, but this association was not statistically significant (P = 0.06). The level of expression of pRb2/p130 did not differ significantly between patients with tumors limited to the uterine corpus (stage I) and those in whom the tumor had spreads outside the corpus uteri (stage > I), (P = 0.4). No significant difference in the incidence of pRb2/p130 negativity was found among the histologic types, nor among patients with different degrees of myometrial infiltration.

Expression of pRb2/p130, tumor ploidy, FIGO stage and grade of differentiation were significantly correlated with disease-free interval and actuarial survival, by Univariate Cox analysis, as shown in Table 2.
Other clinico-pathological features, including age, histologic type and depth of myometrial invasion were not associated with the outcome (data not shown).

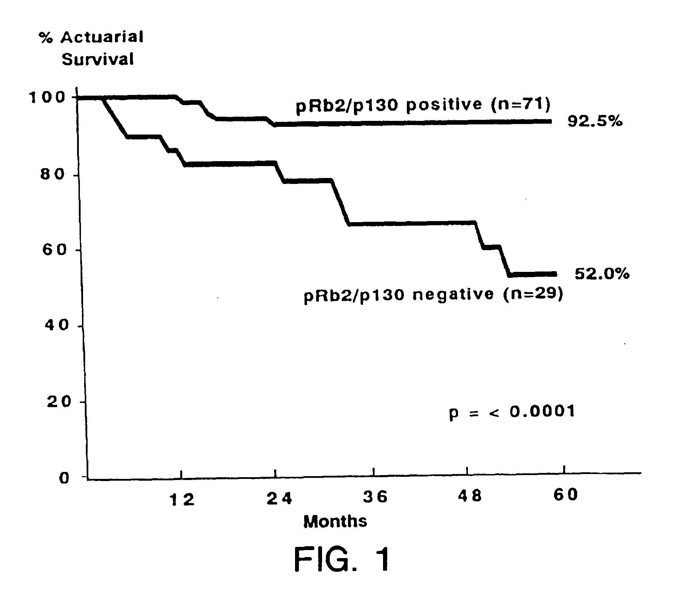

As shown in Figure 1, patients with pRb2/p130 negative tumors had a significantly reduced disease-free interval and survival (P=0.001 and P < 0.0001, respectively); the five-year survival probability was 52.0 percent in patients with such tumors, in contrast with 92.5 percent in patients with pRb2/p130 positive tumors.

**Table 2. Significant Predictors Of Clinical Outcome In 100 Patients With Endometrial Carcinoma, According To Cox Univariate Analysis For Disease-free Interval And Actuarial Survival.**

| **Variable** | **Recurrence Rate Ratio** | **95% Confidence Interval** | **P Value** | **Death Rate Ratio** | **95% Cl+** | **P Value** |
|---|---|---|---|---|---|---|
| **pRb2/p130** | | | | | | |
| positive | 1 | | | 1 | | |
| negative | 4.83 | 1.70 - 13.64 | 0.003 | 6.68 | 2.32 - 19.27 | <0.0001 |

| **FIGO stage** | | | | | | |
|---|---|---|---|---|---|---|
| I | 1 | | | | 1 | |
| > 1 | 5.42 | 1.86 - 15.77 | 0.002 | 5.08 | 1.78 - 14.51 | 0.002 |

| **Ploidy status** | | | | | | |
|---|---|---|---|---|---|---|
| diploid | 1 | | | 1 | | |
| aneuploid | 3.43 | 1.24 - 9.51 | 0.01 | 5.94 | 2.14 - 16.42 | <0.001 |
| **Grade of differentiation** (1 = well differentiated, 2= moderately differentiated, 3 = poorly differentiated) | | | | | | |
| 1 | 1 | | | 1 | | |
| 2 | 7.73 | 1.54 - 38.67 | 0.01 | 13.88 | 1.65 -116.27 | 0.01 |
| 3 | 7.45 | 1.43 - 38.78 | 0.01 | 18.36 | 2.23 -151.10 | 0.007 |

Table 3 shows the results of Cox proportional-hazards regression analysis in which the response to pRb2/p130 immunostaining, tumor ploidy, FIGO stage and grade of differentiation were tested simultaneously to estimate the rate ratios for the occurrence of death from disease in patients with endometrial cancer. Negative immunostaining for pRb2/p130 resulted as the strongest independent predictor of poor outcome. Patents with pRb2/p130 negative tumors had a significantly higher rate ratio for dying due to disease (4.91) than patients with pRb2/p130 positive tumors. Multivariate analysis revealed that tumor spread outside the corpus uteri (stage > I) and aneuploidy were also associated with a higher probability of death from disease, whereas grade of differentiation yielded no independent prognostic information. By the combined use of pRb2/p130 expression and FIGO stage, a more accurate definition of risk of death was possible.

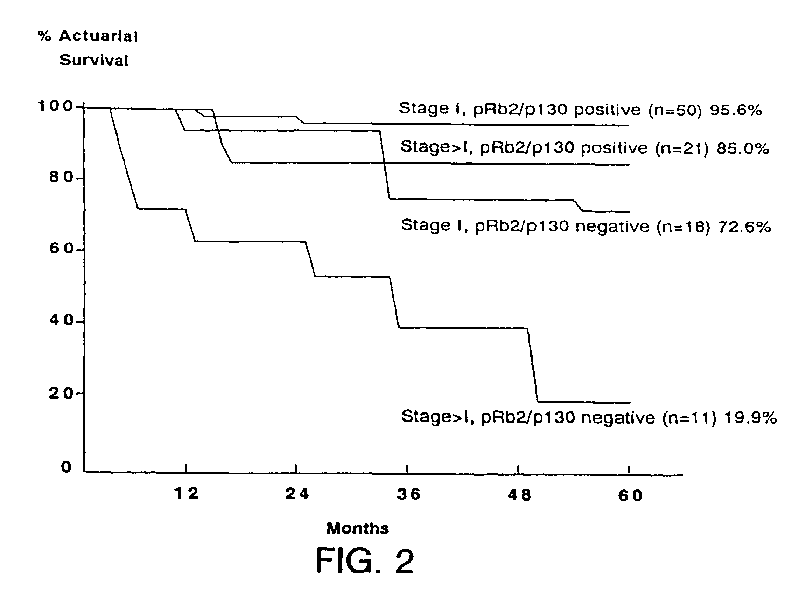

Figure 2 presents Kaplan Meier survival estimates according to these stratified risk groups. The following is the comparison between the groups by the log-rank test:
Stage I, pRb2/p130-Positive versus Stage > I, pRb2/p130-Positive: difference not significant;
Stage I, pRb2/p130-Positive versus Stage I, pRb2/p130-Negative: P = 0.01; Stage I, pRb2/p130-Negative versus Stage > I, pRb2/p130-Negative:
P = 0.005;
Stage > I, pRb2/p130-Positive versus Stage > I, pRb2/p130-Negative:
P = 0.003;
Stage > I, pRb2/p130-Positive versus Stage I, pRb2/p130-Negative: difference not significant.

**Table 3. Results Of Cox Proportional-Hazards Regression Analysis For Survival Data.**

| **Variable** | **Rate Ratio** | **95% Confidence Interval** | **P Value*** |
|---|---|---|---|
| pRb2/p 130 | | | |
| positive | 1 | | |
| negative | 4.91 | 1.66 - 14.54 | 0.004 |

| FIGO stage | | | |
|---|---|---|---|
| I | 1 | | |
| > I | 4.18 | 1.43 - 12.23 | 0.009 |

| Ploidy status | | | |
|---|---|---|---|
| Diploid | 1 | | |
| Aneuploid | 3.36 | 1.17 - 9.62 | 0.02 |

| | | | |
|---|---|---|---|
| * Chi-square of the model, P < 0.001 | | | |

### Example 2

### Expression of pRb2/p130 in Ovarian Cancer

### A. Tumors

Sixty archived (formalin fixed and paraffin-embedded) epithelial carcinoma specimens were obtained from the Department of Pathology at Pennsylvania Hospital. The specimens included Grade 1, Grade 2, and Grade 3 tumors.

### B. Immunohistochemistry

Immunohistochemical staining was performed using an automated immunostainer (Ventana ES, Ventana Medical Systems, Tucson. AZ) and a Peroxidase-DAB immunodetection kit (Ventana Medical Systems). Five micron sections were cut from each tumor specimen. The sections were mounted on slides and air-dried. The sections were deparaffinized in xylene and hydrated through a graded alcohol series into water. A polyclonal anti-RB2 primary antibody was applied at a dilution of 1:500 for 30 minutes at 37°C. The slides were then incubated with a biotinylated goat anti-rabbit antibody for 30 minutes. The slides were then incubated with a horseradish peroxidase conjugated-avidin. Hydrogen peroxide was used as the oxidizing substrate, and diaminobenzidine (DAB) was used as the chromagen. The slides were counterstained with hematoxylin, dehydrated, and mounted. The intensity of pRb2/p130 immunostaining was evaluated.

### C. Results

The preliminary results are shown in Table 4. These results suggest that as the grade of tumor increases, less expression of the pRb2/p130 protein is detected. The pRb2/p130 expression level may therefore be useful in grading and as a prognostic indicator in human epithelial ovarian cancer.

**Table 4. Immunohistochemical Detection Of pRb2/p130 In Human Epithelial Ovarian Carcinoma Specimens**

| **Grade of Tumor** | **Intensity of Immunostaining** | | | |
|---|---|---|---|---|
| | Negative | + | ++ | +++ |
| Grade 1 | 20% | 40% | 40% | 0% |
| Grade 2 | 50% | 33% | 17% | 0% |
| Grade 3 | 37% | 26% | 23% | 14% |

### Example 3

### Expression of pRb2/p130 in Lung Cancer, Series I

### A. Antibody Against pRb2/p130

The rabbit polyclonal immune serum designated ADL1. as described in Example 1G, was used in these studies.

### B. Antibody Against p107

Rabbit polyclonal immune serum was prepared against p107 (ADL2) by immunizing rabbits with a bacterially expressed GST-p107 fusion protein. Expression of the fusion protein was performed according to the procedure reported by Smith and Johnson, Gene 67:31-40 (1988) and Frangioni and Neel, Anal. Biochem. 210:179-187 (1993). Rabbits were immunized with the fusion protein by subcutaneous injection once every two weeks until a total of three injections were given. The initial injection (primary immunization) comprised 500 µg protein in 500 µl PBS, plus 500 µl of incomplete Freund's adjuvant. The second and third injections (boosts) comprised 100 µg of the protein in 500 µl PBS, plus 500 µl of incomplete Freund's adjuvant. The rabbits were bled after the third injection. Subsequent boosts, with the same composition as the second and third injections, were given once a month.

### C . Antibody Against pRb/p105

An anti-pRb/p105 monoclonal antibody (XZ 77), prepared as described by Hu et al., Mol. Cell. Biol. 11:5792-5799 (1991), was used in these studies.

### D. Tissue Samples

Lung tissue specimens from 51 patients with surgically resected lung cancer were obtained from patients who had not received chemo- or radiotherapy prior to surgical resection. The samples consisted of 39 squamous cell carcinomas and 12 adenocarcinomas. Histological diagnosis and grading were performed by a skilled lung pathologist. Samples were graded on the scale of 1-2-3 with "3" representing the most malignant disease and "1" representing the least malignant disease. Normal lung tissue samples containing the stratified columnar epithelia of trachea, bronchi and adjacent glands were obtained either from biopsy or autopsy performed within 10 hours of the patient's death.

### E. Immunohistochemistry

Sections from each lung tissue specimen were cut at 3-5 µm, mounted on glass and dried overnight at 37°C. All sections were then deparaffinized in xylene, rehydrated through a graded alcohol series and washed in phosphate-buffered saline (PBS). The same buffer was used for all subsequent washes and for dilution of antibodies.

Tissue sections for pRb2/p130 and p107 detection were sequentially quenched in 0.5% hydrogen peroxide and blocked with diluted 10% normal goat anti-rabbit serum (Vector Laboratories). The slides were incubated for 1 hour at room temperature with the rabbit polyclonal immune serum (ADL1) raised against pRb2/p 130 at a dilution of 1:2000, or the ADL2 antibody against p107 at a dilution of 1:500. The slides were then incubated with diluted goat anti-rabbit biotinylated antibody (Vector Laboratories) for 30 minutes at room temperature.

Sections for pRb/p105 detection were heated twice in a microwave oven for 5 min each at 700 W in citrate buffer (pH6), were quenched sequentially in 0.5% hydrogen peroxide, and were blocked with diluted 10% normal horse anti-mouse serum (Vector Laboratories. Inc.) The monoclonal mouse anti-human pRb/p105 antibody XZ77 (at a dilution of 1:500) was added and incubated for 120 min. at room temperature. After being washed in PBS, the slides were incubated with diluted horse anti-mouse biotinylated antibody (Vector Laboratories, Inc.) for 30 min. at room temperature.

Slides were processed by the so-called "ABC" method according to the instructions of the biotinylated antibody manufacturer (Vector Laboratories) for 30 minutes at room temperature. Diaminobenzidine was used as the final chromagen, and hematoxylin as a nuclear counterstain. Negative controls for each tissue section consisted of substitution of the primary antibody with pre-immune serum for ADL1 and ADL2, or leaving out the primary antibody for XZ77.

Three pathologists scored the expression of pRb2/p130 protein as the percentage of positively stained nuclei on a scale of 0-1-2-3: 0 = undetectable level of expression; 1 = low expression level (1-30% cells stained positive); 2 = medium expression level (30-60% cells stained positive); 3 = high expression level (60-100% cells stained positive). The normal lung tissue samples comprising the stratified epithelia of the trachea, bronchi and adjacent glands were strongly stained, indicating a high expression level.

### F. Results

The results are shown in Table 5.

**TABLE 5**

| **Sample No.** | **Type** | **Grading** | **pRb2/p130 Level** | **p107 Level** | **pRb/p105 Level** |
|---|---|---|---|---|---|
| 1 | squamous | 3 | 0 | 2 | 3 |
| 2 | squamous | 2 | 3 | 1 | 3 |
| 3 | squamous | 1 | 3 | 1 | 3 |
| 4 | squamous | 1 | 3 | 1 | 3 |
| 5 | squamous | 2 | 2 | 1 | 2 |
| 6 | squamous | 2 | 3 | 1 | 2 |
| 7 | squamous | 3 | 1 | 1 | 3 |
| 8 | squamous | 2 | 3 | 1 | 2 |
| 9 | squamous | 2 | 1 | 1 | 2 |
| 10 | squamous | 2 | 3 | 1 | 1 |
| 11 | squamous | 2 | 3 | 1 | 2 |
| 12 | squamous | 1 | 3 | 1 | 3 |
| 13 | squamous | 3 | 1 | 1 | 1 |
| 14 | squamous | 1 | 3 | 1 | 3 |
| 15 | squamous | 3 | 0 | 2 | 3 |
| 16 | squamous | 2 | 2 | 1 | 2 |
| 17 | squamous | 2 | 3 | 1 | 2 |
| 18 | squamous | 2 | 1 | 1 | 2 |
| 19 | squamous | 1 | 3 | 1 | 3 |
| 20 | squamous | 3 | 1 | 1 | 1 |
| 21 | squamous | 2 | 3 | 1 | 2 |
| 22 | squamous | 3 | 2 | 1 | 3 |
| 23 | squamous | 2 | 3 | 1 | 3 |
| 24 | squamous | 2 | 3 | 1 | 1 |
| 25 | squamous | 2 | 3 | 1 | 2 |
| 26 | squamous | 1 | 3 | 1 | 3 |
| 27 | squamous | 3 | 1 | 2 | 3 |
| 28 | squamous | 2 | 3 | 1 | 3 |
| 29 | squamous | 1 | 3 | 1 | 3 |
| 30 | squamous | 1 | 3 | 1 | 3 |
| 31 | squamous | 2 | 2 | 1 | 2 |
| 32 | squamous | 2 | 3 | 1 | 2 |
| 33 | squamous | 3 | 3 | 1 | 3 |
| 34 | squamous | 2 | 3 | 1 | 2 |
| 35 | squamous | 2 | 0 | 1 | 2 |
| 36 | squamous | 2 | 3 | 1 | 1 |
| 37 | squamous | 2 | 3 | 1 | 2 |
| 38 | squamous | 1 | 3 | 1 | 3 |
| 39 | squamous | 3 | 1 | 1 | 0 |
| 40 | adenocarcinoma | 3 | 0 | 2 | 2 |
| 41 | adenocarcinoma | 1 | 2 | 1 | 2 |
| 42 | adenocarcinoma | 2 | 1 | 2 | 1 |
| 43 | adenocarcinoma | 2 | 1 | 1 | 2 |
| 44 | adenocarcinoma | 2 | 0 | 2 | 1 |
| 45 | adenocarcinoma | 2 | 1 | 1 | 2 |
| 46 | adenocarcinoma | 1 | 2 | 1 | 2 |
| 47 | adenocarcinoma | 3 | 0 | 2 | 2 |
| 48 | adenocarcinoma | 1 | 2 | 1 | 2 |
| 49 | adenocarcinoma | 3 | 0 | 2 | 2 |
| 50 | adenocarcinoma | 2 | 1 | 2 | 1 |
| 51 | adenocarcinoma | 2 | 0 | 1 | 2 |

### Statistical Analysis

The data from Table 5 were analyzed using the Jonkheere-Terpstra test and STATXACT statistical software (Cytel Software Corp., Cambridge, MA) determine whether there is a relationship between tissue grade and protein expression level.

A statistically significant inverse relationship was found between the pathological grading and the expression of pRb2/p130 in squamous cell carcinomas (p < .0001) and adenocarcinomas (p < .004).

Although a statistically significant inverse relationship was found between pathological grading and the expression of pRb/p105 in squamous cell carcinomas (p=0.004), no such relationship was found between pRb/p105 expression and grading of adenocarcinomas.

### Example 4

### Expression of pRb2/p130 in Lung Cancer, Series II

### A. Lung Cancer Specimens

One hundred and fifty eight lung cancer specimens were obtained from patients that underwent a surgical resection (lobectomy or pneumonectomy) in the Departments of Thoracic Surgery of the V. Monaldi Hospital and of the II University of Naples (Italy) between January 1995 and April 1996. Specimens were obtained only from patients who had not received chemo- or radiotherapy prior to surgical resection.

The histological diagnoses and classifications of the tumors were based on the WHO criteria, and the postsurgical pathologic TNM stage was determined using the guidelines of the American Joint Committee on Cancer.

The routine histopathological evaluation of the 158 tumor specimens analyzed was performed independently of the pRb2/p130 immunostaining. Thirty two tumors were adenocarcinomas. 118 were squamous carcinomas. 4 were carcinoids and 4 were small cell lung cancers. Eighty seven tumors (55.1 %) were classified as stage I. 43 tumors (27.1%) were classified as stage II and 28 tumors (17.7%) were classified as stage IIIa. The adenocarcinomas and squamous carcinomas were classified by grade, as shown in Table 6.

### B. Immunohistochemistry

Sections of each specimen were cut at 3-5 µm, mounted on glass and dried overnight at 37°C. All the sections were then deparaffinized in xylene, rehydrated through a graded alcohol series and washed in PBS. This buffer was used for all subsequent washes and for the dilution of the antibodies. Sections were heated twice in a microwave oven for five minutes each at 700 W in citrate buffer (pH 6), sequentially quenched in 0.5% hydrogen peroxide and blocked with diluted 10% normal goat anti-rabbit serum. Slides were then incubated for one hour at room temperature with rabbit polyclonal immune serum raised against pRb2/p130 at a dilution ranging from 1:500 to 1:1500, then incubated with diluted goat anti-rabbit biotinylated antibody (Vector Laboratories) for 30 minutes at room temperature. After washings in PBS, the slides were processed by the ABC method (Vector Laboratories) for 30 minutes at room temperature. Diaminobenzidine was used as the final chromogen, and hematoxylin as the nuclear counterstain. Negative controls for each tissue section were obtained by substituting the primary antibody with pre-immune serum.

All samples were processed under the same conditions. Three pathologists (A. Baldi, G.G. Giordano and F. Baldi) evaluated the staining pattern of the protein separately and scored it for the percentage of positive nuclei: score 1, less than 10% of positive cells (low to undetectable level of expression); score 2, from 10% to 50% of positive cells (medium level of expression); score 3, more than 50% of positive cells (high level of expression). The level of concordance, expressed as the percentage of agreement between the observers was 90% (142 of 158 specimens). In the remaining specimens the score was obtained from the opinions of the two investigators in agreement. At least 20 high power fields were chosen randomly and 2000 cells were counted. This coded score was preferred to facilitate the statistical analyses.

### C. Statistical Analysis

Statistical analyses, using the chi square test, were performed to evaluate the significance of associations between the different variables of the considered tumors (histological type and grading, evidence of metastasis, pRb2/p130 expression levels). A p value < .05 was considered statistically significant.

### D. Results

pRb2/p130 immunostaining was mostly nuclear, but some specimens clearly exhibited cytoplasmatic staining with a low to absent background.

Immunohistochemical staining patterns of the tumors can be summarized as follows: 50 specimens (31.6%) showed low to undetectable levels of pRb2/p130 (score 1), 73 specimens (46.2%) exhibited medium pRb2/p130 expression levels, while high levels of expression were detected in 35 specimens (22.2%). The small number of small cell lung cancers and carcinoids included in this study did not allow statistical analysis in these histological groups. All the SCLCs specimens exhibited low to undetectable pRb2/p130 expression levels, while a high level of expression of this protein was recognized in all carcinoids.

Statistical analyses revealed that pRb2/p130 expression did not correlate with tumor stage or with TNM status (p = n.s.). However, a negative significant relationship was found between pRb2/p130 expression level and the histological grading (p<.0001). The correlation between histological grade and pRb2/p130 expression is shown in Table 6.

**TABLE 6**

| **Type** | **Grade** | **No.** | **pRb2/p130 Level** | | |
|---|---|---|---|---|---|
| | | | **1** | **2** | **3** |
| Squamous | 1 | 13 | 2 | 0 | 11 |
| Squamous | 2 | 42 | 8 | 28 | 6 |
| Squamous | 3 | 63 | 30 | 27 | 6 |
| Adenocarcinoma | 1 | 8 | 0 | 2 | 6 |
| Adenocarcinoma | 2 | 27 | 4 | 16 | 2 |
| Adenocarcinoma | 3 | 2 | 2 | 0 | 0 |

The mean follow-up period was too short to allow a detailed analysis of the disease free and the overall survival time of the patients. However, in looking at the development of metastasis in the patients, we found a significant inverse relationship between metastasis and the expression of pRb2/p130 (p<.0001).

### Example 5

### Isolation and Characterization of Genomic Clones

### A. Isolation of Genomic Clones

To isolate the entire human pRb2/p130 gene, a human P1 genomic library (Genome System Inc., St. Louis, MO) was screened by using two primers made from the published cDNA sequence, Li et al., Genes Dev. 7:2366-2377 (1993). The sequences for the primers used to isolate the genomic clones are GTATACCATTTAGCAGCTGTCCGCC (SEQ ID NO: 116) and the complement to the sequence GTGTGCCATTTATGTGATGGCAAAG (SEQ ID NO: 115).

One of the clones identified upon screening the P1 genomic library (clone no. 1437. Fig. 3B) was confirmed by Southern blot hybridization to contain a part of the pRb2/p130 gene. To obtain the additional 5' flanking sequence of the pRb2/p130 gene containing the putative promoter region, a human placenta genomic DNA phage library (EMBL3 SP6/T7) from Clontech, Palo Alto, CA was screened with a cDNA probe according to the method of Sambrook et al., Molecular Cloning:A Laboratory Manual, Second Edition, pp. 12.30-12.38, Cold Spring Harbor Laboratory Press, Cold Spring Harbor, NY (1989), the entire disclosure of which is incorporated herein by reference. The cDNA probe, labeled with [γ-³²P], corresponded to the first 430 bp after the start codon of the published cDNA sequence, Li et al., *supra*. Of the two positive clones obtained, one, identified as φSCR3 (Fig. 3B), was determined to contain the 5' flanking region of the pRb2/p130 gene.

### B. Identification of Exon/Intron Boundaries

To precisely characterize the position of the exons and the exon/intron boundaries in the genomic DNA, a set of oligonucleotide primers were used to sequence the genomic DNA clones. The primers were synthesized based upon the cDNA nucleotide sequence of pRb2/p130 such that they annealed to the genomic DNA at roughly 150 bp intervals. The exon/intron boundaries were identified from those positions in which the genomic DNA sequence differed from that of the published cDNA sequence.

### C. Sequencing of Clones

Sequencing of the recombinant clones was carried out in part by automated DNA sequencing using the dideoxy terminator reaction chemistry for sequence analysis on the Applied Biosystem Model 373A DNA sequencer and, in part, by using a dsDNA Cycle Sequencing System kit purchased from GIBCO BRL, Gaithersburg. MD, according to the instructions of the manufacturer.

### D. Synthesis of Oligonucleotide Primers

All oligonucleotide primers used herein were synthesized using Applied Biosystems DNA-RNA synthesizer Model 394, using beta-cyanoethyl phosphoramidite chemistry.

### E. Results of the Genomic Clones Characterization

The human pRb2/p130 gene consists of 22 exons and 21 introns and spans more than 50 kb of genomic DNA. The organization of these exons and introns are shown approximately to scale in Figure 3A. The location and size of each exon and intron of pRb2/p130, as well as the nucleotide sequences at the exon-intron boundaries are shown in Table 7 (SEQ ID NOS:6-47). The exons range in size from 65 to 1517 bp in length. The introns, which range in size from 82-9837 bp in length, have been completely sequenced. The nucleotide sequences are given as SEQ ID NOS:48-68.

### Example 6

### Characterization of Transcriptional Control Elements

### A. Cell Culture and RNA Extraction

The human HeLa (cervix epithelioid carcinoma) cell line was obtained from the American Type Culture Collection and maintained in culture in Dulbecco's modified Eagle medium (DHEM) with 10% fetal calf serum (FCS) at 37°C in a 10% CO₂-containing atmosphere. Cytoplasmatic RNA was extracted utilizing the RNAzol B method (CINNA/BIOTECX, Friendswood, TX).

**TABLE 7**

| | | | |
|---|---|---|---|
| Exon-Intron Boundaries of the Human pRb2/p130 Gene | | | |

| Exon No. (bp) | 5' Donor sequence | 3' Acceptor sequence | Intron No. (bp) |
|---|---|---|---|
| 1(240) | ACGCTGGAG³⁰⁹gtgcgctcgc (SEQ ID NO:6) | tcttttacag³¹⁰GGAAATGAT (SEQ ID NO:7 ) | 1 (4220) (SEQ ID NO:66) |
| 2(131) | AGAGCAGAG⁴⁴⁰gtaactatgt (SEQ ID NO:8) | ttaataccag⁴⁴¹CTTAATCGA (SEQ ID NO:9) | 2(3507) (SEQ ID NO:67) |
| 3(201) | GAAACAGCG⁶⁴¹gtaggttttc (SEQ ID NO: 10) | tcccccaaag⁶⁴²GCGACAGCC (SEQ ID NO: 11) | 3(3865) (SEQ ID NO:48) |
| 4(65) | ATGCAAAAG⁷⁰⁶gtaagaaaat (SEQ ID NO: 12) | aatcctgcag⁷⁰⁷GTAATTTCC (SEQ ID NO: 13) | 4(4576) (SEQ ID NO:49) |
| 5(129) | ATTTTAAAG⁸³⁵gtaggtttgt (SEQ ID NO: 14) | acaccatag⁸³⁶GCTTATCTG (SEQ ID NO: 15) | 5(1618) (SEQ ID NO:50) |
| 6(161) | GAAAAAAAG⁹⁹⁶gtttgtaagt (SEQ ID NO: 16) | ttcatcatag⁹⁹⁷CTCCTTAAG (SEQ ID NO: 17) | 6(92) (SEQ ID NO:51) |
| 7(65) | AGAGAGTTT¹⁰⁶¹gtgagtactt (SEQ ID NO: 18) | ttcctatag¹⁰⁶²TAAAGCCAT (SEQ ID NO:19) | 7(889) (SEQ ID NO:52) |
| 8(187) | TTTGACAAG¹²⁴⁸gtgagtttag (SEQ ID NO:20) | ttttctttag¹²⁴⁹TCCAAAGCA (SEQ ID NO:21) | 8(4586) (SEQ ID NO:53) |
| 9(167) | GATTCTCAG¹⁴¹⁵gttagtttga (SEQ ID NO:22) | ccttttttag¹⁴¹⁶GACATGTTC (SEQ ID NO:23) | 9(2127) (SEQ ID NO:54) |
| 10(90) | GTGCTAAAG¹⁵²⁵gtaattgtgc (SEQ ID NO:24) | atttctacag¹⁵²⁶AAATTGCCA (SEQ ID NO:25) | 10(716) (SEQ ID NO:55) |
| 11(104) | GATTTATCT¹⁶²⁹gtgagtaaaa (SEQ ID NO:26) | attttatag¹⁶³⁰GGTATTCTG (SEQ ID NO:27) | 11 (837) (SEQ ID NO:56) |
| 12(138) | TTTTATAAG¹⁷⁶⁷gtatttccca (SEQ ID NO:28) | tttatttcag¹⁷⁶⁸GTGATAGAA (SEQ ID NO:29) | 12(1081) (SEQ ID NO:57) |
| 13(165) | TGTGAAGAG¹⁹³²gtgaaaatca (SEQ ID NO:30) | tcttcatag¹⁹³³GTCATGCCA (SEQ ID NO:31) | 13(1455) (SEQ ID NO:58) |
| 14(112) | TTGGAAGGA²⁰⁴⁴gtaagtttaa (SEQ ID NO:32) | ttgacccctag²⁰⁴⁵GCATAACAT (SEQ ID NO:33) | 14(2741) (SEQ ID NO:59) |
| 15(270) | CTGTGCAAG²³¹⁴gtaaggaagg (SEQ ID NO:34) | ctgtcactag²³¹⁵GTATTGCCA (SEQ ID NO:35) | 15(197) (SEQ ID NO:60) |
| 16(281) | TTTAGAAAG²⁵⁹⁵gtaatttttc (SEQ ID NO:36) | tatctcctag²⁵⁹⁶GTATACCAT (SEQ ID NO:37) | 16(82) (SEQ ID NO:61) |
| 17(177) | ATGGCAAAG²⁷⁷²gtgagtacca (SEQ ID NO:38) | gtttgccag²⁷⁷³GTCACAAAA (SEQ ID NO:39) | 17(1079) (SEQ ID NO:62) |
| 18(72) | CGGAGCCAG²⁸⁴⁴gtaactacat (SEQ ID NO:40) | ttctctaaag²⁸⁴⁵GTGTATAGA (SEQ ID NO:41) | 18(659) (SEQ ID NO:63) |
| 19(107) | AAGATAGAA²⁹⁵⁰gtgggatctt (SEQ ID NO:42) | ctggctgcag²⁹⁵¹CCAGTAGAG (SEQ ID NO:43) | 19(572) (SEQ ID NO:64) |
| 20(202) | CAGGCAAAT³¹⁵³gtaagtatga (SEQ ID NO:44) | tttttaaacag³¹⁵⁴ATGGGATGC (SEQ ID NO:45) | 20(901) (SEQ ID NO:65) |
| 21(165) | CCTTCAAAG³³¹⁸gtgagcctaa (SEQ ID NO:46) | cccaccatag³³¹⁹AGACTGAGA (SEQ ID NO:47) | 21(9837) (SEQ ID NO:68) |
| 22(1517) | to the polyadenylation signal | | |

### B. Primer Extension Analysis

To characterize the pRb2/p130 promoter, a primer extension analysis was performed to locate the transcription initiation site. The primer for this analysis was an oligonucleotide, 5'ACCTCAGGTGAGGTGAGGGCCCGG 3' (SEQ ID NO:114), complementary to the pRb2/p130 genomic DNA sequence starting at position -22 (*See* Fig. 4, SEQ ID NO:4). The primer was end labeled with [γ³²P]ATP and hybridized overnight with 20 µg of HeLa cytoplasmatic RNA at 42°C. The primer-annealed RNA was converted into cDNA by avian myeloblastosis virus reverse transcriptase in the presence of 2 mM deoxynucleotides at 42°C for 45 minutes. The cDNA product was then analyzed on 7% sequencing gel containing 8 M urea. The position of the transcription start site was mapped from the length of the resulting extension product.

### C. SIGNAL SCAN Program

Several of the transcription factor-binding motifs were identified through the use of SIGNAL SCAN VERSION 4.0. SIGNAL SCAN is a computer program that was developed by Advanced Biosciences Computing Center at the University of Minnesota, St. Paul, MN. This program aids molecular biologists in finding potential transcription factor binding sites and other elements in a DNA sequence. A complete description of the program can be found in Prestridge, D.S., CABIOS 7: 203-206 (1991), the entire disclosure of which is incorporated herein as if set forth at length.

SIGNAL SCAN finds sequence homologies between published signal sequences and an unknown sequence. A signal, as defined herein, is any short DNA sequence that may have known significance. Most of the known signals represent transcriptional elements. The program does not interpret the significance of the identified homologies; interpretation of the significance of sequences identified is left up to the user. The significance of the signal elements varies with the signal length, with matches to short segments having a higher probability of random occurrence.

### D. Results of the Primer Extension Analysis And SIGNAL SCAN

Figure 5 shows the results of the primer extension analysis done to locate the transcription initiation site for pRb2/p130. A major extended fragment of 78 bp was detected (lane 1) from the primer extension done with HeLa Cells as the template. The probable position of the identified transcription start site is indicated by the arrow in Fig. 4. Putative transcription factor-binding sites were identified by their similarity to consensus sequences for known transcription factor-binding sites. The sequence motifs corresponding to Sp1, Ker1, and MyoD are also indicated in Fig. 4.

### Example 7

### Detection of Heterozygous Mutations By PCR

### A. Preparation of Genomic DNA

The genomic DNA used herein was obtained from human peripheral blood lymphocytes. The samples were prepared by the methods of Sambrook et al., Molecular Cloning:A Laboratory Manual. Second Edition, pp. 9.16-9.23, Cold Spring Harbor Laboratory Press, Cold Spring Harbor, NY (1989).

### B. Synthesis Of PCR Primers

The PCR primers used herein were synthesized as described in Example 5D. The specific primer sequences used and their annealing temperatures are given in Table 8. as SEQ ID NOS:69 to 112.

**Table 8**

| Exon Amplified | Sequence Of Primer (5'-3') | Annealing Temperature (°C) | Size Of PCR Product (bp) |
|---|---|---|---|
| Exon 1 | TTCGCCGTTTGAATTGCTGC (SEQ ID NO:93) | 55 | 359 |
| Exon 1(rev) | ACCGGTTCACACCAACTAGG (SEQ ID NO:94) | | |
| Exon 2 | GAGATAGGGTCATCATTGAAAC (SEQ ID NO:95) | 55 | 206 |
| Exon 2(rev) | CATTAGCCATACTCTACTTGT (SEQ ID NO:96) | | |
| Exon 3 | GCTAATTTAACTCTGTAACTGC (SEQ ID NO:97) | 55 | 327 |
| Exon 3(rev) | CACTGCAGCACAGACTAATGTGT (SEQ ID NO:98) | | |
| Exon 4 | TCTCTCCCTTTAACTGTGGGTTT (SEQ ID NO:99) | 55 | 245 |
| Exon 4(rev) | GGAGTTGACGAGATTAATACCTG (SEQ ID NO: 100) | | |
| Exon 5 | CTCTGTAACTGCTTATAATCCTG (SEQ ID NO:69) | 55 | 235 |
| Exon 5(rev) | CTAGGAAACCTGTACAACTCC (SEQ ID NO:70) | | |
| Exon 6 | GGCTTATTGTGTGCTGATATC (SEQ ID NO:71) | 55 | 289 |
| Exon 6(rev) | AGAGATCCTTAAGTCGTCATG (SEQ ID NO:72) | | |
| Exon 7 | CATGACGACTTAAGGATCTCTT (SEQ ID NO: 101) | 55 | 196 |
| Exon 7(rev) | CTCAGTTTCCAGAGTACAAAC (SEQ ID NO: 102) | | |
| Exon 8 | CAGTTTCTGTGAGAGAGTACA (SEQ ID NO:73) | 55 | 283 |
| Exon 8(rev) | GGCTTACCTGCTCCTGTATTT (SEQ ID NO:74) | | |
| Exon 9 | GTGAATTAAAGTCTTTCTGGCC (SEQ ID NO: 103) | 55 | 277 |
| Exon 9(rev) | ATCTTAGAAAGCAGACAGGGC (SEQ ID NO: 104) | | |
| Exon 10 | GAGACATTTTATCCCCTTGTG (SEQ ID NO: 105) | 55 | 289 |
| Exon 10(rev) | TCCATGCCTCCAGTCTAAAGT (SEQ ID NO:106) | | |
| Exon 11 | GAGGAGGAATGGGCCTTTATT (SEQ ID NO:75) | 55 | 244 |
| Exon 11(rev) | AACCCACAGAATAGGGCAGGA (SEQ ID NO:76) | | |
| Exon 12 | CACTTAAGTTGCACTGGGTA (SEQ ID NO: 107) | 55 | 273 |
| Exon 12(rev) | CAACAGGAAGTTGGTCTCATC (SEQ ID NO:108) | | |
| Exon 13 | TAAAAGGAAGAGCGGCTGTTT (SEQ ID NO: 109) | 55 | 378 |
| Exon 13(rev) | TTAAACCTAACTGCCACCCTC (SEQ ID NO:110) | | |
| Exon 14 | GGATACTGGCATTCTGTGTAAC (SEQ ID NO:77) | 55 | 197 |
| Exon 14(rev) | ATTTCCAGATAGTAAGCCCCA (SEQ ID NO:78) | | |
| Exon 15 | AGCTTGGACGGAAGTCAGATC (SEQ ID NO:79) | 55 | 413 |
| Exon 15(rev) | TCTAGCCAAACCTCGGGTAAC (SEQ ID NO:80) | | |
| Exon 16 | AATTGTAAACCTCTGCCC (SEQ ID NO:81) | 55 | 394 |
| Exon 16(rev) | ATTTCCCAAGCTCATGCT (SEQ ID NO:82) | | |
| Exon 17 | AGCATGAGCTTGGGAAAT (SEQ ID NO:83) | 55 | 277 |
| Exon 17(rev) | TGAAGACCTATCTTTGCC | | |
| Exon 18 | (SEQ ID NO:84) GTTCACAGAGCTCCTCACACT | 55 | 230 |
| Exon 18(rev) | (SEQ ID NO:85) AGGCCACAGAGTCAACTATGG | | |
| Exon 19 | (SEQ ID NO:86) AGGTCCTATCACCAAGGGTGT | 55 | 250 |
| Exon 19(rev) | (SEQ ID NO:87) GCTTAGTTACTTCTTCAAGGC | | |
| Exon 20 | (SEQ ID NO:88) GTAGCTGTTCCCTTTCTCCTA | 55 | 364 |
| Exon 20(rev) | (SEQ ID NO:89) CCTCAACACTCATGAGAGTGA | | |
| Exon 21 | (SEQ ID NO:90) TGGTTTAGCACACCTCTTCAC | 55 | 325 |
| Exon 21 (rev) | (SEQ ID NO:91) GCTTAGCACAAACCCTGTTTC | | |
| Exon 22 | (SEQ ID NO:92) CTGAGCTATGTGCATTTGCA | 55 | 232 |
| Exon 22(rev) | (SEQ ID NO:111) AAGGCTGCTGCTAAACAGAT (SEQ ID NO:112) | | |

### C. PCR Amplification

The sample DNA was amplified in a Perkin-Elmer Cetus thermocycler. The PCR was performed in a 100 µl reaction volume using 2.5 units of recombinant Taq DNA-polymerase and 40 ng of genomic DNA. The reaction mixture was prepared according to the recommendations given in the Gene Amp DNA Amplification kit (Perkin-Elmer Cetus). The reaction mixture consisted of 50 mM/l KCl, 10mM/l Tris-HCl (pH 8.3), 1.5 mM MgCl. 200 µM each deoxynucleotide triphosphate and 1 µM of each primer. Thirty five (35) PCR cycles were carried out, with each cycle consisting of an initial denaturation step at 95°C for one minute, one minute at the annealing temperature (55°C), an extension step at 72°C for one minute, and followed by a final incubation period at 72°C for seven minutes. Suitable annealing temperatures are shown in Table 8 for each of the primers designed in accordance with this invention. Minor adjustments in the annealing temperatures may be made to accommodate other primers designed in accordance with this invention.

### D. Amplification Products of PCR

The size of the amplification products produced by PCR are shown in Table 8 above. The lengths of the PCR products ranged from 196 bp to 413 bp.

### E. Sequencing of PCR Products

Sequencing of the amplification products of pRb2/p130 can be conducted according to the method set forth in Example 5C above. Sequencing can also be performed by the chain termination technique described by Sanger et al., Proc. Nat'l. Acad. Sci. , U. S. A. 74:5463-5467 (1977) or Sambrook et al., Molecular Cloning: A Laboratory Manual, Second Edition, pp. 13.42-13.77, Cold Spring Harbor Laboratory Press, Cold Spring Harbor, NY (1989) with appropriate primers based on the pRb2/p130 genomic sequence described herein.

### Example 8

### Detecting Mutations By SSCP Analysis

### A. General Methods

The SSCP analysis was performed according to the methods of Orita et al., Genomics 5: 874-879 (1989) and Hogg et al., Oncogene 7: 1445-1451 (1992), each of which is incorporated herein by reference. For the SSCP analysis, amplification of the individual exons was, in some experiments, performed as described in Example 7 with the exception that 1 µCi of [³²P]dCTP (3000 Ci mmol⁻¹) was added to the mixture in order to obtain a labeled product. A 10% aliquot of the PCR-amplified product was diluted with a mixture of 10-20 µl of 0.1 % SDS and 10 mM EDTA. Following a 1:1 dilution with 95% formamide. 2mM EDTA, 0.05% bromophenol blue, and 0.05% xylene cyanol loading solution (United States Biochemicals. OH), the diluted sample was run on a 6% non-denaturing gel. The DNA was electrophoresed in TBE (0.09 M Tris base, 0.09 M boric acid and 2.5 mM EDTA) running buffer at constant wattage at room temperature. The gel was dried on filter paper and exposed to X-ray film for 12 to 72 hours without an intensifying screen.

Polymorphisms and mutations were detected by observing a shift in the electrophoretic mobility pattern of the denatured PCR-amplified product relative to a corresponding wild type sample or normal tissue sample from the same patient. Once a band shift was identified, the segment was sequenced to confirm the exact nature of the polymorphism or mutation.

### B. Detection Of pRb2/p130 Gene Mutations In the CCRF-CEM Cell Line

DNA was extracted from the CCRF-CEM line (human lymphoblastoid cells), and amplified. For the amplification, 50 µl of the PCR reaction mix containing 4 ng of genomic DNA, 0.2 mM of each deoxynucleotide triphosphates. 2 U of Taq polymerase and 0.4 µM of each primer were used. Fifty-Five cycles of denaturation (95°C, 1 minute), annealing (55°C, 1 minute) and extension (72°C, 1 minute) were carried out in a thermal cycler. The SSCP analysis was performed using an MDE mutation detection kit (AT Biochem). The PCR products were heated to 95°C for two minutes and placed directly on ice for several minutes. The samples were run through the MDE gel at 8 Watts constant power for eight hours at room temperature, in 0.6X TBE running buffer. The gel was stained for 15 minutes at room temperature in a 1 µg/ml ethidium bromide solution, made in 0.6X TBE buffer, and placed on a UV-transilluminator to visualize the bands. Exon 20 showed a different migration relative to the control, suggesting the presence of mutations.

The sequences of the PCR products were determined by automated DNA sequencing, using dideoxy-terminator reaction chemistry. Two point mutations were identified: ACC to GCC at position 2950 of SEQ ID NO: 1, resulting in a threonine to alanine substitution; and CCT to CGT at position 3029 of SEQ ID NO: 1, resulting in a proline to arginine substitution.

### C. Detection of pRb2/p130 Gene Mutations in Other Cell Lines

Using the SSCP and DNA sequencing methods described above, mutations in the pRb2/p130 gene were identified in the following human tumor cell lines:
Jurkat cell line (human leukemia, T-cell lymphoblast): point mutations in exon 22;
K562 cell line (human chronic myelogenous leukemia, erythroblastoid cells): point mutations in exon 22, deletion in exon 21;
Molt-4 cell line (human T-cell leukemia, peripheral blood lymphoblast): point mutations in exon 21, mutation(s) in exon 22;
Daudi cell line (human thyroid lymphoma, lymphoblast B cell): point mutations and insertion in exon 19, point mutations and insertions in exon 21, mutations(s) in exon 22;
Cem cell line (lymphoblastoid cell line, T-lymphocytes): mutation(s) in exon 20, point mutations and insertions in exon 22:
Saos-2 cell line (human primary osteogenic sarcoma): point mutations and insertions in exon 21, point mutations and insertion in exon 22;
U2-Os cell line (human primary osteogenic sarcoma): point mutations in exons 19 and 21, point mutation and insertion in exon 22:
MG63 cell line (human osteosarcoma): point mutations in exon 19:
Hos cell line (human osteogenic sarcoma. TE85): point mutations in exon 19: insertions in exon 22;
U1752 cell line (human lung tumor): point mutations in exon 19. point mutations and insertion in exon 21, point mutation and insertion in exon 22;
H69 cell line (human lung tumor): point mutations in exon 21, point mutations and insertions in exon 22;
H82 cell line (human lung tumor): point mutations in exon 21; and
Hone cell line (human nasopharyngeal carcinoma): mutations and insertion in exon 21, mutation(s) in exon 22.

### D. Detection of pRb2/p130 Gene Mutations in Primary Tumors

Using the SSCP and DNA sequencing methods described above, mutations in the pRb2/p130 gene were identified in the following primary human tumors:

13 NPC primary tumor (human nasopharyngeal carcinoma): point mutations in exon 21, point mutation and insertions in exon 22; and
5 NPC primary tumor (human nasopharyngeal carcinoma): point mutations and insertion in exon 22.

### Example 9

### Detecting Mutations By The PRINS Technique

The PRINS technique was performed according to the method of Cinti et al., Nuc. Acids Res. Vol. 21, No. 24: 5799-5800 (1993) using human peripheral lymphocytes as the source of genomic DNA. The oligonucleotide primers were designed such that they included portions of the introns flanking exon 20. The sequences of the primers utilized to amplify exon 20 are listed in Table 8 above (SEQ ID NOS:89 and 90).

Human fixed metaphase chromosomes or interphase nuclei from PHA stimulated peripheral blood lymphocytes were spread onto glass slides and allowed to air dry for ten days. The DNA was dehydrated in an ethanol series (70%, 90%, and 100%) and then denatured by heating to 94°C for 5 minutes. Using a reaction mixture containing 200 pmol of each oligonucleotide primer, 5 µl of 10 X PCR Buffer II (AmpliTaq, Perkin-Elmer), 2 µl DIG DNA labeling mixture (1 mM dATP, 1mM dCTP, 1mM dGTP, 0.65 mM dTTP, 0.35 mM DIG-dUTP, Boehringer-Mannheim) and 2 Units of Taq I DNA polymerase (AmpliTaq, Perkin-Elmer), the samples were incubated for 10 minutes at 55 °C and for 30 minutes at 72°C. Suitable annealing temperatures for other primers designed in accordance with this invention are shown in Table 8. The samples were then washed two times in 2 X SSC (pH 7.0) and in 4 X SSC (pH 7.0) for 5 minutes at room temperature. The DNA samples were then placed in a solution of 4 X SSC and 0.5% Bovine Serum Albumin (BSA) (pH 7.0), incubated at room temperature for 45 minutes with anti-Digoxigenin-FITC (Boehringer-Mannheim), and diluted 1:100 in 4 X SSC and 0.5% BSA (pH 7.0). After washing the samples in 4 X SSC and 0.05% Triton X-100, the samples were counterstained with 1 µg/ml Propidium Iodide (PI).

The slides were examined under a Confocal Laser Scanning Microscope (CLSM Sarastro, Molecular Dynamics). The FITC and PI signals were detected simultaneously, independently elaborated and the final projections were superimposed with a Silicon Graphic Computer Personal IRIS-4D/20 workstation.

Figure 6 shows the results of a PRINS reaction on normal human interphase nuclei. The bright spots correspond to a DNA segment containing exon 20 of pRb2/p130. This individual is homozygous for the presence of exon 20 of pRb2/p130. Had there been a mutation in exon 20 of this individual, either one or both of these areas would have been diminished in intensity or not visible in its entirety. To determine the exact nature of this mutation, the patient's pRb2/p130 DNA segment would he sequenced by methods known to those skilled in the art and compared to a wild type sample of pRb2/p130 DNA.

All the references discussed herein are incorporated by reference. Some or all of the reagents, compositions, and supplies needed to carry out the methods, procedures, and techniques disclosed herein may be provided in the form of a kit. Such kits are another embodiment of the present invention.

One skilled in the art will readily appreciate that the present invention is well adapted to carry out the ends and advantages mentioned, as well as those inherent therein. The nucleic acids, compositions, methods, procedures, and techniques described herein are presented as representative of the preferred embodiments, and are intended to be exemplary and not limitations on the scope of the invention. The present invention may be embodied in other specific forms without departing from the spirit or essential attributes thereof and, accordingly, reference should be made to the appended claims, rather than to the foregoing specification, as defining the scope of the invention.

## Claims

1. A method for grading a cancer comprising:
determining the level of expression of the pRb2/p130 gene in a sample of tissue from a patient suffering from cancer, the level of expression being indicative of the grade of the cancer, wherein the cancer is a gynecologic cancer.

2. The method according to claim 1 wherein determining the level of expression of the pRb2/p130 gene comprises determining the relative number of RNA transcripts of the gene in the sampled tissue, relative to a standard number of said transcripts.

3. The method according to claim 1 wherein determining the level of expression of the pRb2/p130 gene comprises determining the relative level of the corresponding pRb2/p130 protein in the sampled tissue, relative to a standard level of said protein.

4. The method according to claim 3 wherein the level of the protein in the sampled tissue is determined by an immunoassay whereby an antibody which binds said pRb2/p130 protein is contacted with said sampled tissue.

5. The method according to claim 1 wherein the gynecologic cancer is endometrial carcinoma or ovarian cancer.


SEQUENCE LISTING

<110>  Giordano, Antonio

<120>  METHODS FOR THE DIAGNOSIS AND PROGNOSIS OF
       CANCER

<130>  DTC1191/8/Div

<160>  116

<170>  PatentIn version 3.3

<210>  1
<211>  4853
<212>  DNA
<213>  Artificial

<220>
<223>  CDS

<400> 1

TTCGCCGTTT GAATTGCTGC GGGCCCGGGC CCTCACCTCA CCTGAGGTCC GGCCGCCCAG      60

GGGTGCGCT ATG CCG TCG GGA GGT GAC CAG TCG CCA CCG CCC CCG CCT      108
          Met Pro Ser Gly Gly Asp Gln Ser Pro Pro Pro Pro Pro
          1               5                   10

CCC CCT CCG GCG GCG GCA GCC TCG GAT GAG GAG GAG GAG GAC GAC GGC      156
Pro Pro Pro Ala Ala Ala Ala Ser Asp Glu Glu Glu Glu Asp Asp Gly
    15                  20                  25

GAG GCG GAA GAC GCC GCG CCG TCT GCC GAG TCG CCC ACC CCT CAG ATC      204
Glu Ala Glu Asp Ala Ala Pro Ser Ala Glu Ser Pro Thr Pro Gln Ile
30                  35                  40                  45

CAG CAG CGG TTC GAC GAG CTG TGC AGC CGC CTC AAC ATG GAC GAG GCG      252
Gln Gln Arg Phe Asp Glu Leu Cys Ser Arg Leu Asn Met Asp Glu Ala
                50                  55                  60

GCG CGG CCC GAG GCC TGG GAC AGC TAC CGC AGC ATG AGC GAA AGC TAC      300
Ala Arg Pro Glu Ala Trp Asp Ser Tyr Arg Ser Met Ser Glu Ser Tyr
            65                  70                  75
ACG CTG GAG GGA AAT GAT CTT CAT TGG TTA GCA TGT GCC TTA TAT GTG      348
Thr Leu Glu Gly Asn Asp Leu His Trp Leu Ala Cys Ala Leu Tyr Val
        80                  85                  90

GCT TGC AGA AAA TCT GTT CCA ACT GTA AGC AAA GGG ACA GTG GAA GGA      396
Ala Cys Arg Lys Ser Val Pro Thr Val Ser Lys Gly Thr Val Glu Gly
    95                  100                 105

AAC TAT GTA TCT TTA ACT AGA ATC CTG AAA TGT TCA GAG CAG AGC TTA      444
Asn Tyr Val Ser Leu Thr Arg Ile Leu Lys Cys Ser Glu Gln Ser Leu
110                 115                 120                 125

ATC GAA TTT TTT AAT AAG ATG AAG AAG TGG GAA GAC ATG GCA AAT CTA      492
Ile Glu Phe Phe Asn Lys Met Lys Lys Trp Glu Asp Met Ala Asn Leu
                130                 135                 140

CCC CCA CAT TTC AGA GAA CGT ACT GAG AGA TTA GAA AGA AAC TTC ACT      540
Pro Pro His Phe Arg Glu Arg Thr Glu Arg Leu Glu Arg Asn Phe Thr
            145                 150                 155

GTT TCT GCT GTA ATT TTT AAG AAA TAT GAA CCC ATT TTT CAG GAC ATC      588
Val Ser Ala Val Ile Phe Lys Lys Tyr Glu Pro Ile Phe Gln Asp Ile

        160                 165                 170

TTT AAA TAC CCT CAA GAG GAG CAA CCT CGT CAG CAG CGA GGA AGG AAA 636
Phe Lys Tyr Pro Gln Glu Glu Gln Pro Arg Gln Gln Arg Gly Arg Lys
    175                 180                 185

CAG CGG CGA CAG CCC TGT ACT GTG TCT GAA ATT TTC CAT TTT TGT TGG 684
Gln Arg Arg Gln Pro Cys Thr Val Ser Glu Ile Phe His Phe Cys Trp
190                 195                 200                 205

GTG CTT TTT ATA TAT GCA AAA GGT AAT TTC CCC ATG ATT AGT GAT GAT 732
Val Leu Phe Ile Tyr Ala Lys Gly Asn Phe Pro Met Ile Ser Asp Asp
                210                 215                 220

TTG GTC AAT TCT TAT CAC CTG CTG CTG TGT GCT TTG GAC TTA GTT TAT 780
Leu Val Asn Ser Tyr His Leu Leu Leu Cys Ala Leu Asp Leu Val Tyr
            225                 230                 235

GGA AAT GCA CTT CAG TGT TCT AAT CGT AAA GAA CTT GTG AAC CCT AAT 828
Gly Asn Ala Leu Gln Cys Ser Asn Arg Lys Glu Leu Val Asn Pro Asn
        240                 245                 250

TTT AAA GGC TTA TCT GAA GAT TTT CAT GCT AAA GAT TCT AAA CCT TCC 876
Phe Lys Gly Leu Ser Glu Asp Phe His Ala Lys Asp Ser Lys Pro Ser
    255                 260                 265

TCT GAC CCC CCT TGT ATC ATT GAG AAA CTG TGT TCC TTA CAT GAT GGC 924
Ser Asp Pro Pro Cys Ile Ile Glu Lys Leu Cys Ser Leu His Asp Gly
270                 275                 280                 285

CTA GTT TTG GAA GCA AAG GGG ATA AAG GAA CAT TTC TGG AAA CCC TAT 972
Leu Val Leu Glu Ala Lys Gly Ile Lys Glu His Phe Trp Lys Pro Tyr
                290                 295                 300

ATT AGG AAA CTT TAT GAA AAA AAG CTC CTT AAG GGA AAA GAA GAA AAT 1020
Ile Arg Lys Leu Tyr Glu Lys Lys Leu Leu Lys Gly Lys Glu Glu Asn
            305                 310                 315

CTC ACT GGG TTT CTA GAA CCT GGG AAC TTT GGA GAG AGT TTT AAA GCC 1068
Leu Thr Gly Phe Leu Glu Pro Gly Asn Phe Gly Glu Ser Phe Lys Ala
        320                 325                 330

ATC AAT AAG GCC TAT GAG GAG TAT GTT TTA TCT GTT GGG AAT TTA GAT 1116
Ile Asn Lys Ala Tyr Glu Glu Tyr Val Leu Ser Val Gly Asn Leu Asp
    335                 340                 345

GAG CGG ATA TTT CTT GGA GAG GAT GCT GAG GAG GAA ATT GGG ACT CTC 1164
Glu Arg Ile Phe Leu Gly Glu Asp Ala Glu Glu Glu Ile Gly Thr Leu
350                 355                 360                 365

TCA AGG TGT CTG AAC GCT GGT TCA GGA ACA GAG ACT GCT GAA AGG GTG 1212
Ser Arg Cys Leu Asn Ala Gly Ser Gly Thr Glu Thr Ala Glu Arg Val
                370                 375                 380

CAG ATG AAA AAC ATC TTA CAG CAG CAT TTT GAC AAG TCC AAA GCA CTT 1260
Gln Met Lys Asn Ile Leu Gln Gln His Phe Asp Lys Ser Lys Ala Leu
            385                 390                 395

AGA ATC TCC ACA CCA CTA ACT GGT GTT AGG TAC ATT AAG GAG AAT AGC 1308
Arg Ile Ser Thr Pro Leu Thr Gly Val Arg Tyr Ile Lys Glu Asn Ser
        400                 405                 410

CCT TGT GTG ACT CCA GTT TCT ACA GCT ACG CAT AGC TTG AGT CGT CTT 1356
Pro Cys Val Thr Pro Val Ser Thr Ala Thr His Ser Leu Ser Arg Leu
    415                 420                 425

CAC ACC ATG CTG ACA GGC CTC AGG AAT GCA CCA AGT GAG AAA CTG GAA 1404
His Thr Met Leu Thr Gly Leu Arg Asn Ala Pro Ser Glu Lys Leu Glu

430                 435                 440                 445

CAG ATT CTC AGG ACA TGT TCC AGA GAT CCA ACC CAG GCT ATT GCT AAC     1452
Gln Ile Leu Arg Thr Cys Ser Arg Asp Pro Thr Gln Ala Ile Ala Asn
                450                 455                 460

AGA CTG AAA GAA ATG TTT GAA ATA TAT TCT CAG CAT TTC CAG CCA GAC     1500
Arg Leu Lys Glu Met Phe Glu Ile Tyr Ser Gln His Phe Gln Pro Asp
            465                 470                 475

GAG GAT TTC AGT AAT TGT GCT AAA GAA ATT GCC AGC AAA CAT TTT CGT     1548
Glu Asp Phe Ser Asn Cys Ala Lys Glu Ile Ala Ser Lys His Phe Arg
        480                 485                 490

TTT GCG GAG ATG CTT TAC TAT AAA GTA TTA GAA TCT GTT ATT GAG CAG     1596
Phe Ala Glu Met Leu Tyr Tyr Lys Val Leu Glu Ser Val Ile Glu Gln
    495                 500                 505

GAA CAA AAA AGA CTA GGA GAC ATG GAT TTA TCT GGT ATT CTG GAA CAA     1644
Glu Gln Lys Arg Leu Gly Asp Met Asp Leu Ser Gly Ile Leu Glu Gln
510                 515                 520                 525

GAT GCA TTC CAC AGA TCT CTC TTG GCC TGC TGC CTT GAG GTC GTC ACT     1692
Asp Ala Phe His Arg Ser Leu Leu Ala Cys Cys Leu Glu Val Val Thr
                530                 535                 540

TTT TCT TAT AAG CCT CCT GGG AAT TTT CCA TTT ATT ACT GAA ATA TTT     1740
Phe Ser Tyr Lys Pro Pro Gly Asn Phe Pro Phe Ile Thr Glu Ile Phe
            545                 550                 555

GAT GTG CCT CTT TAT CAT TTT TAT AAG GTG ATA GAA GTA TTC ATT AGA     1788
Asp Val Pro Leu Tyr His Phe Tyr Lys Val Ile Glu Val Phe Ile Arg
        560                 565                 570

GCA GAA GAT GGC CTT TGT AGA GAG GTG GTA AAA CAC CTT AAT CAG ATT     1836
Ala Glu Asp Gly Leu Cys Arg Glu Val Val Lys His Leu Asn Gln Ile
    575                 580                 585

GAA GAA CAG ATC TTA GAT CAT TTG GCA TGG AAA CCA GAG TCT CCA CTC     1884
Glu Glu Gln Ile Leu Asp His Leu Ala Trp Lys Pro Glu Ser Pro Leu
590                 595                 600                 605

TGG GAA AAA ATT AGA GAC AAT GAA AAC AGA GTT CCT ACA TGT GAA GAG     1932
Trp Glu Lys Ile Arg Asp Asn Glu Asn Arg Val Pro Thr Cys Glu Glu
                610                 615                 620

GTC ATG CCA CCT CAG AAC CTG GAA AGG GCA GAT GAA ATT TGC ATT GCT     1980
Val Met Pro Pro Gln Asn Leu Glu Arg Ala Asp Glu Ile Cys Ile Ala
            625                 630                 635

GGC TCC CCT TTG ACT CCC AGA AGG GTG ACT GAA GTT CGT GCT GAT ACT     2028
Gly Ser Pro Leu Thr Pro Arg Arg Val Thr Glu Val Arg Ala Asp Thr
        640                 645                 650

GGA GGA CTT GGA AGG AGC ATA ACA TCT CCA ACC ACA TTA TAC GAT AGG     2076
Gly Gly Leu Gly Arg Ser Ile Thr Ser Pro Thr Thr Leu Tyr Asp Arg
    655                 660                 665

TAC AGC TCC CCA CCA GCC AGC ACT ACC AGA AGG CGG CTA TTT GTT GAG     2124
Tyr Ser Ser Pro Pro Ala Ser Thr Thr Arg Arg Arg Leu Phe Val Glu
670                 675                 680                 685

AAT GAT AGC CCC TCT GAT GGA GGG ACG CCT GGG CGC ATG CCC CCA CAG     2172
Asn Asp Ser Pro Ser Asp Gly Gly Thr Pro Gly Arg Met Pro Pro Gln
                690                 695                 700

CCC CTA GTC AAT GCT GTC CCT GTG CAG AAT GTA TCT GGG GAG ACT GTT     2220
Pro Leu Val Asn Ala Val Pro Val Gln Asn Val Ser Gly Glu Thr Val

            705                 710                 715

TCT GTC ACA CCA GTT CCT GGA CAG ACT TTG GTC ACC ATG GCA ACC GCC 2268
Ser Val Thr Pro Val Pro Gly Gln Thr Leu Val Thr Met Ala Thr Ala
        720                 725                 730

ACT GTC ACA GCC AAC AAT GGG CAA ACG GTA ACC ATT CCT GTG CAA GGT 2316
Thr Val Thr Ala Asn Asn Gly Gln Thr Val Thr Ile Pro Val Gln Gly
    735                 740                 745

ATT GCC AAT GAA AAT GGA GGG ATA ACA TTC TTC CCT GTC CAA GTC AAT 2364
Ile Ala Asn Glu Asn Gly Gly Ile Thr Phe Phe Pro Val Gln Val Asn
750                 755                 760                 765

GTT GGG GGG CAG GCA CAA GCT GTG ACA GGC TCC ATC CAG CCC CTC AGT 2412
Val Gly Gly Gln Ala Gln Ala Val Thr Gly Ser Ile Gln Pro Leu Ser
                770                 775                 780

GCT CAG GCC CTG GCT GGA AGT CTG AGC TCT CAA CAG GTG ACA GGA ACA 2460
Ala Gln Ala Leu Ala Gly Ser Leu Ser Ser Gln Gln Val Thr Gly Thr
            785                 790                 795

ACT TTG CAA GTC CCT GGT CAA GTG GCC ATT CAA CAG ATT TCC CCA GGT 2508
Thr Leu Gln Val Pro Gly Gln Val Ala Ile Gln Gln Ile Ser Pro Gly
        800                 805                 810

GGC CAA CAG CAG AAG CAA GGC CAG TCT GTA ACC AGC AGT AGT AAT AGA 2556
Gly Gln Gln Gln Lys Gln Gly Gln Ser Val Thr Ser Ser Ser Asn Arg
    815                 820                 825

CCC AGG AAG ACC AGC TCT TTA TCG CTT TTC TTT AGA AAG GTA TAC CAT 2604
Pro Arg Lys Thr Ser Ser Leu Ser Leu Phe Phe Arg Lys Val Tyr His
830                 835                 840                 845

TTA GCA GCT GTC CGC CTT CGG GAT CTC TGT GCC AAA CTA GAT ATT TCA 2652
Leu Ala Ala Val Arg Leu Arg Asp Leu Cys Ala Lys Leu Asp Ile Ser
                850                 855                 860

GAT GAA TTG AGG AAA AAA ATC TGG ACC TGC TTT GAA TTC TCC ATA ATT 2700
Asp Glu Leu Arg Lys Lys Ile Trp Thr Cys Phe Glu Phe Ser Ile Ile
            865                 870                 875

CAG TGT CCT GAA CTT ATG ATG GAC AGA CAT CTG GAC CAG TTA TTA ATG 2748
Gln Cys Pro Glu Leu Met Met Asp Arg His Leu Asp Gln Leu Leu Met
        880                 885                 890

TGT GCC ATT TAT GTG ATG GCA AAG GTC ACA AAA GAA GAT AAG TCC TTC 2796
Cys Ala Ile Tyr Val Met Ala Lys Val Thr Lys Glu Asp Lys Ser Phe
    895                 900                 905

CAG AAC ATT ATG CGT TGT TAT AGG ACT CAG CCG CAG GCC CGG AGC CAG 2844
Gln Asn Ile Met Arg Cys Tyr Arg Thr Gln Pro Gln Ala Arg Ser Gln
910                 915                 920                 925

GTG TAT AGA AGT GTT TTG ATA AAA GGG AAA AGA AAA AGA AGA AAT TCT 2892
Val Tyr Arg Ser Val Leu Ile Lys Gly Lys Arg Lys Arg Arg Asn Ser
                930                 935                 940

GGC AGC AGT GAT AGC AGA AGC CAT CAG AAT TCT CCA ACA GAA CTA AAC 2940
Gly Ser Ser Asp Ser Arg Ser His Gln Asn Ser Pro Thr Glu Leu Asn
            945                 950                 955

AAA GAT AGA ACC AGT AGA GAC TCC AGT CCA GTT ATG AGG TCA AGC AGC 2988
Lys Asp Arg Thr Ser Arg Asp Ser Ser Pro Val Met Arg Ser Ser Ser
        960                 965                 970

ACC TTG CCA GTT CCA CAG CCC AGC AGT GCT CCT CCC ACA CCT ACT CGC 3036
Thr Leu Pro Val Pro Gln Pro Ser Ser Ala Pro Pro Thr Pro Thr Arg

975                 980                 985

CTC ACA GGT GCC AAC AGT GAC ATG GAA GAA GAG GAG AGG GGA GAC CTC          3084
Leu Thr Gly Ala Asn Ser Asp Met Glu Glu Glu Glu Arg Gly Asp Leu
990                 995                 1000                1005

ATT CAG TTC TAC AAC AAC ATC TAC ATC AAA CAG ATT AAG ACA TTT GCC          3132
Ile Gln Phe Tyr Asn Asn Ile Tyr Ile Lys Gln Ile Lys Thr Phe Ala
                1010                1015                1020

ATG AAG TAC TCA CAG GCA AAT ATG GAT GCT CCT CCA CTC TCT CCC TAT          3180
Met Lys Tyr Ser Gln Ala Asn Met Asp Ala Pro Pro Leu Ser Pro Tyr
            1025                1030                1035

CCA TTT GTA AGA ACA GGC TCC CCT CGC CGA ATA CAG TTG TCT CAA AAT          3228
Pro Phe Val Arg Thr Gly Ser Pro Arg Arg Ile Gln Leu Ser Gln Asn
        1040                1045                1050

CAT CCT GTC TAC ATT TCC CCA CAT AAA AAT GAA ACA ATG CTT TCT CCT          3276
His Pro Val Tyr Ile Ser Pro His Lys Asn Glu Thr Met Leu Ser Pro
    1055                1060                1065

CGA GAA AAG ATT TTC TAT TAC TTC AGC AAC AGT CCT TCA AAG AGA CTG          3324
Arg Glu Lys Ile Phe Tyr Tyr Phe Ser Asn Ser Pro Ser Lys Arg Leu
1070                1075                1080                1085

AGA GAA ATT AAT AGT ATG ATA CGC ACA GGA GAA ACT CCT ACT AAA AAG          3372
Arg Glu Ile Asn Ser Met Ile Arg Thr Gly Glu Thr Pro Thr Lys Lys
                1090                1095                1100

AGA GGA ATT CTT TTG GAA GAT GGA AGT GAA TCA CCT GCA AAA AGA ATT          3420
Arg Gly Ile Leu Leu Glu Asp Gly Ser Glu Ser Pro Ala Lys Arg Ile
            1105                1110                1115

TGC CCA GAA AAT CAT TCT GCC TTA TTA CGC CGT CTC CAA GAT GTA GCT          3468
Cys Pro Glu Asn His Ser Ala Leu Leu Arg Arg Leu Gln Asp Val Ala
        1120                1125                1130

AAT GAC CGT GGT TCC CAC TGA GGTTAGTCTC TTGTATTAAA CTCTTCACAA             3519
Asn Asp Arg Gly Ser His  *
    1135                1140

AATCTGTTTA GCAGCAGCCT TTAATGCATC TAGATTATGG AGCTTTTTTC CTTAATCCAG        3579

CTGATGAGTT ACAGCCTGTT AGTAACATGA GGGGACATTT TGGTGAGAAA TGGGACTTAA        3639

CTCCTTCCAG TGTCCTTAGA ACATTTTAAT TCATCCCAAC TGTCTTTTTT TCCCTACCAC        3699

TCAGTGATTA CTGTCAAGGC TGCTTACAAT CCAAACTTGG GTTTTTGGCT CTGGCAAAGC        3759

TTTTAGAAAT ACTGCAAGAA ATGATGTGTA CCCAACGTGA GCATAGGAGG CTTCTGTTGA        3819

CGTCTCCAAC AGAAGAACTG TGTTTCAAGT TCAATCCTAC CTGTTTTGTG GTCAGCTGTA        3879

GTCCTCATAA AAAGCAAAAC AAAAATTAGG TATTTTGTCC TAAAACACCT GGTAGGAGTG        3939

TGTGATTTTT TGCATTCCTG ACAAAGGAGA GCACACCCAG GTTTGGAGGT CCTAGGTCAT        3999

TAGCCCTCGT CTCCCGTTCC CTTTGTGCAC ATCTTCCCTC TCCCCATTCG GTGTGGTGCA        4059

GTGTGAAAAG TCCTTGATTG TTCGGGTGTG CAATGTCTGA GTGAACCTGT ATAAGTGGAG        4119

GCACTTTAGG GCTGTAAAAT GCATGATTTT GTAACCCAGA TTTTGCTGTA TATTTGTGAT        4179

AGCACTTTCT ACAATGTGAA CTTTATTAAA TACAAAACTT CCAGGCTAAA CATCCAATAT        4239

TTTCTTTAAT GCTTTTATAT TTTTTTAAAA TGTTAAAACC CCTATAGCCA CCTTTTGGGA        4299

ATGTTTTAAA TTCTCCAGTT TTTTGTTATA TAGGGATCAA CCAGCTAAGA AAAGATTTTA 4359

AGTCAAGTTG AATTGAGGGG ATTAATATGA AAACTTATGA CCTCTTCCTT TAGGAGGGAG 4419

TTATCTAAAA GAAATGTCTA TTAAGGTGAT ATATTTAAAA ATATTTTTGG GTGTTCCTGG 4479

CAGTTTAAAA AAATTGGTTG GAGAATTTAG GTTTTTATTA GTACCATAGT ACCATTTATA 4539

CAAATTAGAA AATGTTATTT AACAGCTGAA TTATCTATAC ATATCTTTAT TAATCACTAT 4599

TGTTCCAGCA GTTTTCAAGT CAAATTAATA ATCTTATTAG GGAGAAAATT CAATTGTAAA 4659

TTGAATCAGT ATAAACAAAG TTACTAGGTA ACTTCATATT GCTGAGAGAA ATATGGAACT 4719

TACATTGTTC AATTAGAATA GTGTTCTCCC CAAATATTTA TAAAACTTCT CAAGATACTG 4779

CTACGTGTAA TTTTATATGA AGATAAGTGT ATTTTTCAAT AAAGCATTTA TAAATTAAAA 4839

AAAAAAAAAA AAAA 4853

<210> 2
<211> 1140
<212> PRT
<213> Artificial

<220>
<223> CDS

<400> 2

Met Pro Ser Gly Gly Asp Gln Ser Pro Pro Pro Pro Pro Pro Pro Pro
1               5                   10                  15

Ala Ala Ala Ala Ser Asp Glu Glu Glu Glu Asp Asp Gly Glu Ala Glu
            20                  25                  30

Asp Ala Ala Pro Ser Ala Glu Ser Pro Thr Pro Gln Ile Gln Gln Arg
        35                  40                  45

Phe Asp Glu Leu Cys Ser Arg Leu Asn Met Asp Glu Ala Ala Arg Pro
    50                  55                  60

Glu Ala Trp Asp Ser Tyr Arg Ser Met Ser Glu Ser Tyr Thr Leu Glu
65                  70                  75                  80

Gly Asn Asp Leu His Trp Leu Ala Cys Ala Leu Tyr Val Ala Cys Arg
                85                  90                  95

Lys Ser Val Pro Thr Val Ser Lys Gly Thr Val Glu Gly Asn Tyr Val
            100                 105                 110

Ser Leu Thr Arg Ile Leu Lys Cys Ser Glu Gln Ser Leu Ile Glu Phe
        115                 120                 125

Phe Asn Lys Met Lys Lys Trp Glu Asp Met Ala Asn Leu Pro Pro His
    130                 135                 140

Phe Arg Glu Arg Thr Glu Arg Leu Glu Arg Asn Phe Thr Val Ser Ala
145                 150                 155                 160

Val Ile Phe Lys Lys Tyr Glu Pro Ile Phe Gln Asp Ile Phe Lys Tyr
                165                 170                 175

Pro Gln Glu Glu Gln Pro Arg Gln Gln Arg Gly Arg Lys Gln Arg Arg
            180                 185                 190

Gln Pro Cys Thr Val Ser Glu Ile Phe His Phe Cys Trp Val Leu Phe
        195                 200                 205

Ile Tyr Ala Lys Gly Asn Phe Pro Met Ile Ser Asp Asp Leu Val Asn
    210                 215                 220

Ser Tyr His Leu Leu Leu Cys Ala Leu Asp Leu Val Tyr Gly Asn Ala
225                 230                 235                 240

Leu Gln Cys Ser Asn Arg Lys Glu Leu Val Asn Pro Asn Phe Lys Gly
                245                 250                 255

Leu Ser Glu Asp Phe His Ala Lys Asp Ser Lys Pro Ser Ser Asp Pro
            260                 265                 270

Pro Cys Ile Ile Glu Lys Leu Cys Ser Leu His Asp Gly Leu Val Leu
        275                 280                 285

Glu Ala Lys Gly Ile Lys Glu His Phe Trp Lys Pro Tyr Ile Arg Lys
    290                 295                 300

Leu Tyr Glu Lys Lys Leu Leu Lys Gly Lys Glu Glu Asn Leu Thr Gly
305                 310                 315                 320

Phe Leu Glu Pro Gly Asn Phe Gly Glu Ser Phe Lys Ala Ile Asn Lys
                325                 330                 335

Ala Tyr Glu Glu Tyr Val Leu Ser Val Gly Asn Leu Asp Glu Arg Ile
            340                 345                 350

Phe Leu Gly Glu Asp Ala Glu Glu Glu Ile Gly Thr Leu Ser Arg Cys
        355                 360                 365

Leu Asn Ala Gly Ser Gly Thr Glu Thr Ala Glu Arg Val Gln Met Lys
    370                 375                 380

Asn Ile Leu Gln Gln His Phe Asp Lys Ser Lys Ala Leu Arg Ile Ser
385                 390                 395                 400

Thr Pro Leu Thr Gly Val Arg Tyr Ile Lys Glu Asn Ser Pro Cys Val
                405                 410                 415

Thr Pro Val Ser Thr Ala Thr His Ser Leu Ser Arg Leu His Thr Met
            420                 425                 430

Leu Thr Gly Leu Arg Asn Ala Pro Ser Glu Lys Leu Glu Gln Ile Leu
        435                 440                 445

Arg Thr Cys Ser Arg Asp Pro Thr Gln Ala Ile Ala Asn Arg Leu Lys
    450                 455                 460

Glu Met Phe Glu Ile Tyr Ser Gln His Phe Gln Pro Asp Glu Asp Phe
465                 470                 475                 480

Ser Asn Cys Ala Lys Glu Ile Ala Ser Lys His Phe Arg Phe Ala Glu
                485                 490                 495

Met Leu Tyr Tyr Lys Val Leu Glu Ser Val Ile Glu Gln Glu Gln Lys
            500                 505                 510

Arg Leu Gly Asp Met Asp Leu Ser Gly Ile Leu Glu Gln Asp Ala Phe
        515                 520                 525

His Arg Ser Leu Leu Ala Cys Cys Leu Glu Val Val Thr Phe Ser Tyr
    530                 535                 540

Lys Pro Pro Gly Asn Phe Pro Phe Ile Thr Glu Ile Phe Asp Val Pro
545                 550                 555                 560

Leu Tyr His Phe Tyr Lys Val Ile Glu Val Phe Ile Arg Ala Glu Asp

                565                 570                 575

Gly Leu Cys Arg Glu Val Val Lys His Leu Asn Gln Ile Glu Glu Gln
            580                 585                 590

Ile Leu Asp His Leu Ala Trp Lys Pro Glu Ser Pro Leu Trp Glu Lys
        595                 600                 605

Ile Arg Asp Asn Glu Asn Arg Val Pro Thr Cys Glu Glu Val Met Pro
    610                 615                 620

Pro Gln Asn Leu Glu Arg Ala Asp Glu Ile Cys Ile Ala Gly Ser Pro
625                 630                 635                 640

Leu Thr Pro Arg Arg Val Thr Glu Val Arg Ala Asp Thr Gly Gly Leu
                645                 650                 655

Gly Arg Ser Ile Thr Ser Pro Thr Thr Leu Tyr Asp Arg Tyr Ser Ser
            660                 665                 670

Pro Pro Ala Ser Thr Thr Arg Arg Arg Leu Phe Val Glu Asn Asp Ser
        675                 680                 685

Pro Ser Asp Gly Gly Thr Pro Gly Arg Met Pro Pro Gln Pro Leu Val
    690                 695                 700

Asn Ala Val Pro Val Gln Asn Val Ser Gly Glu Thr Val Ser Val Thr
705                 710                 715                 720

Pro Val Pro Gly Gln Thr Leu Val Thr Met Ala Thr Ala Thr Val Thr
                725                 730                 735

Ala Asn Asn Gly Gln Thr Val Thr Ile Pro Val Gln Gly Ile Ala Asn
            740                 745                 750

Glu Asn Gly Gly Ile Thr Phe Phe Pro Val Gln Val Asn Val Gly Gly
        755                 760                 765

Gln Ala Gln Ala Val Thr Gly Ser Ile Gln Pro Leu Ser Ala Gln Ala
    770                 775                 780

Leu Ala Gly Ser Leu Ser Ser Gln Gln Val Thr Gly Thr Thr Leu Gln
785                 790                 795                 800

Val Pro Gly Gln Val Ala Ile Gln Gln Ile Ser Pro Gly Gly Gln Gln
                805                 810                 815

Gln Lys Gln Gly Gln Ser Val Thr Ser Ser Ser Asn Arg Pro Arg Lys
            820                 825                 830

Thr Ser Ser Leu Ser Leu Phe Phe Arg Lys Val Tyr His Leu Ala Ala
        835                 840                 845

Val Arg Leu Arg Asp Leu Cys Ala Lys Leu Asp Ile Ser Asp Glu Leu
    850                 855                 860

Arg Lys Lys Ile Trp Thr Cys Phe Glu Phe Ser Ile Ile Gln Cys Pro
865                 870                 875                 880

Glu Leu Met Met Asp Arg His Leu Asp Gln Leu Leu Met Cys Ala Ile
                885                 890                 895

Tyr Val Met Ala Lys Val Thr Lys Glu Asp Lys Ser Phe Gln Asn Ile
            900                 905                 910

Met Arg Cys Tyr Arg Thr Gln Pro Gln Ala Arg Ser Gln Val Tyr Arg
        915                 920                 925

Ser Val Leu Ile Lys Gly Lys Arg Lys Arg Arg Asn Ser Gly Ser Ser
    930                 935                 940

Asp Ser Arg Ser His Gln Asn Ser Pro Thr Glu Leu Asn Lys Asp Arg
945                 950                 955                 960

Thr Ser Arg Asp Ser Ser Pro Val Met Arg Ser Ser Ser Thr Leu Pro
                965                 970                 975

Val Pro Gln Pro Ser Ser Ala Pro Pro Thr Pro Thr Arg Leu Thr Gly
            980                 985                 990

Ala Asn Ser Asp Met Glu Glu Glu Glu Arg Gly Asp Leu Ile Gln Phe
        995                 1000                1005

Tyr Asn Asn Ile Tyr Ile Lys Gln Ile Lys Thr Phe Ala Met Lys Tyr
    1010                1015                1020

Ser Gln Ala Asn Met Asp Ala Pro Pro Leu Ser Pro Tyr Pro Phe Val
1025                1030                1035                1040

Arg Thr Gly Ser Pro Arg Arg Ile Gln Leu Ser Gln Asn His Pro Val
                1045                1050                1055

Tyr Ile Ser Pro His Lys Asn Glu Thr Met Leu Ser Pro Arg Glu Lys
            1060                1065                1070

Ile Phe Tyr Tyr Phe Ser Asn Ser Pro Ser Lys Arg Leu Arg Glu Ile
        1075                1080                1085

Asn Ser Met Ile Arg Thr Gly Glu Thr Pro Thr Lys Lys Arg Gly Ile
    1090                1095                1100

Leu Leu Glu Asp Gly Ser Glu Ser Pro Ala Lys Arg Ile Cys Pro Glu
1105                1110                1115                1120

Asn His Ser Ala Leu Leu Arg Arg Leu Gln Asp Val Ala Asn Asp Arg
                1125                1130                1135

Gly Ser His  *
            1140

<210>  3
<211>  21
<212>  PRT
<213>  Artificial

<220>
<223>  CDS

<400> 3

Glu Asn His Ser Ala Leu Leu Arg Arg Leu Gln Asp Val Ala Asn Asp
1               5                   10                  15

Arg Gly Ser His Cys
            20

<210>  4
<211>  561
<212>  DNA
<213>  Artificial

<220>
<223>  CDS

<400> 4

CAGCCCTGTT GAATGTTCTC ACGGTGGGGA GGTACGTGTT TAAAATACGG GGAAGGTGCT 60

TTTATTTCAC CCCTGGTGAA ACTAGGGGAG CTAATTTTTT TAAACATGAT TTTTGTCCCC 120

CTTGAACCGC CGGCCTGGAC TACGTTTCCC AGCAGCCCGT GCTCAAGACT ACGGGTGCCT 180

GCAGGCGGTC AGCGTCGTTT GCGACGGCGC AGACGCGGTG CGGGCGGCGG ACGGGCGGGC 240

GCTTCGCCGT TTGAATTGCT GCGGGCCCGG GCCCTCACCT CACCTGAGGT CCGGCCGCCC 300

AGGGGTGCGC T ATG CCG TCG GGA GGT GAC CAG TCG CCA CCG CCC CCG CCT 350
Met Pro Ser Gly Gly Asp Gln Ser Pro Pro Pro Pro Pro
1               5                   10

CCC CCT CCG GCG GCG GCA GCC TCG GAT GAG GAG GAG GAG GAC GAC GGC 398
Pro Pro Pro Ala Ala Ala Ala Ser Asp Glu Glu Glu Glu Asp Asp Gly
    15                  20                  25

GAG GCG GAA GAC GCC GCG CCG TCT GCC GAG TCG CCC ACC CCT CAG ATC 446
Glu Ala Glu Asp Ala Ala Pro Ser Ala Glu Ser Pro Thr Pro Gln Ile
30                  35                  40                  45

CAG CAG CGG TTC GAC GAG CTG TGC AGC CGC CTC AAC ATG GAC GAG GCG 494
Gln Gln Arg Phe Asp Glu Leu Cys Ser Arg Leu Asn Met Asp Glu Ala
                50                  55                  60

GCG CGG CCC GAG GCC TGG GAC AGC TAC CGC AGC ATG AGC GAA AGC TAC 542
Ala Arg Pro Glu Ala Trp Asp Ser Tyr Arg Ser Met Ser Glu Ser Tyr
            65                  70                  75

ACG CTG GAG GTGCGCTCGC 561
Thr Leu Glu
        80

<210> 5
<211> 80
<212> PRT
<213> Artificial

<220>
<223> CDS

<400> 5

Met Pro Ser Gly Gly Asp Gln Ser Pro Pro Pro Pro Pro Pro Pro Pro
1               5                   10                  15

Ala Ala Ala Ala Ser Asp Glu Glu Glu Glu Asp Asp Gly Glu Ala Glu
            20                  25                  30

Asp Ala Ala Pro Ser Ala Glu Ser Pro Thr Pro Gln Ile Gln Gln Arg
        35                  40                  45

Phe Asp Glu Leu Cys Ser Arg Leu Asn Met Asp Glu Ala Ala Arg Pro
    50                  55                  60

Glu Ala Trp Asp Ser Tyr Arg Ser Met Ser Glu Ser Tyr Thr Leu Glu
65                  70                  75                  80

<210> 6
<211> 19
<212> DNA
<213> Artificial

<220>
<223> CDS

<400> 6

ACGCTGGAGG TGCGCTCGC 19

<210> 7
<211> 19
<212> DNA
<213> Artificial

<220>
<223> CDS

<400> 7

TCTTTTACAG GGAAATGAT 19

<210> 8
<211> 19
<212> DNA
<213> Artificial

<220>
<223> CDS

<400> 8

AGAGCAGAGG TAACTATGT 19

<210> 9
<211> 19
<212> DNA
<213> Artificial

<220>
<223> CDS

<400> 9

TTAATACCAG CTTAATCGA 19

<210> 10
<211> 19
<212> DNA
<213> Artificial

<220>
<223> CDS

<400> 10

GAAACAGCGG TAGGTTTTC 19

<210> 11
<211> 19
<212> DNA
<213> Artificial

<220>
<223> CDS

<400> 11

TCCCCCAAAG GCGACAGCC 19

<210> 12
<211> 19
<212> DNA
<213> Artificial

<220>
<223>  CDS

<400> 12

ATGCAAAAGG TAAGAAAAT                                                    19

<210>  13
<211>  19
<212>  DNA
<213>  Artificial

<220>
<223>  CDS

<400> 13

AATCCTGCAG GTAATTTCC                                                    19

<210>  14
<211>  19
<212>  DNA
<213>  Artificial

<220>
<223>  CDS

<400> 14

ATTTTAAAGG TAGGTTTGT                                                    19

<210>  15
<211>  18
<212>  DNA
<213>  Artificial

<220>
<223>  CDS

<400> 15

ACACCATAGG CTTATCTG                                                     18

<210>  16
<211>  19
<212>  DNA
<213>  Artificial

<220>
<223>  CDS

<400> 16

GAAAAAAAGG TTTGTAAGT                                                    19

<210>  17
<211>  19
<212>  DNA
<213>  Artificial

<220>
<223>  CDS

<400> 17

TTCATCATAG CTCCTTAAG                                                    19

<210>  18

<211>  19
<212>  DNA
<213>  Artificial

<220>
<223>  CDS

<400> 18

AGAGAGTTTG TGAGTACTT                                                    19

<210>  19
<211>  18
<212>  DNA
<213>  Artificial

<220>
<223>  CDS

<400> 19

TTCCTATAGT AAAGCCAT                                                     18

<210>  20
<211>  19
<212>  DNA
<213>  Artificial

<220>
<223>  CDS

<400> 20

TTTGACAAGG TGAGTTTAG                                                    19

<210>  21
<211>  19
<212>  DNA
<213>  Artificial

<220>
<223>  CDS

<400> 21

TTTTCTTTAG TCCAAAGCA                                                    19

<210>  22
<211>  19
<212>  DNA
<213>  Artificial

<220>
<223>  CDS

<400> 22

GATTCTCAGG TTAGTTTGA                                                    19

<210>  23
<211>  19
<212>  DNA
<213>  Artificial

<220>
<223>  CDS

<400> 23

CCTTTTTTAG GACATGTTC                                                    19

<210>  24
<211>  19
<212>  DNA
<213>  Artificial

<220>
<223>  CDS

<400> 24

GTGCTAAAGG TAATTGTGC                                                    19

<210>  25
<211>  19
<212>  DNA
<213>  Artificial

<220>
<223>  CDS

<400> 25

ATTTCTACAG AAATTGCCA                                                    19

<210>  26
<211>  19
<212>  DNA
<213>  Artificial

<220>
<223>  CDS

<400> 26

GATTTATCTG TGAGTAAAA                                                    19

<210>  27
<211>  18
<212>  DNA
<213>  Artificial

<220>
<223>  CDS

<400> 27

ATTTTATAGG GTATTCTG                                                     18

<210>  28
<211>  19
<212>  DNA
<213>  Artificial

<220>
<223>  CDS

<400> 19

TTTTATAAGG TATTTCCCA                                                    19

<210>  29
<211>  19
<212>  DNA
<213>  Artificial

<220>
<223>  CDS

<400> 29

TTTATTTCAG GTGATAGAA                                                    19

<210>  30
<211>  19
<212>  DNA
<213>  Artificial

<220>
<223>  CDS

<400> 30

TGTGAAGAGG TGAAAATCA                                                    19

<210>  31
<211>  18
<212>  DNA
<213>  Artificial

<220>
<223>  CDS

<400> 31

TCTTCATAGG TCATGCCA                                                     18

<210>  32
<211>  19
<212>  DNA
<213>  Artificial

<220>
<223>  CDS

<400> 32

TTGGAAGGAG TAAGTTTAA                                                    19

<210>  33
<211>  20
<212>  DNA
<213>  Artificial

<220>
<223>  CDS

<400> 33

TTGACCCCTA GGCATAACAT                                                   20

<210>  34
<211>  19
<212>  DNA
<213>  Artificial

<220>
<223>  CDS

<400> 34

CTGTGCAAGG TAAGGAAGG                                                    19

<210>  35

<211>  19
<212>  DNA
<213>  Artificial

<220>
<223>  CDS

<400> 35

CTGTCACTAG GTATTGCCA                                                    19

<210>  36
<211>  19
<212>  DNA
<213>  Artificial

<220>
<223>  CDS

<400> 36

TTTAGAAAGG TAATTTTTC                                                    19

<210>  37
<211>  19
<212>  DNA
<213>  Artificial

<220>
<223>  CDS

<400> 37

TATCTCCTAG GTATACCAT                                                    19

<210>  38
<211>  19
<212>  DNA
<213>  Artificial

<220>
<223>  CDS

<400> 38

ATGGCAAAGG TGAGTACCA                                                    19

<210>  39
<211>  18
<212>  DNA
<213>  Artificial

<220>
<223>  CDS

<400> 39

GTTTGCCAGG TCACAAAA                                                     18

<210>  40
<211>  19
<212>  DNA
<213>  Artificial

<220>
<223>  CDS

<400> 40

CGGAGCCAGG TAACTACAT 19

<210> 41
<211> 19
<212> DNA
<213> Artificial

<220>
<223> CDS

<400> 41

TTCTCTAAAG GTGTATAGA 19

<210> 42
<211> 19
<212> DNA
<213> Artificial

<220>
<223> CDS

<400> 42

AAGATAGAAG TGGGATCTT 19

<210> 43
<211> 19
<212> DNA
<213> Artificial

<220>
<223> CDS

<400> 43

CTGGCTGCAG CCAGTAGAG 19

<210> 44
<211> 19
<212> DNA
<213> Artificial

<220>
<223> CDS

<400> 44

CAGGCAAATG TAAGTATGA 19

<210> 45
<211> 20
<212> DNA
<213> Artificial

<220>
<223> CDS

<400> 45

TTTTTAAACA GATGGGATGC 20

<210> 46
<211> 19
<212> DNA
<213> Artificial

<220>
<223>  CDS

<400> 46

CCTTCAAAGG TGAGCCTAA                                                    19

<210>  47
<211>  19
<212>  DNA
<213>  Artificial

<220>
<223>  CDS

<400> 47

CCCACCATAG AGACTGAGA                                                    19

<210>  48
<211>  3865
<212>  DNA
<213>  Artificial

<220>
<223>  CDS

<400> 48

GTAGGTTTTC TTGTTGGTTC ATCAGGAATA CACATTAGTC TGTGCTGCAG TGTTGATATT      60

CTGCTAGGTT TTTTTTTTCT GGTTTTAAAA AAGAAATAAG ATTTAAAAAA TCTTTTTCCT     120

CAGTCGTTTT CTTTTAATGA TGCTTCCGGG GCTTCACATT GTGGGTTAGC CATGAAGAGT     180

GGCTTTCACA TATTGCTAAA TGTATACAGG TCTGTGTTTC TATAAACTAC ATGTGTCTTA     240

TTTCATTTTA TTATTATTTA CCTCCTCAGT GATCCTTGTT CTGAAACCTT CCTTTTTCAT     300

TTAAGCAACA AAAAATGCAG ACTGTACAAG TCAGACTTAG GGATTTTCAC CCTTTCGCCG     360

CCTTGGAGAG TTCTGTATCT GTATCTGGAT ATATATATTT TTTATTGCGC AGGGGCCATG     420

CTAATCAATG TATTGTTCCA ATTTTAGTAT ATGTGCTGCC GAAGGGAGCA CTGCCCTAGA     480

TATAGATCAC TATATTAACC ACTATATTTT CTACTAGTGA TTATATAGAC TATTTTATGT     540

CAAACTGAGT AATAAATAAT CCCCTTGAAA TGACTTCTCT ATGTATTTTG ATGTTTATAA     600

TGAATTCAGA ATAGAGAGAC TGGATTGGGA AAAGACAGGA GAACTGAAAC TATTATGAAT     660

TTGTGCTTTC TGATCACTTC TGCAAAGTCT ATAAGCATGC TCTGACTCAG TGTTTTCTAC     720

CTTTCCTGAT AGATAAAGGC AGTTATGGAA TACACATTTT CCTTCTTTAT CATTGAAAGT     780

TTTTTCATAA AGTAGAAATG AAAATTCTAA CAATTAAAAA AATGTTGACA AGAAAAGTAA     840

AGGGAAAGGA GTTAAAATTA TTTGGCTAGA ATAAATAATG TTTGCTTCTC TTTAAATATA     900

AAAGTTTTCC CAGACTGTGA AGGATGTTTA CATTAAGTGT AACCTTTTAA AAATAAAATG     960

GAATGACAAA CCAGGAGGAA AAAAAATTTA AAAAAACTAG AACTATTTAC ATTTTAATAT    1020

AGATGGCACC ACTGATACAG AAGCATCTGG TCTAGCTCAC TTACAGTTTT GGGGAATTGA    1080

CTATTTAAAA TGAAGCATTC TGAGCCAGGC GGGTTGGCTC ACGCCTGTAA TCCCAGCACT    1140

TTTATGAGGC TGAGGCAGGC GAATCACCTG AGTTCAGGAG TTCAATACCA GCCTGGCCAA    1200

CGTGGCAAAA CCCCGTCTCT ACTAAAAATA CAAAAATTAG CTGTGCATGG TGGTGCATGC 1260
CTATAATCCC AGCTACTCGG GAGGCTGAGT CAGTTGAATC CCTTGAACCG AGAAGCAGAG 1320
GTTGTGAGCC AAGATCGTAC CATTGCATTC GAGCCTGGGC GACAGAATGA AACTCCATCT 1380
CATAAATAAA TAAATAAACT AATAAAATGA CATATTCTCC TAGCACTTTG GGAGGCCGAG 1440
GCAGGTGGAT TGCTGGAGGT CAGGAGTTCA AGACTAGCTT GGCCAATGTG CCAAAACCCC 1500
ATTTCCATTA AAAATACAAA AATTAGGCAG GTATGGTGGT GTGTGCCTGT TGTCCCAGTT 1560
ACTTGAGGGC TGAGGCAGGT GAATCACTTG AACCCAGGAG TCGGAGGTTT CAGTGAGCTG 1620
CGATCGCGCC AATGCACTCC AGCTTAGGTG ACAGAGTGAG ACTTCGTCTC CAAATAAATA 1680
AATAAAAAAT GAAGTATTCT AAAGGTTTGA ATAGAAGCTT TGTACTGAGT CTGAGTGAGG 1740
CCAATGTGAT CATTTATGGG AAGATATCTT CTTTGTTTGG AGTATCTGGA AAATAATTTC 1800
AGATTGCACT TGTTTTGCTA TTTCTTAGGA TATATATACT ACCTAATTCT AATTAAGAGA 1860
ATTTTAAAAG GCCATGTGCA GTGGCTCACA CCTGATCCCC AGCACTTTGG GAGGCTGAAG 1920
TGGACAGATC ACTTGAGCCC AGGAGTTTGA GACCAGCCTG GACAGTATGG CGAAACTTCA 1980
TCTCCACAAA AAATACAAAA ATTAGCTTGG AGTGGTGGCG CACACCTGTG GTCCCAGCTA 2040
CTGGGGAGGC TGGAGGTGGG GGATCACTTT GAGCCTGGGA GGTTGAGGCT GCAGTGAGCT 2100
GTGCTCATAC CACTGTACTC CAGTTTGGGT GACAGAGCAA GACCTTGTTT CAAAAAAAAA 2160
AAAAAAAAGT AAATCACTTT ATTAGAGATT TTACATTTTA ATCACTTTGT ATACTTTCTG 2220
TTAGCTCTTT CTGTTAACTA TAGTCATAAT GTATAGCACT TACTGAGCAT TTACTTTGGG 2280
GCAGGGACTC TTAAGACTTC AATATGTATT ACTTCAGTTA ATCCCTCTGA CAACCTTGTG 2340
ATACTCATAC TATTGTTAGA TAGAGAAAAT TAACCGCAGA GAGGTTAAGT AATTTGGCCA 2400
GGGTCGCACA ACCAAGCGTG GAGTTCTTAT TGAAACTGAC TGCGGGAACC CATGTGCTTT 2460
ACTGTGACTA TATACTGCAT CTCTCACACA CTATCTGAAA ATGTGTCACT ATTTGTTTAG 2520
CACTTATCCA CAGGAAATAC TGTCAGGTAT TATGTAGGAC ACAAGCATTT TTTAAAACAC 2580
CAAACCCCAC AGTTTTTGTT TTCTGAGAGC TTACAGTACA GTCAGCGAGA TGAGGCAGGT 2640
ATGAAGATTC CAGTGCATGC AATGCAGTGT GTTATAAAAG TCCCATGACT ACCAGAGGGA 2700
ATACAGATGT AAAACTTAGG AGGAAAAGAA ATCACTCTGG ATGAGCCAGT CAGGTAAGTT 2760
TACATGGAAT AAGTAGAAAT GGGTCTTGAA AGATGGGTAC GAGTTTGATA GGTGAATTTG 2820
AAGATACAGA TAGCACCTTC TGTGTAGAGG AAACAAGAAA AGACAAAAGC AGTAAAGCAA 2880
GAAGAAATGT GGGAGGTTAG TCAAGTTTTT TTTTCTAGAA TTCTCAAGTT GTAGAGCCAG 2940
AATTAAGAGT AGCTTAAGTG TTAAGCTAAA AAAAATTGAA TTTTATTTTG GTAGGCAACT 3000
AAAACTAGAA ATAGTTTATC ATGCGCCTAT GGTAGAGAGG ATACTTTTAA AAGCAGAACA 3060
CTGACATTTA ATCCTTGCCA TGGAGTGGTG AACTAAGTAC AGTATTGTAC CCAAGTAGAG 3120
TAATCTTTTG ACAGATGAAA TGACTAAGGC CCAGGTGAGC AAGTGTACCC TAGCTAATGG 3180
CAGTGCTGGA ACTAAATCTA ATCTAATCTT CTCCACGGAA TTTCGTTCTT CTGGGCACCT 3240

TGTTAGAATA AGGCTGTTGG GAGGTGGAGA CCACAGATTT CTTGTCTAAA AGTTGTCAGA 3300
GGTTTTGGTA GAAAAGCCAA GCTTAAAGCA GGTCTGAAAC TTGGCAGACT ACTTGGCAAT 3360
ATACAACAGG TACTCTTAAT GGATGGAAGT ATAAGGAATT ATAGGAAGCT CATAATTTAC 3420
ATTAAAAAGG CCTTTTGTGA TTTGATATAG TCTGGAATAT CTTTAAGGAG GGAGGGAGGG 3480
ATACAGGTCA TTAGCTATGA TAAAGGAGAA AAAAATAAGG ACATATCTGA CTGCATATAG 3540
TGGTCCTGAA TCAGCATAGC ATTGCTGTGT CATCGAAAGA ACTATTTTTA TTCATTTTAT 3600
TTTCCACCTC ACCTATCTTG CCTTCACAAA ACTTTAAAAG ATTCTTTAAG AATTTTCTTT 3660
TCTTTGAGAT GGGCTCTTTC CCTGGTACCC AGCTATTTCC TACCAATATT TTGTTAAGGC 3720
AGAACGTCCA CGTTTTCCAT GTGAAGCTGA ATCTGTTGTC TCTCCCTTTA ACTGTGGGTT 3780
TTATTTTACA CCTGATTTAT AATCATTTGG GATTTTTTTT TCTGATCTTC TGGTGTCTCG 3840
TGACTGGGGT TTTCTTCCCC CAAAG 3865

<210> 49
<211> 4576
<212> DNA
<213> Artificial

<220>
<223> CDS

<400> 49

GTAAGAAAAT AGTAATATTT ATTTAGATTT AATATGTCTA TTTACATTAC CAGGTATTAA 60
TCTCGTCAAC TCCTAATATG TATCAGGAAA AGATTTCCAC TGAAAATTTT CTCAAGGGTT 120
TTAATCCTAG ATTCTTTTTT AAGTATTGCC TTTCCATCAA AGGATCTATT GGATTTCTTT 180
ACAATATCCA AATCTCTCTT ATTAAATGGA AAGTCCATTA ACTTCGTTGT ATACAACATC 240
TTTCCTACCC AAAGCTACTC TCCTCAAATT ATGAGCTGAA AACACATAAT CCTGTATATG 300
CTTGTATTGC GAACTCTATC TTCCATGAGA TGTATCTTAT TTAGTCTGAG CGCAATTACT 360
GATCAACCTC AGAGCTGTTC AGATTTTTTT GTGTGTCTTG TTCACATAAG TATACTTAGT 420
CAAATGCTTT TATATACTAT TTATTTTCTT TCCCTTTTTT CTTGTCTCAT TTAACCTACC 480
CAAGGTCTGC ATTCAGTGAA ATACATGTCT CTATTATTTT TTGTCCTTTT TGTATTTATT 540
TATTTATTTA TTTATTTGAG ATGGAATCTC ATTCTGTCTC CCAGGGCTAG ATTGTAGTGG 600
CACAATCTCG GCTCACTGCA GGCTACACCT CCCAGGTTCA AGTAATTCTC CTGCCTCAGC 660
CTCCCGAATA GCCGTGATTA CAGGCGCCCA CCACCATGCC CAGCTAATTT TTGTGTTTTC 720
AGTAGAGATG GGGTTTCACC ATGTTGGCCA GGCTGGTCTC AAACTCCTGA CCTCAGGTGA 780
TCTGCCTGCC CTGGCCTCCC ACAGTGCTGG GATTATAGGC ACGAGCCACT GCGTCCAGCA 840
CCTTAGTATC TTTCTATGTA GAACGAATGC TCCCAGGTAG ATGGGAAAGT GCAGATATAT 900
TATTATGTAG TCAGCTCCTG TATACCATGT GGCTTGGCCT TCGTCACTAA GATGGCTCAC 960
TCTGAATGCA AAGTTATCAC AGAGTCTTAG GTGCTGGAAG GAGTTGCACA GGTATCACTG 1020
AGACTCTCAT TATTAGATTA ACTAGCTTAA CTTACTTTAT TTTTTTTTGA GATGGAGTCT 1080

CACTCTGTTG CCCAGGCTGG AGTGCAGTGG TGCGATCTCG GCCCACTGCA ACCTCTGCTG 1140
CCCGGGTTCA AGCGATCTCC TGCCTCAGCC TCCCGAGTAG CTGGGATTAC AGGTGCCTGC 1200
CACTGTGCCC GGCTAATTTT TTGTCGTTTT AGTAGACACG GAGTTTCACC ATCTTGGCCA 1260
GGCTGGCCTT GAACTCCTGA CCTCGTGATC CACCTGCGTC AGCCTCCCAA AGTGCTGGGC 1320
TTACAGGCGT GAGCCATCGC ACCCAGCCTA GCTTAACTCA GTTACTTTAT TTTCTATTTT 1380
TATTTTTATT TTTGACACAG GATCTTGCTC TGTTGCCCAG GCTGGAGTGC AGTGGTATGA 1440
TCTCTGCTCA CTGCAACCTC CGCCTCTTGT GTTCAAGTTG ATTCTTGTGG CTCAGCCTCT 1500
TGAGTAGCTG GGATTGCAGG CATGCACCAT TATACCTGGC TAATTTTTGT ATTTTTAGTA 1560
GTGTTGGGGT TTTGCCATGT TGGCCAGGGT GGTCTCGAAC TCCTGACCTC AAGTGATCTG 1620
CCACCTCGGC CTCCCAAAGT GTTGGGATTA CAGGTGTTGA GCCACCATGC TCAATCAGCT 1680
TAGTTACTTT AAAGATTAGG CAGCTGAGCC CAGAAACTAG CTGCTGGGAA CAAAGCTAAG 1740
ATTGAACTCA GATCTCCTGG TTCCTGGTTC TTAGTTTCAT ACTGGCTGTG AAGGCCTCTG 1800
GGAAGAATGT GTTACATTGT TGGTCTCCAG GTTTGATTTG TCCTGGTCCC TCTCTGGCTA 1860
ATTAGGGTGA GAGCCGCCAT CCTTCCTTCC CTGAGCTGCA TGCTTGATTC AAGAGAAAAA 1920
TCTTTCTTTT GTCATACATG ACACTGGCAT GTTTCTTTAA TGATGATAAA GGCGACATGA 1980
TCAGTGGCAT GAAATAAAGG TTTTGGAGTA TATAAACCAT TTTTACAGCG GCTACAAATT 2040
TTAGAATGTG TGACTGCTAT TATGTATGAT GGTAATCTTT TCATATGATT GTATTGGGCA 2100
AGTATGTCTC ATTTCTAGGG TTTTTATCTG TTTTGTTTGT CTTTTATGGC ATATGTGTAC 2160
TTAGAAGTAA ATATAGTTGG TACTATATAT AATATGTACA ATACAATAAA AAATAATTTC 2220
ATTGTCCTTA TTTTGTTCTC ACTGGACCTG TTGGGGTGGT TTTTTCTCTG TAATTAACTC 2280
AGTGTTTGAC TTTTATCTCA TTAATTCAGT TTATAATAAT TCCACCTTAA GAACCTTTGT 2340
GGATTGGGCA TGTTGGCGTA TGCCTGGAAC CTAGCTACTT GGGAAGTTGA AGTGGGAAGC 2400
GGAGGCTGCA GTGAGCTGAG ATTGCACCTC CAGTTTGGGC GAATTTGAGA CCGTGTTTCG 2460
AAAAAAAAAA AAAAAAAAAA AGAAACTTGG TCCTTTCACA GTCCACCACT GTGATCTTTT 2520
ATAATACACG ATGATCTTTT TCTAATAGTC ATTTAATTGC TTTAATTCAG TTCTCATTTA 2580
TTTGGGGGAA AGGTGTACTC TTTTATAGCC ACCTTTCTAA TGACAAATAA GCCAACTCTG 2640
GAGATGAAAC ATTTCTATTT ACTTGTTATC TTTGTTGATT AAAAGATAAA ATACCTCACA 2700
AAGTCAGATT TATTTGTAAG GTCAGGATTT GAAATAGAAA ATACGTCATG TTGAGAGAGT 2760
CCTAGAATTT AATTTAAATT AGATTCTGAT CTTTAGGGGC ATTTCAGCTT TTTATTAGAT 2820
GTTACGAGTA CTGTTTTTTT TTTTTTTTTT TTTGCCTTCT ATGGCAAGTG CACACCAGTA 2880
ACAAGTTTAG GCTTGTTGGT GTGATGGGCT TTGTAGCTTG AAATCAGTAG GTGCTACTTA 2940
CTTACTTTTT TACACATGAG GAACCAAGTA TATTTTAATA TTAAACCTCT TTATAGGAGA 3000
GCCAAGCAAG TTGGTTTGGC TGTATCAATG CGCAGTTTGA TGTGGTGATT ATCGTTTGCC 3060
TGCTTTGGCA GAGGAGGATT TTTTTTTCTC TTTAGTTCAT TTAAGTTGAT TTGTTGAATG 3120

TTTCCATCTA AACAAAAAAG AATTGCTTTG TATACGCTGA GGTAAGTGGT AACTTTCTTT 3180

GGAGGAACAG AGAGAAAGGG AAACCTGAAA CAAAACTGCA GGTGTGTGTG TGTGTGTACA 3240

TGTACACTTG GGTAGGCGTT AAGTGTGAAA TGCTGAGGTT TGGAAATAAT TCTTCATATG 3300

TATGTTAGCT TATTTAAATT GAATTTATCT GATGATACAA GAATGTAAAA TCACCATGAA 3360

GCATACATGT GCAGTGTTTA ACTAAAAAAG GATGGGCTTG AAGTTATAAA ATAACTAGAA 3420

ATAATTCTTA ATTTCTAGAA AATTAAGATA ATAATAAAAT GGTTTAACTA CACGTAAAAA 3480

TGTGTTCAGT GTTAGAGTTC AACCAGCACT GCAGAAAATT ACATGTTTCT GTCAGTTTAG 3540

GTTTTTGATT TCTTATTTCC CTGTTACCAA GCATCAGCAA TTATTCTTGG GATTATTAGC 3600

CCTGGAATTG AAAGATATTT AATGGTACTC CTGTTGCATT AATTTGTCTG AGTTTATGTA 3660

GAAAAGTATT AAAAATGTTA CTGTTGGAGT CTGATAAAAA GTTCTGGTCT TTTAAAAATA 3720

TGTGTATGAG AAATAGCATG AACTCAGGAG GCAGAGCTTG CAGTGAGCTG AGATCGTGCC 3780

ACTGCACTCC AGCCTGGGCG ACAGTGAGAC TCCATCTCAA AAAAAAAAAA TGTATATGAG 3840

AATAATTAAG TGAATTATTT TTTCGGCTGT CTCCTAAGTA TTTCTAATAA TTTTCATGAC 3900

AGAAAAATGT TTTCATGCAA AACAATTTCC TTACAGTTTG AGATAATTTA TAAATGTTTT 3960

GTGTTCAGAA TTTTCAAAGA AAAGACCAAT GATAAAGTTT TATTCAGCTA CTAGGTATTT 4020

AATAAACACT TAATGATGAA TGGCATTTTT AGTAAAGTTA TAGTTTTCAC TAAGCTGTTA 4080

GACATTTATT AATTTATTAA AGGCCAGGCA TGGTGGTTTA CACCTGTAAT CCTAGCACTT 4140

TGGGAGGCCA AGGCAGAAGG ATCACTTGAG TCCAGGAGTT CAAGACCAGC CTGGGCAACA 4200

TAGCAAGACT CCATCTCTAA AAAAAGTTTT TAAATTAGCC ATGTGTGGTG GCGTGTACCT 4260

GTAATTTGCA GCTGCCCAGG AGGCTGAGAC AGGAAGCCCT TGAGCCCAAG AGGTTGAGGG 4320

TGCAGTGAGC CATGATCATA CCACTGTACT CCAGCCTGGG TGACCCACCA AGACTCTGTC 4380

TCTTGAAATA AATAAATAAA GAAATTTATT AAGATATTAG AGTAATATGT CGGATGTAAA 4440

TTTGCCAAAA CACTTATTGT AATGAGTCAA TTTTGTACAA TTGTTTTGTA ATGTCATAAT 4500

AAGAAAGGAA GAAATTTTTT AAAAATGTTA CAAAGTCAAT GCTAATTTAA CTCTGTAACT 4560

GCTTATAATC CTGCAG 4576

<210> 50
<211> 1618
<212> DNA
<213> Artificial

<220>
<223> CDS

<400> 50

GTAGGTTTGT AAATCAAAGA TTTTTGGGCA ATCTGCGTTT CTGTGTTATG TTTACCCTTG 60

GAGTTGTACA GGTTTCCTAG CATCAGTATT TTGAAGAGCT CCTGTCATTA CGGCTATCCA 120

GGGTACTTAT AACTAAGAGT CAAGCTGCCT GTAAAAATAT TTTTGGATAA ACAGTTGCAG 180

ATACCACAAA GTTTAAAGTC TTAAATGACA ACTTCAAGAA GTTTCTGAAA TATATACTCA 240

ACAAGGAGAA GGCATTTAGA AACTCAGAGT TGCGAAGATG ACATTAAAGC CGATAATGTT 300

TCCTACATTG GCAAACTTTG TGCCTGACAC ATTGTAGGAG ATCAAAAAGA ATTTGTTGAA 360

AGAATCTTAC TTCAAATTTT GGTACAGAAG AATAGTTATG GTTCTAAAAT AAAGAAAATG 420

AACTTTCATC TTTTAAACTA ACAGATATAT GGAAATGATG ATTTTGGCAT TGCATTTAAT 480

AGAACTTAGG TATATAATTT CTATGAATGA TAAACAGTTA CAAGCCCAAA TTATGATTTA 540

CAAAGCAAAT ATTAAAAAGT ATGTATAGAG TTAAAATAAA TATTGCTGCT GCTATTTGAG 600

TAATATTGTA ATAGGATTCT GGGTGATTCT CAGTTTGGAG GTAATTTCAG TTAAAATTTC 660

AGCTTGTCTA TCAAGGTAGA TTTTTAAAAT TAGTGGAGTT CAGTTGCTCC TGGTATGGTA 720

AATTTAATGT TCCTCATCTT CTTTTCTGTT CTTTCTCTCA TTTCTATCAT AACTCCCTTG 780

TATATTCCCA AAAAGCTGCT TCCTTTCACT TTTATCTTTT TTTGGTTTTA AATTAAAAAG 840

AATTTTTTTT TTGGAGACAG GGTCTCACTC TGTCACCCAG GTTGGGATGC AGTGGTGAAA 900

TCACAATTCA CTGCAGCCTC AATCTCCTGG GCTCAGATGA TCCTCTCATC TCAGCCTCCC 960

AGGTAGCTGG GACTACAGAC ATACACCACC ACACCCAGTT AATTTTTTTG TATTTTTCAG 1020

TATAGATGAG GTTTCACCAT GTTTCCTGGG TTGTCTCAAA CTCCTGGACT CAAGCGATGT 1080

ACCCACCTTG GCCTCCCAAA GTGGATTATA GGAATGGAGC CACTATGCCC AACCTTTACC 1140

TCTTTTATTT TTAGTTGATT TTTTTTCTTT TGTGCTGAGT CTAGGGCAAG AATAAATTGT 1200

AAACTAGTAT GAAATACATC TAATACATTC AAATTAAAGA TATAAATATC TGAACAGTGT 1260

AATTTTTTAA AGTGGTGTTT TTTGTTTAAA AGTAGACTTA CTTGCAAAGT TGTATTTTGT 1320

GGTTTTTAGA TCTTAGTATC CTAAAATTTG ATTACCTAAA ATTTAAGTTT TAAGTTTCCC 1380

TTAACCATCT CTACATAAAT AATTGAATAA CTGAAATCTT TCGAGTAATG ATACACTTTA 1440

CTTCTATTTG CCATTTTTTG ACAAATTCTT AGTGTTGAAA TAGGCCCATA TATACTGTTT 1500

CCTATACATT TGTATGCTAA GTGGTATACT GATTATACTC TATGTTTTAC ATTTTAGTTT 1560

ATTACAAATT GGCTTATTGT GTGCTGATAT CTCTGTTTTG TGATTCTATA CACCATAG 1618

<210> 51
<211> 92
<212> DNA
<213> Artificial

<220>
<223> CDS

<400> 51

GTTTGTAAGT AGCAAAGAAA TAACGTGAAA ATGTTTTCTG GAGAAAAACT TGATTTAACA 60

TGACGACTTA AGGATCTCTT CTTTCATCAT AG 92

<210> 52
<211> 889
<212> DNA
<213> Artificial

<220>
<223> CDS

<400> 52

GTGAGTACTT CTGTATAAAA TGTTTTAATA TTTTAAATTG TATACTTAGG AAACTTCAGA 60

AGTTAGTGTT TTTATTGTTT GTACTCTGGA AACTGAGAAT ATGTTTTGTG AGAGAATACA 120

GGGAAGCAAA AATTCTGTCA CCTAAATATA AGCACACTTT TTAAATGTGT TCAAAATTGT 180

ATGGCTGTCT CCGAAGTTTC TTTAAGCTTC TGGATTATAA ATTCTGAAAT AAATTCTCTG 240

GGAACTATAT GGGTGAAAAT TGATGATGTG TAAGTGTGGA AAGTCTTCAG GGGTGCCTAG 300

AGCAGCTAGA CAGATAGTTA AGCTTCTCAC CGGAAGTTGC ACCTACCAGC AGCTGAAACA 360

CTGTCAGCAA AAATACTTGT CCTGTGTGAT GGATGAGCTT GGGGATAGCA GGATTACATG 420

TGATACTATC CAGTTTTTGT TTTGTTTTGT TTTTTGAGAT GGAGTCTCGC TGTGTCGCCC 480

AGGCTGGAAT GCAGTGGCAT GATCTCGGCT CACTGCAACC TCTGCCTCCC AGGTTCAAGC 540

GATTCTTCTG CCTCAGCCTC CTGAGTAGCT GTGAATACAG GCACGTGCCA CCATGCCCAG 600

CTAATTTTTG TATTTTTAGT AGAGACAGGG TTTCACCATA TTGGCCAGGC TGGTCTCAAA 660
CTCCTGACTT CGTGACCACC TGCCTCAGCC TCCCAAAGTG CTGGGATTAC AGACGGGAGC 720

TACTGCACCC AGCTATACTA TCCAGTTCTT ATAACTACAA GTTACCCTAC CAAAGTTTAA 780

CTTTCCAAAA AACTATTAGA ACTTTTAGTA AATAAAAAAA TGAAATAATT AATTGAAATG 840

GCAGTTTCTG TGAGAGAGTA CATTTTGTCT GTATTTGTTT TTCCTATAG 889

<210> 53
<211> 4586
<212> DNA
<213> Artificial

<220>
<223> CDS

<400> 53

GTGAGTTTAG CCATGCCAGA AGAGTAGAAA TACCAGGAGC AGGTAAGCCA GGGGTTCTTT 60

TTTATTTGGG TAATTTCATG TTTGTGTTTT ACTTGCCTAC AGTATGAAGG AGAAAATTCT 120

CATCATACTT CTCTTAATTG AAAAAGGTAT CTCTATGATA TTTGCTTTGT TAATATCAAC 180

TTTCATTCAT TTTAGTGAGG TCTGAGAAAA GAAATTAATA TAAATTTAAA ACAAATGTGT 240

CATGCTGATA ATTGTTGGTT TTAAAAAGAT GGGCCAGTAA TATATGGTCT TATATGTAGT 300

GAACATAGTG TAGGCATTTA GAAAGTGATA ATTGACCTGA CTGGGGCCTT CATTTAAGAG 360

ACTGGAGTAA AATGAGGATC TACAGTCTTT AAGAAAATTC TTTCAAACTG AATTTCAGGA 420

CCACGTGGTA TTATTTCTAA CAGACACTTA GAGTGATGCA GGCCAAGAGT TTCCCTCCTG 480

CTATGTGGTG GAACAGAAAA CACCAAACTT CTGGAAAGTG CCACCAGGGG AAACACTGGG 540

TAATCCAAGG GCCAGTTCAC CTGGATAGTG AGCTGCTTCA GACTTGAGAC TGGTCTGCTT 600

ATTCATTCAA CAGATATTCC TAAAGCATTT TATATGTCAG GTTGTGTCCT GGACACTGGA 660

GATAAAGCAG TGAACAAAAT AACCACGAGA ACCCTGTTCT AAAGAAGCTT ATATTCCAGT 720

GTGGGGAGAT GGACAGGAGA TAAACAAGTA AATATATAGT ATGTTGGGTG ATGATAGATG 780

AAGAAAATAG AGTAGTAATA CAAAATATTG AGGGGAGGGG AGAATGGGAT GGCTGGGCTG 840
TGGTAGGTAA GGTGGTTGGG AACGGTGTCA CACACCAGAA GTAAGTGAGG AAGCAAGCCA 900
TATGAATAGC TGGGTAAATG TATTTGAAGC TGAGAGCATA ACAAATGCAA AGCCATGAGG 960
TTGGAACAGG ATTAGCTTTT TGGAGGAACA GTGAGAATGC TAGTGTGGTA GGAATAGAGT 1020
GAGGGAAAAA GTGGTAAGAA GTGACGGGAG GCCAGGTGTG ATGGCTCATA CTTGTAATCC 1080
TAGCACATTG GGAGACTGAG GCAGAAGACT GCCTGAGCCC AGGAGTTCAA GACTAGTCTG 1140
GGCAACAAAG TGAGACCCCG TCTCTACATA AAATATTAAT ACAAAAAATA AGCTGGCCAT 1200
GGTTGTGTCC ACCTGTGGGC CCAGCTACTT GCGAGGCTGA GTTAGGAGGA TTCGTTGAGC 1260
CCAGGAGTTC CAGGCTGCAG TGAGCCGTGA TCGCGTCACT GCCCTCCAGC CTGGGTGACA 1320
GAGCAAGAGC CTGTCTTTAA AAAAAAAGAA AAAAAGAAGA AGAAAAAGAA ATGCAGGGAA 1380
GAGGGAACAA GAGAGCCAGA CAGACCGTGT AGGCTTTGGA AGCCATCGTA AGGACTTTTG 1440
CTTCTGCTCT GATTGAGGTG AAAGCCATTA AGAGGGTTAT TAAGAGGAGT GACTGATTTA 1500
CATTTTTAAA GGTCTTCTGG GAAAGTGGGA TTAGAGGCAA GGGTGGAAGT AGGGAGTTAA 1560
GAAGCTATTG GAATGATTCT GGCAATAGTT TATGGTGGCT TGCTTCAGAA AATGGTTTGT 1620
AGCTGGGCCA TATTTTGGAG ATGGCACCCA CAGGATTTAC CGAGGGTTTG TATCTAGGGT 1680
ATGAGAAAAA GAGAACAGTG ATGTCTCCAG TTGGGTGAAT GATATAAAAG CTAAAATCCT 1740
GACAAGTGCC TGTAATGTTG TAAGTTATCT GGCCCTGGCT CTCTCTGAAT TCATCTACTT 1800
TCCTCCCTCC TCACCCACTT ATGCCACATT AACCTCCTTT TTTGTTCTTC AGATATGCCA 1860
GGCATGCCTG CAACACAAAG CCTTTGCCTT TGCAATTCCC TCTGCCTAAA CTGTATTGCT 1920
TCAAGAGATT CATGTGGCTT CCTTCTCACT TCATTCTGGT CTCTGATAAC CCAACTGCTA 1980
TGTCAATAAT AACCACAACA TCCTCCCCAA CCCTCAGGAC TTCTTTTCCC CCTGACTCTG 2040
CTTGCTAGTG TTTCTCTTCG TATTTATCAC TGTCTGACAG TAAGTACGGA CGTACGTACA 2100
AAAGAATTGT TTATTACCTG TCTCCTTGCA TTAGAATATA AGCTTCACCA AGGCTGTGAC 2160
CAGTGTTGTA TGCAGCGCTT GGCACATAGT AAACATTCGG GGAACATTTA CTACTGAAAT 2220
TTATTAACCA GGGAACAAGT CTGGGGGAAC GGGAATCAAC AAGTTACGGT TATTACCATG 2280
TTAAATTACA GATGTCTTTT AAGCATCCTA CTAGAGAAGT TGAATACACA CTTGAGGTAT 2340
ACAAGACAGG AGTTCACAGT TCACACTACA GGTTAGGGGT TGTGTATATA TGTCCTGGGG 2400
TCATCAGGGT GGGTACAGAT AGCCTTTTTT TTTTTTTTTT TTTTTTTTTT TTTTTTTTTT 2460
TTTTTTTTTG AGATGGATCT CGCTCTTCAC CCAGGGTGGA GTGCAGTGGT GCAATCTTGG 2520
CTGCAGCTGT GACCTGTGCC ACGGTGGGTT GCAAGGGATT CTCCTGCCTC AGCGTCGTGA 2580
GTAGCTGGGA TTACAGGTGC CTGCCACCAT GCCCAGCTAA TTTTTTGTGA TTTTTGAGTA 2640
GAAACGGCAT TTCACCATCT TGGCTAGTCT GATCTTGACT CCTGCCCTCA TGATCTTCCC 2700
ACCTCGACTT CCTGAAGTGC TGGGATTATA GGCGTGAGCC ACCATACCCA GCCGTAGATG 2760
GCTGTTAAAG CTATAAAATG AGGAGGGATT ACTTAGAGGT ATGAATTGAG AGAGAATACA 2820

AGAGGTCTAA GGACAAAGCT CAGGGTCACT CCAAATTTTG TAAGTCTTCA TTTGGAGATG 2880

GAACATCCTA ATATTTTTAA GATACCGACT TAATATTTGC ACCCAAGTTA AAGATCCTCT 2940

TGATCAGAAT GAACAGGAAG CTTTAAGCTA AGCACAGTGC TACCAAGAAG CACCATGTTG 3000

ACCTTGAGGA CTCTGGCAGG AAGCTGTTTG TGGTTGTCAC ACCTAGTTTC CTCTGTGAAA 3060

CTACTGCTGC CTGTGGGTGA TGTGGTTATA TGCTGCTGGC TGCTGTTGAT TCTCCTGTTT 3120

GTGTACAAGG TGTTTTTCCC TCCCAGTACC TCCCAATGTA GGCATCGGTT CATGCACAGT 3180

GAAGTAGTTG CCTGCGAGAA ACCTTGTAAG GCAGGGAGCA GCCTTTTGAA TGCAATAATC 3240

TACCCGAATC ATTTTAATGA CTTAATTATA GAATGAATTT CTTTGAGACA AAGTGAAAGT 3300

CTTAGTTGTA TTACACTTTT AGACATAGAG GAGACATGTA GGTTTGTTTC TGTATACAGT 3360

AAATTTCTGT GCTTTTCTAT ATCTTATGAA ACTTGAATAG TTGGCTCTGT TGCCAGGTGA 3420

AAGTTTTGCT AGGTTTTTTA GGAAATTAGG ATGAGTACAT TTAAGACACA GGGAAATTTT 3480

ATCTTGAATA GTAAAAGACA TTGTTAAGCT ATCGATTCCT TTCAGAGTTT ATTTGGAAAA 3540

TCAGAGAGAT GTTTTACTGG CTCCTTTGAC ACCAAGTCAC ATCTTCTCCT AATTTATTGT 3600

GAAGAATGTT GACATTAACT TATTTCTCTG AAGACCTGTC TACCTTAGGG GGCTGTTCTG 3660

CATCAAGTTG CCTTTTTAGG GGATGTACAA CTTATTATCT GTCTCTGAAG CAAATATGAA 3720

TATTTGGATG GTGGGTGTAT TAATTCATTT TAACACTGCT GATAAAGACA TGCCCCAAAC 3780

TGGGGAACAA AAAGAGGTTT AATTGGACTT TACAGTTCCA CATGACTGGG GAGTCCTCAG 3840

AATCATGGTG TGAGACGAAA GGCACTTCTT AGGTGGCGGT GGCAAGAGAA AAATGAGGCA 3900

GAAGCAAAAG TGGAAACCCC TGATAAGACC GTCAGATCTC GCGAGACGTA TTCACTATCA 3960

CAAGAATAGG ACGGGAAAGA CTGGCCTCCA TAATTCAATT ACCTCCCACT GGGTGCCTCA 4020

CACAGCACAT GGGAATTCTG GGAAAAACAA TTCAATGGGA GGCTTCGATG CAGACATAGC 4080

CAAACCATAT CAGTAGGCTT TTGTTAAATC ATGGATTTTT TTTGGAACCA AATTTAATCA 4140

CAATTTTCTT TTATCTTTGA GTGTCTCCCA AAATAGCAGT AGATGGGAAT TGTGAAATTC 4200

TGTTTCTCAG AGCTGAGAAT AATCTTAATT TTTCAGGTGA GCAGAATGCT TATCTTTGCC 4260

TCCGAGCATA AGTTTTACAA GAGGGTATGT AGGGAGCTGT ACCTTATTTT AGAGTTTTAA 4320

CTTTTAAGAG ACAAACTTTT AGTTAGCTAA AATACAAATT ATTCTTTCAC ACCTTCGTCT 4380

TCACATGGAT ATTGGCGGCT CTTAATGCTG TTATGTTTAA ATTCCAAAGA ATGGTGACAT 4440

TTGAGTCACT AAAATTTATT GATATTGTAA AGATAAAGTC TATCTGGCTT GAAGTCCCAT 4500

TTGTGAAGTG AATTAAAGTC TTTCTGGCCT AAAATAATGT TCTTTAAAAA ATGTTTATTA 4560

ATTCTGTGTA ATTTTTTTTT CTTTAG 4586

<210> 54
<211> 2127
<212> DNA
<213> Artificial

<220>
<223> CDS

<400> 54

GTTAGTTTGA GCCCTGTCTG CTTTCTAAGA TTTGGTTATT GACCATTTTC CAATTTCCTA 60

TTCTTTCATT ATTAATGCCT TAATTCACCC ATGAATAATT TTTTATCAAT TGTATACTCA 120

GTCCTGTTGT GAGTCTATAG AGGACCTAGC AATAAGATGT ATAAGTGGAA GATCTTCTTT 180

CCTTAGATTT CTTTAATATA ATACAAGACA CAGTAACTAA TAACACCAGA CAGTGTAGAG 240

TAAAACACAA AAGTGTCTTA TTGCCAACTG TTCTTTCAAG ATTTCAGGGA GTGGTGACGT 300

GGCGGCGGGG GGAAGCTCAG TGATGATGGG AATAATTGTC AAAGGACTTT ATGAAGAGGG 360

TTGACCTGAG GTAAGTTCTG AAGGGTGACT CAGATTTGCC AAGATTAATA GAGTTCCACA 420

TGTTCATAAA GCAGGACAAA AACCACTGTA ACTTTTGTAA GCTCTATAAA ACATCCTTAT 480

CCTGGAAAGG AAGTTGACTG CATTTAGCTC CTTTGATCTC CCTGAGACTG GTAGGAATAT 540

CATTGAGTTT TAATTAAAAG CCCAGTAGGC TGAATCTCAT CATCTTATGC ATAACCTTTG 600

GCAAGTTGAT TTGAAAAGCT ACCTCCAAGG TCCCTCTCAG TCCTAAAACC TTATGATATG 660

ATAACGTTGA CCCAAAAGGA CCCCATTTCT TTTCTGATGA TGGTATATCA AGAAGACCCT 720

ATATGTACAC ATAAGTAATT TCCCACTCAT AGCCAGGCTT CTTAAATGCC AACTACTTTT 780

CCTTTAACAT TTCAGTGAAG TCTGCTTTAT TCATAAACTT GATTGTGATT TATACTCAAC 840

AAGTTATATC TCTGTGGCCT CTTCCTGAGT CATGTTTTTC AGATGCACCT TGTTTGGCTT 900

GAATTTAGAA GCATTTCGTA AATACATTTC AGAAGCCATC TTAATCTCTG TGTCTTCCAG 960

ATCGCTTTAC AGTTTCTAAC TAGGCATAAC AGCATTTTAA ATCTTAGGGA CCATTAGTGG 1020

GGTTAAATAA TTATTACCAG TAAATACTAG GTAAAATAAA GGGTGCTATT TTTGCTGAAA 1080

GGTATGTGTG CGTGTGTTCC CAGAAAAATT CTGCTTGTAT ATGTATTCAG TAGTTATCTC 1140

TAGCAGGACT GTAATTGATT TCTATTCTCT TTATAATTTT TTAAACTTGC TTCATTTTCA 1200

CAAAGAATAT GTATATAATT ATATATATAT TTGTGATCAA GATAAAAACA GTTGTTACAA 1260

AAAGCTTACA TGGTGATAAT TTGTATAATG CTTCTGGATT GAACATATAT TGCTCCCTAA 1320

TAATAGAAAG ACTGAAGTAA ACCTCGTTGG CGGGAAAAAA ATGTAGAATG CCAGGAACAG 1380

TTTATGTGAG TCTGTAGTAT GGGTTTTACA CCCCTTCATT CTATTTTCTT CCAGGTGTTC 1440

TTAATGGGAG TTTTACTGTC CTCTAGGGAA ATAGTTAAGG GCAAGTTTGG GATAATCAGT 1500

GACTGGGGAT GTGTAGGACA GGTGGGGGAC AGTCATAGAT ATCGAATGGG CCCAGGCCAA 1560

GGTTGCTAAA CTTCCTGCAC TGAAAGGTGT ATCCCCGGCC GGGCGAAGTG GTTCATTCCT 1620

GTAATCCTAA CACTTTGGGA GCCTGAGGCA AGTGGATCAC TTGAGGCCAG GAGTTCGAGA 1680

CCAGCCTGGC CAACATGGTG AAACCCCATC TCTACTGAAA ATACAAAAAT TAGCTGGGCG 1740

TGGTGGCAGG TGCCTGCAGT TCCAGCTACT TTGGAGGCTG AGGCAGGAGA ATCACTTGAA 1800

CCTGGGAGGT GGAGGTTGCA GTGAGCCAAG ACTGCATCAC TGCATTCCAT CCTGGGTGAA 1860

AGAGCGAGAC TCTGTCTCAA AAAAAATATA TATATATAAA AATAAAAGGT GTAGCTCCCA 1920

CAAGAAAAGT TTTTTTTTTT TCATTCAAAC TGGTAATACC ACCACCTTTG AAAAGGAAGT 1980

ATGGGATCTC TTGGATTAAT TTGGGAAGTG TATAGTTTCT GTTCAGAGTG TTTTATATTT 2040

ACATGTTAGT GAAATTATAG AGACATTTTA TCCCCTTGTG ACTTGACAAG ACCTTTAAAT 2100

TATGTTATTT CTCATTACCT TTTTTAG 2127

<210> 55
<211> 716
<212> DNA
<213> Artificial

<220>
<223> CDS

<400> 55

GTAATTGTGC TAAAGGTAAG GTTTAACATT GTTATTCTGC TTCCATGTTT GAAGTTTAAC 60

TAAATGGAGT CATTTCTTAC TAACTAAGAA AGATGAGGAA AAGATTTATG ACTTTAGACT 120

GGAGGCATGG ATATGGCTGT CCAATTTTTC TGGTCAACCA ACTGATTTCT GAGCCCTTCT 180

CAGTAAGATA GAAATTTTAG AATGGTATCT TTATTATATT GGACTACTGA TGCTTCCCTA 240

TCTGCAAATC TTTAGGTTTC CCTTGTAAAC TGGAAATTAA ATAGAAGTGT AGTGATTCTT 300

CAACATATTG AGAATAAGGA CAGGAGATAT CACTGTTATG GGCGGAAACC TGGGCTAGGA 360

ATTGTTTGCT GTCAGGAATT GGAACTAAGT AGGTGTGGAC TAGTAAGCCA ATTACATACC 420

TCTTAGCATT GGTCTGTTTT GTTCCAACAT AGAGGAAAAA AAAGGGTGTT AGTCTTAAAT 480

GATATTACAG TTCCTTATGT GCCAATTTCA TTTAATAATT TTAGAAAAAT GTGACTGTTA 540

CCATGAAGAA AATTAAGGTA TCTTAGGGAT AATTAAAACA CCAATCATAA GAAGTGTGCA 600

TATCTAAAGT ATTGGGTTGG TTTTGAATTT TATTTTGTGA GTAAAGGAGG AGGAATGGGC 660

CTTTATTTTC TTTGTGTTCC AATTTTGTGG GGGTTTTTTT TTTATTATTT CTACAG 716

<210> 56
<211> 837
<212> DNA
<213> Artificial

<220>
<223> CDS

<400> 56

GTGAGTAAAA TAACCAATGT ATTGATCAGC ACAATGAAAC ATAATTTCCT TCCTGCCCTA 60

TTCTGTGGGT TGTTTCCTTA CTTTATATAT AGTCTCCTTT CATACACAAA AGTTTTTAAT 120

TTTGATGAAA TCCAATATAT TTTTTCACTA GTTGCCTGTG CTTTCGTTTC ATGTATGTAT 180

GTATGTATGT ATTTACCTAT TCGAGATGGA GTCTCGCGCT GTCGCCAAGG CTGGAGTGTA 240

GTGGCACGAT CTCGGCTCAA TGCAACCTCC GCCTCCTGGG TTCAAGCAAT TCTCCTGCCT 300

CAGCCTCCCA AATAGCTGGG ATTATAGGCA TGTGCCACCA TGCCCAGCTC ATTTCTGTCT 360

TTTTCGTAGA GATGGCGTTT AGTCATGTTG GGCAGGATGT TCTCGAACTC CAGACCTCAT 420

GTGGACCACA TTCCTTGTGC TCCCAGAGTG CTAGTATTAC AGCTGTGAGC CACCCATGCC 480

TTGCCTGTTG CCTGTGCCTT TGGCTCTTCA ATAACTTTTA TTTATAACAT CTTTGCCCTG 540

TCATTGTTCT TCTAAGCATC AGTGTGTGTG TATTTTGGTT AGAGATGTAA TCTCTTTTAA 600

GATACATTTT ATATAGGTAA GGTTTTAAAA TTCTCATACA TTCCTTTTAT ATATTTCCTC 660

TACTAAAAAA TGGGCTTTAT TTATATAATT AAGAAAGGTT TTGTAAGAAA ATAAGGACAC 720

ACTTTGCACT CACTCAGAAA ATGAGACTTT CTTTGGTATT TTCACTTAAG TTGCACTGGG 780

TATGAAATGA CTTTTTAGAC TAAGTAGATG TTTCTAATGC TGTACTTTAT TTTATAG 837

<210> 57
<211> 1081
<212> DNA
<213> Artificial

<220>
<223> CDS

<400> 57

GTATTTCCCA AAAAATATGA TACTAATGGG GATATTGTAG ATGAGACCAA CTTCCTGTTG 60

TTAGTCATTT AGTTCAAGTT AACATCTAAG AACATTTATT CTGTTTCTAT TTACATAGTT 120

AATCTCTACT TGTGGAGTAG AAAAGAAATA GAATCTTAAG ACCTATGTAA ATTCTTTTAA 180

TATTGTATGA AAGATCTATT TTGGGTAAAA GCTTCGATTC CTCTCTATCT AATAAAAGTT 240

TTTAGAATAC TGTGATTTTT ATGAGCTGAG AAGGCTTAAA AAAAGTAGCA CACATGTCAC 300

TAGCTAATCT TGTATAGCAG CCTTTCCTTA TCTTATGAAA ATTAAATACC ATTGAAAATG 360

TCAGAAAAAA AATAAAAAGT TGTCTTTCAT GTGTTACAGA GAGGCATAGA GTTAAAAGCA 420

TTGATTTGGT AGCTAGTTCT TCCCCCTCCG GAGATGGAGT CTTGCTCTGT CGCCCAGCGT 480

GGAGTGCAGT GGCGCCATCT CAGCTCACAG AAAGCTCCAC CTCCTGGGTT CACGCCATTC 540

TCCTGCCTCA GCCTGCCGAG TAGCTGGGAC TACAGGCGGC CGCCACCACA CCCGGCTAAT 600

TTTTTGTATT TTTAGCAGAG ACGGGGTCTA CACCGTGTTA GCCAGGATGG TACTCGATCT 660

CCTGACCTCG TGATCCTGCC CGCCACGGCC CCCCAGAGTG CTGGGATTAC AGGCTGGTAG 720

CTATTTCCTT GATACTGACT TAGCATATGA GTTTATGCTT AACTCTCATA AGATAGACGA 780

AACTAATTTT TATAGTGGCA TAGATTAAAT GTTTAGAGAT TTTTATATGA AATTTTAAGA 840

GTAATGTTTT TCAACCTCAA TGTACAAAAC ATGTATTTTA TTAAAAAATT TTGAAATACA 900

TCACAATGTA AACCATTTTA TATAATTCAT AGTTTGAACT ATAATTATTT ACAAAGACAG 960

TAAAAGGAAG AGCGGCTGTT TCAAAATAAT ACTTCAACTT GTAATTTTGA CTAATTTCTT 1020

GTCTAAATAT TTAAAAAATA TTTAATAATT ATTCAGTGAA CCAAGACATT TTTTATTTCA 1080

G 1081

<210> 58
<211> 1455
<212> DNA
<213> Artificial

<220>
<223> CDS

<400> 58

GTGAAAATCA ACATCTTTTT ATGAGAAAAA TACATCAATA TCTAATCTAT TAATAATCCT 60
TTTGGGGATG GGAGGGTGGC AGTTAGGTTT AATATGTTAT AATTACACCT TGTTATGAGA 120
AAAATCTTGG ACTGTAACGT CCCTCTCTAC CCACAAATTG GGAAGGTGCC AAGAGACCAA 180
AGAATGACTC AGACAAGTCC AGCTCGGCAA GTACATAACG TCTATTAAGA CTTACATATG 240
GAGGAGGCAG AGGTGGTGGG GAAAAATAAA AGACTTATAT ACAGGGTACT CCTAGGTAGC 300
AGCAGGACAG CTCTAGAGAT CCTCGCTACC TCCCATCGCT AAGCTGCTTT TAAGCTAATT 360
TTCTGGCTCT TTGCCTACTA TGTGTGTGCA CGATGGGACT GTTTTCCTTG GTAGTTTCTC 420
AGATCTTCTC TGGGATGTTG GGGTTCTCAG GGACACCTGT TCCTTGGCTG GGCACCATGG 480
CCTTGGCTCA CTGCCTAGCC TTCAGGGTTT AGGCAGCAGA CATACACCCT TAAGTAAGGT 540
AGGTGACCTG TCACATTTCA CCCCATGTCA AAGAGGAAAC GAGTCAGATA ATTTGTGGTT 600
GCCCTAAGAT TTTGGTGACA GAGTAAAAAT TCAGTGTTCT TTCTTGATTT CCTTACCAAG 660
TTTCTTTCCC ATAGAGCAGT GGTCCATCCT TTTTGGCACC AAGGACCAGT TTCATGGAAG 720
ACAATTTTTC CATGGACAGG GTTGGGGGTT GGAGAGATTT TGGGATGATT CATCTGCCTT 780
ACATTTATTG CACACTTTAT TTCTATTATT ATTACGTGGT AATATATAAT GAAATAATTA 840
TACAACTCAC CAAAATGTAG AGTCAGTGGG AGCCCTGAGC TTGTTTTCCT GCAACTAGAT 900
GGTCCCATCT GGGGGCGGTG GGAGACAGTG ACAGATCAGC AGGCATTAGA TTCTCATAAG 960
GAGCATGCAA CCTAGATCCC TTATGTGTGC AGTTCACAAT AGGGTTCACA CTCCTGTGAG 1020
AATCTAATGC CACCACTAAT CTGACAGGAG GCCAGCACAG GCGGCAATGT GAGCGATGGG 1080
GAGCAGCTTT ACATACAGAT GAAGCTTTGC TCGGATGCTC ACTGCCTGCT GCTCACCTCC 1140
TGCTATGTTG CCCAGTTCCT AACAGGGTCC ATGGCCCAGG GGTTGGGGAC TCCTGCTTTA 1200
GAGTGGTTGA TATTCAAACT CCTCTCCAAA CCAGTCAATG AAGTTTGACT CATATTTAGT 1260
ATCCAATTAC AAGGTTTTGA ATTTTTTGAC TGCCAAAAGT TTTTTTTTTA ACTTTATTAT 1320
TAAAATGGGA AAGACAGCTG ATTTTATTTA GATGGAATAA TTGTTAAGAT ACTTCTTCTG 1380
CCTTAGATTA CTATTGTATT TGTAATTAAA GTGCTCGTTT GGATACTGGC ATTCTGTGTA 1440
ACCAATTCTT CATAG 1455

<210> 59
<211> 2741
<212> DNA
<213> Artificial

<220>
<223> CDS

<400> 59

GTAAGTTTAA CAATACTAGG AGAATATCTT GGGGCTTACT ATCTGGAAAT TTAAATTTCA 60
TCTAACCCTA CAAGTGAAGT TAATAGGGTA TACATAGAAG AAAATATTCT ATGCATTTTG 120
GTACCCATGG ATCACTTAAA AGAAGGGCCT TTAAAGACTA AGAACACAGG AAAATGCATG 180
ATATAACAGG TATCTTTTAA AAAGGATAGA CTGCTTTATT TATTTATTTA TTTATTGAGA 240

CAGAGTCTTG CTCTGTCACT CAAGCTGGAG TGCAGTGGCC CAATCTCAGC TCACTGCAAC 300
CTCTGCCTGC CGGGTTCAAG CGATTCTCAT GCCTCAGCCT CCTGAGTAGC TGGGACTACA 360
GGCATGCGCC ACCACGCCTA GCTAATTTTT GTATTTTTAG TAGAGAAGGG GTTTTGCCAT 420
ATTGGCCAGG CTGGCCTTGA ACTCCTGACC TCAAGTGATC CGTCTACCTC GTTCTCCCAA 480
AGTGTTGGAA TTACAGGCAT GGGCACCGTG CCCGGCTGAC TGCTGTATAT TTAATATGAT 540
CCCTATTTTT AAAGTGTATG TTTATTTATG AGCATACAAA ATAGTGGAAA TGGAAAAACC 600
AAACTGTTAA GATCATTGTT GGGTGAATGA ATTCCTGGTG ATTTCTGTAA AATTTTTAAG 660
GCAAATACAT ATTACTTTTA AAATCAGAAA TAGAAAAGCC TTCTTAAAGA TAGAGCTGCA 720
TGATCCAGTT AGGTATAGAC AAGCCAGTGA GTTAAGACAA CTGAGTATGT TCCACTTTGT 780
TGAGCTGTGC TACCCTAGTT AATGTGACAT TAGTGCTGGC CCAAGAAATA CAGAAAAGGG 840
CAGTTTTGCT ATCTATCTGG TTTATATTTT TTAGGCAGCT GCTTAGAAGA TCTGCAAGGT 900
GAAAGGTTTT AGTTTACATA TGTGAGATAG AACTACTTTT TTAAAGAGCA ATTCAGTAAA 960
TCCAGAGAGT TCTAAATCCT TGGATCCAAT TAAAAGAATA TTGTTATTTG TAGATCAGTT 1020
TTATAATGTA ATTGATAAGA ACTGGCTATA GAAGGAATAC CAGTTTTAAA GTCAGGATTC 1080
ACTCTAGGCT GGGCATGGTG GCTCATGCCT GTAATCCCAG CACTGTGGGA GACCTAGTGG 1140
GGAGGATCAC TTGAGCCCCG GAGTTCAAGA CCATCCTGGG CAACATAGCA AGATACCATC 1200
TCTACCCCCA ACCCCCCCAA AAAAATCACT CTAAGTGTAT ACTTAATACA CATGGATGAT 1260
CCTTATGAAA AGTCCTCATT TTTGAAAGAT CTGAGAGCTG GTCTTTCTTA GTCTATTTTT 1320
GTAGAATTTT CCGTTCCCTA ATCTACAGAT TAGGAAGACT TGACGTTAAC TTCATTTTCA 1380
ATGTCTTACC ACTTGCTCAG TTTTCCTGAG ATCTCTTGAT ATTTTATGGA GGAGAAATGA 1440
TCATAATCTA TTCTTTGCTG ATTCTGCAGC TTTGTACCAA ATACAAACTC AGTAAGTTTA 1500
TTTACTTTTG TATCATCTGG AAATAGAAAT GTTAAGCCAC AGTTTGTTAG GATTTACTCC 1560
TATCAGTACT TCTTACAAAC TTTGCTATGT ATATTTTAAA TTTTAAAAAC ACTCTGATGC 1620
ACAGCTCTTA GAAGTGGACA CAGAAGAAGG AAGAAATGCT TCTCAAAAAT TCAGACATTG 1680
GTGTGAATAC TTAAAAATAG ACTAAGCCAT AATGGGTTGT GTACCACTGA ATCATACACT 1740
TAAAAATGGT TGAATGGTAA ATTTTATGTT ATATATATAA CCACAATTTT AAAAAACTAG 1800
CCTGTAATAC CAGCATTTTG GGAGGCCAAG GCGGGTGGAT CACCTGAGGT CAGGAGTTCG 1860
AGACCAGCCT GGCCAACATG GTGACCTCAT CTCTACTAGG GAGGCGGAAA GTAGCCATGC 1920
CGTGTGGCAT ATGCCTCTAA TCCCAGTTAC TTGGGAGGCT GAGGCGCAAG AATCACTTAA 1980
ACCCAGGAGG CAGAGGTTGT AGTGAACCGA GATCAGGCTA CTGCACTCCA GCCTGGGTGA 2040
TAGAGTAAGA CTCTGTCAAA AAATAAATAG TAACAATTTG CCCCAAACCA TTGAATTGTA 2100
TAATTTAAGT AGATGAAATT TATGGTATAT AAACTGTTTT AAAAAAATAA ATTATGCTTA 2160
ACTGAATCCA AATCATGCAT GTCCACCTTG CTTAAGAACA TTATTGAGTT TTAATAATTT 2220
TTTATATGTG GAAAAAGACA GAGATCCAAA TTGATAAAAC CGGTGGCGGC GGAATGCTCC 2280

TAGATGACAT ACTACCAATC AGGTCCCCTT ATCAAGTAGT GGCTCTGTAG TAAAATCACA 2340

TCTTACATGA GTGGTAGGTA GAAAGTGGAT ATGATAGAAA ATATTATAGA AAAATATAAT 2400

ATAGAAAAAT AGGGTAATTC CTTAAATTGC CCCTAAATCA TGAAGGTTCT TTAGTAGTGG 2460

AAGACAGAGT CAGGTCTGAT TTGGGAAAGG GGGCGTGGAG AAAGGAACAC TGCAAGACAC 2520

AAAATTCCGT TTTAAAATTT TGCTCTCAGT AGTGTTCACT GAACACGAAT GAAAGTTCAC 2580

TAATGAATAT AGGTAAGATT AGACTTCTGT AATTCTTGTT TGCTTTTTGA ATTATGAAGT 2640

ATTTCAAACA CTGTAGTTAT TTTTTAACAT AAGAGCTTGG ACGGAAGTCA GATCTGAGTC 2700

TCCTTGAGTT AAATGCTTTG TTTGATTTGT TTTGACCCTA G 2741

<210> 60
<211> 197
<212> DNA
<213> Artificial

<220>
<223> CDS

<400> 60

GTAAGGAAGG CAGAGTTGGA TATTGAGTTC CTTCTCTGTG GCATGTATTG AAAAGTTACC 60

CGAGGTTTGG CTAGAGTGAC ATAGGGGACA GAGGAGTGAT GGGGAGAGAG GGTTTGGGAG 120

AGCAGAAATT GTAAACCTCT GCCCGGAGAA CCTCTTATTA TCAACATTTT CTTCATGCTT 180

TTTTTCTCTG TCACTAG 197

<210> 61
<211> 82
<212> DNA
<213> Artificial

<220>
<223> CDS

<400> 61

GTAATTTTTC ACATACCTTA TCAGAGCATG AGCTTGGGAA ATACAAGTGT TAAACAAAGT 60

TTGAAATGTT TTTATCTCCT AG 82

<210> 62
<211> 1079
<212> DNA
<213> Artificial

<220>
<223> CDS

<400> 62

GTGAGTACCA TTTGGAATTG TAAAGGCAAA GATAGGTCTT CATTACTGAG TAACATTTTT 60

TAACCACTGT CTTGAGATAC AGTTTACATG CTCTATAATT CACCTATTTA AAATGCACAA 120

CTAAATGGGT CTTAGTATAT TCACAGATAT GTGCAATACT CACCACAATT TTAGAACATA 180

ATATCCCATT GTATAGTTAT ATGAGAGTAT TTTTATCCAT TCATTAGCTA ATGTATATTT 240

CAGTTGTTTC TACTTGGGGC ATATATGCAT AATACCACTA TTAGCATTTG TGTTTGGGTT 300

TTGGTATAGA CATGTATTTT CATTTCTCTA GGGTATATAC CTAGGAATGG GCTGCTGGGT 360

CATACATTAA CTGTGTTTTA CCTATTTAGG GAATTGCTAG ATTGGTTCTC CAAAGTACTG 420

TACCATCTTA CACTTACACA GCAGTATAAT AAAGATTTTA GTTTCTCCAC TATCTCATTA 480

ACACTTACTA TCTTACTTTG TTTAAATAAC TTATTGAGGA GAAATTCACA TAACATAAAA 540

TTAATTGGGT TTTTCTTTTC TTTTGGGAGA TGTTGTTTCA TTCTTGTCAC CCAGGCTGGA 600

GTGCAGTGGT GCATCTCAGC TCACTGCAAC CTCTGCCTCC CAGGTTCAAG CGATTCTCCT 660

GTCGTAGCCT CCCGAGTAGC TGGGATTACA GCCATGTGCC ACCACGCCTG GCTAATTTGG 720

GGATTTTTAG TAGAGATGGG GTTGACCATG TTGGCCAGGC AGGTCTCAAA CTCCTGACCT 780

CAGGTGATCT GCCCACCTCG GTCTCCCAAA GTGCTGGGAT TACAGGTGTG AACCACCGCA 840

CCTGGCCTCT AAGTCTTGAT TCACATACTA TAGACTCCTA TTGTTTTTAT TGAATTTTAA 900

TAGATATTCT TGAATCGATG TATCTTCATT TGCTATATGC CGTTAATACC ATTTCCAGAG 960

ACTTTAAATA GCTTTTATAT AATTTTCACC CCTTTTACTG GGCAGCAGGT TCACAGAGCT 1020

CCTCACACTA TTATGGTGGT AGTTGCTATG TCTCTCAGAG CACTCTTGCT GTTTGCCAG 1079

<210> 63
<211> 659
<212> DNA
<213> Artificial

<220>
<223> CDS

<400> 63

GTAACTACAT TTTCTCTATG GGCTGCAAAA TAAAGCTTAT AGTCTGTGAT GAATACAAAA 60

AATTACCCAT AGTTGACTCT GTGGCCTTTT TTCCAAGATA AACACCTGGG ACTCTACTTA 120

AGGAAGTTTC TACTTTAATC TTTATTCTTG ATGTCACATG TTGATTAAGG TCTCTTTTCC 180

TCAAAAGGCA ACAATGTTAA ATATTTCATT GCCTTCTTAA TTCAGAAAAA TCACAAGATA 240

GGAATTAAGA AGTTACTTGG TTTCTATGTC ACCTTTCATT CTGGTTTAGT AAACATACTG 300

TAGGTTTAAC CAAGAGAATG TCACATGGAA ATTTAAAACC CACTTCGACT TTATTACCAT 360

TCATCTCTGA GAGGCAAATC GGCCAGATCT GTGTATCTTA CTTAGAATGA CTTGACATTA 420

TGGTTGGGTG CTGTCACTGC AGTGTAGTAC TGCAGGTAGT ACTTGGCATG TGATGCTAGA 480

TGGGCTCTGA TTGAATCCTG GATCTGTTAT AATTTGAGTT ATGTTTCTCA ACCTGTTCTG 540

AGGACAACTA TTGCTATACA GGTTATTGTG AAAACCAAGT AACATATGTG AAGGTCCTAT 600

CACCAAGGGT GTGCTCAACA AATACTAGTT TATGTCCCCT CCTCATTGTT TCTCTAAAG 659

<210> 64
<211> 572
<212> DNA
<213> Artificial

<220>
<223> CDS

<400> 64

GTGGGATCTT TGTGAACTAC AAGACAAAAT TAGGAGCTTT TCTTACTTTT TAGGCCTTGA 60

AGAAGTAACT AAGCATTACT AAATGAAATA ACTATAGAAA CTATGAAAGT GTTTTATAGA 120

TCAGTAAACC ATATTCTAGC TGGCAAAACT GTCCATTACA TAGCTTTGGG GCACAATATT 180

ATGTAACATA TTTCTCCAGG AGAATTAGAG CTTTCAGGGA GGAATCTGCT TGCCTGAGTT 240

CCAGAAAGGT CTGATATGTC AATTGGAACC ATGCTATGGA AATACCATCC CCTGCCTGTC 300

TGCTTTGTAC CACTTAGTAC AGGGCTTAGG TCCTAGAAAA TTTGGTGTAA CTTATTAATG 360

GACACTACTC AGAAAGCCCT TGCTATGGTT ATGGCATAGG GAGAAAGTTA ATATCCTAGC 420

TGAGCTTTGC TTTTTGGTGT GAAGAACAGA GTGCCTATTC ACTGTTATTA GCAAGTAGTG 480

CAGGTAGCTG TTCCCTTTCT CCTACTTTTA AAAAATTAAA ACAGTCACTA TTAGCAGCCT 540

TTGTTCGACA GCCTTGGTTC TCCTGGCTGC AG 572

<210> 65
<211> 901
<212> DNA
<213> Artificial

<220>
<223> CDS

<400> 65

GTAAGTATGA CAGGGATTAT TTCATACTTT TCTCACTCAT GAGTGTTGAG GAATCATTTA 60

TGATTTATAT ATGGACCATT CACCTGGTCC GTATATAAAC TAGTTTTGGC CAGGTGTGGT 120

GGCTCACACC TGTAATCCTA GCACTTTGGG AGGCCGAGGA GGGTAGATCA CTTGAGGTCA 180

GGAGTTCAAG ACCAGCCTGG CCAACGTGGC AAAACCCAGT CTCTACTAAA CATACAAAAA 240

TGAGCTGGGC GTGGTGGCAC ACACTTGTAA TCCCAGCTAC TCTGGGGGCT GAGGCAGGAG 300

AATTGTCTGT ACATGGAAGG CGGCGGCTGT AGTGACCTGA CATTGTGCCA CTGCACTCCA 360

GCTTGGGTGA CAGAACAAGA CTCTGTCTCA TCACTAAGCT AGCTCTACAA ACACTTCTCT 420

TATGTACAAT GAGGAAGTCT GTAATCTACC TAACCAATAT AAATTCTACT GTTGTCAAGC 480

ATCAACCGAG TAAGATTGTA TTTGGAGTCC CCGCAAAGTA TAGTAGTACA AGAGGCAGGC 540

TACATGGGTT CAAATTTCCC AGTACTTAAC AGTGGTGGTA ACCCTGCAAA TCATTAAATT 600

TTCTCTGTAC CTCATTTCCT CATATATAAA ATGGGAATAT AACTAGTTCC TAGCATATGG 660

GGTTGTTGTA AGGATGACAT GACATAATGT ATAAAAATTG CTTACAATAA TAACTGGCAC 720

AAACTAAGCA CTTAAGGTTT GCTATTAGAA TATTTTTCTT TAGGTTAAGT TATTGCTAAA 780

ACATCACTCT GTCATTCATA AAACTACTGG TTTAGCACAC CTCTTCACTC AATAATCATT 840

TTCAGTAAAA ATAATTATAA ATTTTTTTTC TTAGAATTAC TGATTTTTTT TTTTTAAACA 900

G 901

<210> 66
<211> 4220
<212> DNA
<213> Artificial

<220>

<223>  CDS

<400>  66

GTGCGCTCGC GGGCGGAGGG GCGCTTCCGG CCTAGTTGGT GTGAACCGGT GCCTTCCGAG      60

CCGTGTCGCG CGCCTCGAGA GACTCTCGGG CGGGTTGCGG GCTCCCAGCC CCGAGAGGGG     120

TGGGGACTCC CTCTGCGCTA TTCCGAGGCT CTTAGCCGCT CCGAGGGTTA ACCCGCTCTC     180

GCCGGGCTTT CCTGCGGCTT CCGAATGGGG AACGTGTCTT GCCCTAAAGT AGCACAGCAA     240

GGTTGAGATC GCGTTGGGGC CCCGTTGAGG AAAATGGGTG TGTGTGGTCC ATCTGACCCC     300

CCGCCCGTCT TGTTAGTAGA ATGAACTAGT GTCGTTGTCA AGACCACACG GACAAGGGGA     360

GGGGACTTGC CCTTATTTGC ACCGCGATTA ACCGGGTTGT GGCACCTGGG TCTCCACGCG     420

TCTCCGTCTG TTCGCTTCCC CCTGTTAACC AAATTGCCTT TGCCCTGGCG TTGCGGGCGT     480

TTGAGTCAAC GTGCTGATGC GTTTTGGGCT GTGTTTACGT CTGTGTAAAC AAATTAATAC     540

TCATTTCCCC CCAGGCCATA TGAAATGAGC CCACCGCCGA CCCGGATGTT TACACATGCC     600

CCCATTTGTC ACTACGATCA GGACTGTGGC TACCTCCAGG GCTTTTTGGT CACCCCGCGC     660

ATTGCACAGG ACTCCTGTTG TCGTCGCCAT CCGGGTGTGT TAGGTCGCAG CCTTCGGCAC     720

AGGGCTTGCA CCATGACAAA AATGGCCATT CTAGCCAGTG AGTGTCAGCT TTGTATGCAC     780

CTCCCCTTCA TGGGCCAATG GGAAGTGACA CGGAAGTACG GATTGTTTAT CACCTGTTTG     840

ACTGTGTGTG TGGCATTTAA ACCTGAGGCC ATTTGATTTC TCAAGTCGTT TTATAATTAA     900

TTTGTACAAA GAGTCGGGCA AATACGTCCA GGATGCAAAG CCTAACGAAG GTATTATTTA     960

AATATGATGT TTTTGGCTAT GTGTACTGAT GACTGAGGTT ATTTTTAATT TGTATTTGCA    1020

TTAATACAAT TTTAATTCAA TTACTAGTTC CCTCTTTGAA TTGTTAGGTC TGCACAACAT    1080

ACTGTATGGT GGCTTTACAA CCCGACAGAC CTGAAACCGC TGAAAAAGTT CAGTATGGTG    1140

ATCTCTAAAC TGGAGATATT TGTGTTTACC TCACAGAGCT GTTCTGAAGA TTAAATAAGG    1200

CAATAATGTA GTTTCTGGCA CATAAAGCAC CCATATGGAC AGTGTTTTCA AGTTTACTAA    1260

GCTCTTTGTA TATTTACATG ATCTGGCTGA GTAAGCTATG TTCCTATTCA TCTCTCAGTG    1320

CCTTTCTGTA GTCTGGCAAA GAGAAGGACT GGTTGGCTTT TTATGTTGTT TTTTGTTTTT    1380

TGGGTTTTTT TTTGGTAAAT GGCCTTAAAG GCTTCCAAAC AAGCTCTTAT TTTACCCTCA    1440

AGATAATCCT GTAAATCAGA TAGAACAAGC ATTATCGCCA TTTATTTGAG GTATTTCAAC    1500

TCATAGCAGT TAAGTTGTAT GAAGTCTAGT GATACATGAG CAAGTATCAC GTAATAGCTG    1560

GTTAGTAAAT TATTTTTGAA ATCATGTTTG ATTACTCAAT TCTTTTGATT ACTGAGACTT    1620

TAGTTTCAGC TTCTTAGCCC AGTTTATCAG TAAATGATTT ACTCAGTAAA ATATTCATCA    1680

AATATTTCTT GAGCACCTAT TACTTGCTAC ACATTGTTCT AGGTGCTGGA TATAGAGCAC    1740

AAACTGCTCT TGTGGGGCTT ACAGTGAGGT ACGCTGTGAC AATATGGGAT GTCATTCTCA    1800

TGGGAGTGCA AGGGTAAAAT AAAGCTCTTA TGATGTTTAA TACAGAATAC TGGTTATGGA    1860

ATTTTAACTT GATTTCTTGT ATTTTCTGTG CATTTTTAAC CTGTAACTCA TTCTCACAGT    1920

CCTCAGCCAA GAAAATGCAG CCTCTGAGAC TGTTAAGTAA TTTCCCCACT GTGTTATAGC 1980
TACTGTATGG CAGAGCCGGA ATTTGAAACC AGATCTATTT GACCCTAGAA GATGTGACCA 2040
TGAGATGTTA ATTTTGAGGA TAACTTTTTT AGTATTATGG AATTTTCAAC ATATATTTTT 2100
TAGGACCAAA GATAAACTAG GCACAGAGTC TACTCTTTGC ATAAATTATT TAAAAGAGCT 2160
TCGCGCTCCA TTTTGTCATC TAAGCACTGT AAAATTCTCA CAAGACTAAT TCTTCTTTTT 2220
AGGAACGATA TAGTTGTAAA CTTTCTATTT TTTTTCTTTT TTTTTTCTCC CTCCACCATC 2280
CAAGTAGTTG TGAATTTTCT AGAGCCAAAA TAGAACACTA TAGATTATCT TTTAAACCCT 2340
TTATTGAAGC AGAGGATAAT GCTGTGACCG ACTTAACTTT ATGCTTTCTA AGAGATATTG 2400
ATATAGTAGA GAAATGCAGT AGTTATGCAT CTCCGTTTGC TTTTACATCA TAAATCAAGA 2460
ATATTATGAA ACCATCTCCC AGAGATATAT GTGATACACA GATCTTGGCT GTTTTTTTTT 2520
TTTACAAAAG TAACATCTAT GCTATTGATA CATATAAGTG GGTTTGTAAG ACAGTCTATG 2580
TGTAAATGTG AAAAAAGGAA GAATTTCCAG TTCTTCTCAT TTTCATTTAG ACCAGTAATG 2640
AATACAGTGA AGCTAAAGGA CATCTTCCAT CCTTCCTCGC TTTTATAGGG AGAGGAAAGT 2700
TGTATCACTT CTTGAGTAAA AAGAATTGTG ACGATCTTTT ACAAACAATG CCTTAAAAAT 2760
TATTATTTTT GAATGATATG TGGTAGTGGG ATCCACAATA GTCTCATTTG GTTATACAAA 2820
TAAATTTTAT GTATTCATGT ATGTGTTTTG ATTAGGTATA AAATTAGTGG CTGAATATCC 2880
ATTCAAGCTT AATTTTGTAT TTCTATCACT TTTGTAGATT TTGAGCAAGA TTAAAAATAT 2940
AAACAATAGG CCAGGCGCAG GGGCTCACGC CTGTAATCCC AGCACTTTGG GAGGTCTAGG 3000
TGGGCGAGTC ACGAGGTCAG GAGATCAAGA CCATCCTGGC TAACACATTG AAACCCAGTC 3060
TGCTACTAAA AATACAAAAA ATTAGCTGAG CGTGGTGGTG GGCACCTGTA GTCCCAGCTA 3120
CTCAGGAGGC TGAGGCAGGA GAATGGTGTG AACCTGGGAG GCAGAGCTTG GAGTGAGCCA 3180
AGATGGAGCC ACTGTACTCC AGCCTGGGTG ACACAGTGAG ACTCCATCTC AAAAAAAATA 3240
AAAAATAAAT AAAAATAAAC AATAATATTG TTTGCATTAC TATGGCTATA TAGCAAATTG 3300
CCTTAAAACT TAGGGGCAGA AAGCAATTTG TTTTGGTCAC AGGTTCTGTG AGTAAGGAAT 3360
TCAGGCTGGG GACAGTGTGG ATGTCATGTT TCTGCGTCAA AATGACTGGT ACCTCACCTG 3420
GAAGACTTGA GCAACTAGGT ACTGGCACAG CTGGAGCTCG TTGGGCATCT CTGTATGTTT 3480
GTTCCATGTG GTCTCACCAG CATGGTGATC CAGGGTAGGT AAATTGTTAC ATGCTGGTTC 3540
AGGACTCCGA AGGCACATGT CCTAAGAGAG AGAACCAAGT GGAATCTATA GTGCGTTGTA 3600
TAATCTTTTA GAATTACATA GTTTCAGTTG TACCTGTGCA ATTATTGATA GAGACAGTTA 3660
ATCAGTGTGA GGGAACACAG ACCCTTGCCC AGGTCCAAGG TGAGGGAACC CTCTGTACCT 3720
GTCAGTGGAA TAATGTTAAT GTCACATTAT AAGAAGAGCC TGACGGGGCT GGGTAGAGTG 3780
GCTCACACCT GTAATCCCAG CACTTTGGAA GACCAAGGCG GATGGATCAC TTGAGGCCAG 3840
GAGTTCAAGA CCAGCCTGGG CGACATGACA AAACCCTGTC TCGACCAAGA AAACATAGAA 3900
TTAGCCAGGT ATGGTGGCGC ACTTCTGTAG TCCCAGCTAC TTGGGAGACT GAGGTAGGAG 3960

GAGTGCTTGA ACCTGGGAGG TGGAGGTTTC AGTGAGCCAA GATTGCGCCA CTGCACTCCA 4020

GCCTGGGTGA CAGAGCAAGA TTCCATCTCC GAGAGAAAAA AAAAAAAAAA AAAAAAAGAG 4080

CGTATGAGAT AGGGTCATCA TTGAAACTAA GTTTCCCACA AAAATATAAA CAACACTTTC 4140

AATTTAAACA TACTTTTAAA AATATTGAAA TATTTATATG TAGCTTTTTA ACTGAAAATC 4200

AATTTTCTTT TCTTTTACAG 4220

<210> 67
<211> 3507
<212> DNA
<213> Artificial

<220>
<223> CDS

<400> 67

GTAACTATGT TAGAGTTTGA CAAGTAGAGT ATGGCTAATG TAAGCTCATA AATCATAGTG 60

ATAGTAAGAA TTATCTCTGC TCATCATTTC CTGAGCATTT GTACCTGTGG ACTGGCGAAA 120

TTAGATGCTA AAACTAGCAT CTAATGATTT TCCTCCTCTA TATCACAGTT AATATCCATT 180

ATATTTTACT TCTTTGGTGA AAATATTTAA ATTTTAATGT TTTAGGCACT TGTATGGCAG 240

AATTTATTTT TAAAGTTTAG GACATTGTGT AATATTGGGA GAAATGAAGG ATATTGAGAA 300

ACTTTAGGAG ATACTCCAAG TTGAAAAGGT AAATAAAATA TTATTTGCTA TTATACTTAG 360

CAAATATGTG CACAGGACTT GTGGTCTTAA TATAAATGGA ACATGTAAGT ATTTCTCAGT 420

TTCCTGTTTG GAGGATAAAT GACATGATTA TAATCCATTT TAGAAAGGGT CAAATATGTT 480

TAAAAGAAGA GGCAGAAATT GCTTTATCTG TTGTGTAATT AAATTGATTA CATTTATTTT 540

TTGTGCCTTT TAGGTGAATT TTCTTACATG GCTTATTAAA GATAAGTGGA AAAATGATGT 600

TTAGCATTTT GGGGGAAATT ACCACTGTCA AAATTTATGG AGTTAATGGT TAAAAAATCA 660

CTTACTAAAT AAAAAAATTA ACTGGGTGTG GTTGTGCATA CCTGCAGGCC TAGCTACTTG 720

GGAGGCTGAG ATGGGAGGAT CACTTGAGCC CTGAATGATG GAGCAGCACT GCACTCCAGC 780

CTGGGCCACA GAGCAAGACC TTGTCTCCAA AAAAAAAAAA AAAAAAGAAG GTTACTATTA 840

AAATAATTAG CAGGCTGGGG GCGGTGGCTC ACACTTGTAA TCCCAGTAAT CCCAGCACTT 900

TGGAGGCCAA GGTGTGTGGA TCACTTGAGG TCAAGAATTG GAGATCAGCC TGGCCAATAT 960

GGTGAAACCC CGTCTCAACT AAAAATACAA AAATTAGCCG AGTGTGGTGA CATGCGCCTG 1020

TAATCTTAGC TACTCAGGAA GCTGAGTCAG GAAAATCACT TGAGCCCAGG AGGCACAGGT 1080

TGCAGTGAGC ACTATTGCAC TCCAGCCTGG GTGACAAGAG CGAGACTCCA TCTCAAAACA 1140

AATAAATAAA ATAAAATAAT TCACAATGTC ATGTTTTAGC TGACATTGTG AATTTTAGTA 1200

ATCTTTTTTT AACCTTTAAC TCCATCCTGA GTTACATTGA CCAAAGAAAT CAGTATCTAG 1260

AATTATATCA GGGAACTACT AACAGGGTTA ATAAAATGAA TAAAGAACAT GACTTCACAA 1320

AGGTTATAAT TCACATAGCT AATAGATACA GGAAGAGATA TTCACTGTCA CTAATAAAGA 1380

CTTTCAAAGT AGAAAGATAA CATTTCATTC TGTTTTTTTT GAGATGGAGT CTTGCTGTTT 1440

CACCCAGGCC AGGGTGCAGG GGCGTGATCT CAGCTCATTG CAGCGTGTGC GTCCCAGGTT 1500
CAAATGATTC TCCCGCTGTG GCCTCCCAAG TAGCTGGGAT TACAGATGCG CACCACCACA 1560
CCTGGCTAAT TTTTTGTATT TTTAGTAGAG ACGGGTTTCA CCATGTTGGC CAGGCTGGTT 1620
TCCAACTCCT GACCTCAGGT GATCCACCCG CCTTGGACTC CCAAAGTGCT GGCATTACAG 1680
GTGTGAGCCA CCATGCCTGG CCAACATTTT ATTCTTATCA TTGGGAAAAT TTGAAGTCTG 1740
GTATACCAAG TTTGGTCACT GTACAGGGAA ACAGGAACTC TATTTTTTTT ATTTTTCAGT 1800
TCTTTTTTTT TTTTTTTTTT TTTTTTTGAG ATGGAGTCTC ACTCTGCTGC CCAGGCTGGA 1860
GTGCAGTAGC TCAATCTCTA CTCACTGCAA CCTCCACTTC CCAGGTTCAG GTGATTCTCA 1920
TGCTTCAGCC TCCCGGAGTA GCTGGGATAA AGGCACATAC CACTATACCT GACTAATTTT 1980
TGTATTTTTT GTGGAGACCA GGTTTCACCG TGTTGACCAG GCTAGTCTCG AACTCCTGAC 2040
CTCAAGTGAT CTACCTGCCT CGGTCTCCCA AAGTGCTGGG ATTACAGGCA TGAGCCACTG 2100
CGCTCAGGCA GGAACTCTAT ATTGCTGGTG TACATTGGTG AGAGTCAAAA TTGACACAAC 2160
TACTTTACTA GCAAATTTGG TGGTATTTAG TAATATTGAA GGTGCACATT CTCTTACTGT 2220
ACTTCTTGGA GTAGTCCCCA AAGAAACTCC TGCACACATG TATAAGGATG TTTTCATTAC 2280
AACATGTTTT GTTATCATGG AATATTAGAA ACAACCTAAA TTTCCATTGG TTGGGGAGTG 2340
AATGCAAAAA GTCATTGTAT GTTCATATGA AAGAATGTTT TTAGCAATTA AAATGAATAT 2400
ATCTTACATA TCAACATTAA TGTCAGAAAC ATTATTGAGT GTGAAAAAGC AAGTTGCAGA 2460
ATACCACTGA AGTATGATAG CATTTATATA AAATGTAAAA ACACGTAATA AGATATTGCT 2520
TATTGTTTAC ACATACATGT GTATGTGTAG TAAGTGTGAA AACATAGGAA GGATTAAGAC 2580
CAACTTTGGA ATGGTTTTTA TCTTTGGGGT AGAAGGGTAA GGATGGGATT AGGGAGGAGT 2640
ATAAAATGGT AATTTTGACT GTTTCTTTTT CTTTTTCTTT TTCTTTTTTG AGACAGAGTC 2700
TCGCATTGTC GCCAGGCTGG AGTGCAGTGG CGTGATCTCG GCTCACTGCA ACCTCCGCCT 2760
CCCAGGTTTA AGTGATTTTC CTGCCTCAGC CTCCTGAGTA GCTGGGATTA CAGGTGCCCG 2820
CCACCACGCC CAGCTAATTT TTTGTATTTT TAGTAGAGAT CGGGTTTTAC CATGTTGGCC 2880
ATGCTGGTTT CAAACTCCTG ACCTTGTGAA TCTCCCACCT CGGCCTCCCA AAGTGCTGGG 2940
ATTACAGGTG TGAGCTACTG CGCCTAGCCT TGACTGCTTT TATAGTGTTG CTAGTTTAAA 3000
AAAAAATCTG AAGTGGCAGG AGGAGGTGGC TCACACCTGT AATCACAGTG TTCTAGGAAG 3060
CCAAAGTAGG AGGATCACTC AAGCCCAGGA GTCTGCGGTG AGCTGTGATC TTGCCACTGA 3120
ACTCCAACAT GGGTGATAGA ACGAAACCCT ATCTCTTACA AAAACAAAAA CGACAAAATT 3180
TATTTAATAT ATTAACATTT AAAAAATCTG GCAGTGAACC AACGTGAATG TTGGTTAGGT 3240
TACTCTTGTT AATTTTGGTT TGTATTTTCA AATATTTCAT AGTTAACAAA TACTTTAGGT 3300
AACCTAAACA AAATGGATTA GGAGGATCAG AGGAATATAC CAATCTGTAA GAAATTAAGC 3360
TAGTCAGAGA CATGAGTTGT GATTTTATTT CACTGTCTAA AAGTAATATA ATTTAATGCG 3420
ATAATATTGA TTTACTTTTG AATACTTACT TTTGTATACT TTAGCCTTAT GTTAATTATG 3480

AAATATCTTG TTTGTCTTTA ATACCAG 3507

<210> 68
<211> 9837
<212> DNA
<213> Artificial

<220>
<223> CDS

<400> 68

GTGAGCCTAA CATCAATCTT GGCCTTTACT AACCTCAAAA TGCTTCAGAT GCTAGAAACA 60

GGGTTTGTGC TAAGCTTAGG CACTCATTAG AGTGATGAGA GCTGCCAGGG AGCAGTGATC 120

AGTCAGTCCT CATGAAGCAA AACCCAGGGT TGTTTTGTTT TTTGCCTTTT TTGAGGGGGA 180

GGGGGTGGAA TTTAAGGGTG GGAAACAGGG CAAGGGATTT TGATTCTTTT TATTCCCTCT 240

CCTATTTGTA CATTTTGGTG TAAACCTGAA ATTGATTTCT TACCAAAGGC CTGTTTCTGG 300

GACAGGCAGT GTCCTCAGGA GTCTGGCTAA TGGGAGAAGT TGACATTTTT GACATTGCAG 360

TTCAATAGTC ATATTAGCAC AGATGTATGT GGCAACAGCC ACCTCATTCT AAGAAGGGGA 420

AGGAAGCTTG AGTCAGGCCT TAATGTTGAA AAGTCAGGGA GCTGTTGAGG TATGGAAGGG 480

CACTCAGCAG GAAGCAGGTT AAGGGGAAGA AAACAGTGTC CTTGAGGCAG ACAGTGATTC 540

AAAGCTTAAT TACGGGCATC ATGCTATGTT AGCGAGTGGA ACTGGATTGT GACGGCCCTT 600

ACATAATGAG ATTTTTATTG ATAAAGGTTG CTTAGAGGCT GGGCGTTGTG GCTCACACCT 660

GTACTCCCAA CACTTTGGGA GGCCACAGTG GGCAGATCAC CTGAGGTCAG GAGTTCATGA 720

CCAGGCTAGT CAACACGGTG TAAACCTCAT CTCTATTAAA AATACAAAAA TTAGCTGGGT 780

GTGGTGGAAT GCACCTGTAA TCCCAGCTAC TCGGGAGGCT AAGGCAGGAA AATAGCTTGA 840

ACCCAGGAGG TGGAGGTTGC AGTGAGCAGA GCATTGCGCC ATTGCACTCC AGCCTGGGTG 900

ACAAAAGCGA AACTCACTGT CTCAAAAAAA AAAAAAAACC GGTTGCTTAG AAATACACAT 960

TTTTTTTTGG CCTGAACTCT TCAAAAAAAG GTCAGTATGG TAAGAGGACG GGGAAGGTTT 1020

CGTAGAGGAG ACTAGGGAGA CACGACATCC AAATGCAATG CATGATTCTT GACCCTGCAT 1080

AGGAAATCGT CGTTATAAAG GACATTTTGA GGAAAATTTG AATGTGGGCT TTAGTGTATT 1140

TTTTTTTTTA AAGTTTCTTT GGTGTTGATG ATGTCTAGCA GATTATGTAG GAGACTGTGC 1200

TGAAAAGTAT TCAGAGGTAA AGTGTCCCAG TGTCTGCAGC TTACTTTCAA ACGGGTTGGT 1260

TGCAATATAT TTAGGTAGGG AGAGAGTGAA AGTAACTCTT AGACATTAAT GATTGATAAG 1320

TGGCTGTTCA GTGTACTATT TTTTTCAACT CTTTGTAGGC TTGCAATCTT TTAAAAAGTT 1380

GAGGAAAACA GTCCGGGTGC AGTGCCTCAC GCCTGTAATC CCAACATTTT GGCAGGCTGG 1440

GATGGGAAAA TTGCTTGAGG CCAGAATTTG GAAAACGGCT CAGGCAACAT AAAACCCCAT 1500

CCCTACAACA AATAAAAATT AGCTGAGCAT GGTGCCATGC ACCTGTAGTT GTATCTACTC 1560

AGGAGGCTGA GCCCAAAATT TCAAGGCTGC GGTGAGCTAT GGTCGTGCCA CCACACTCCA 1620

GCCTGGGCAA TAAATTGAGA AACCCTGTCT GTTTGGAAAA AAAAGTTGAG GAAAACAATT 1680

AAACAATAAC AGCAAAAATC TGTTATAAAA TGTAATAATG GGCCAGGTGT GGTGGCTCAT 1740
GCCTGTAATC CCACCACTTT GGGAGGCCGA AATGGGTGGA TCACCTGAGG TCAGGAGTTC 1800
AAAATCAGCT TGGCCAACAT GGTGAAACCC CATCTCTGCT AAAATTACAA AAAAATTAGC 1860
TGGGTGCGGT GGCGCACACC TGTAATCCCA GATACTCAGG AGGCTGAGGC AGGAGAATCG 1920
CTTGAACCCA GGAGGCGGAG GTTGCAGTGA GCCGAGATCG TGCCACTACA CTCCAGCCTG 1980
GGCAACAGAG CCAGACTCTG TCTCAAAAAA AAAAAAAAGT TTAATTCACG CAGAGCCAGC 2040
TGAACGGCAG ACAGGAGTTT GGTTATTCAA ATCAGCCTAC CAGAAAATTC GGAGACTGGG 2100
GTTTTTAAAG AATGACTTGG CGGGTAGGGG GCCAGGGATT GGCGAATGCT AATTTGTCAG 2160
GTGGGAGGTG AAATCACAGG GGGTTGAAGT GGGCTCTTGC TGTCTTCTGT TACTGAGTGG 2220
AATTGCAGAA CTTGTTGAGC CAGATTATGG TCTGAGTGGC GCCAGCTAGT GCATTGGAAT 2280
GCGCGGTCTG AAAAGTATCT CCAGCACCAA TCTTAGGTTT TACAATAGTG ATGTTATCCC 2340
TGAGAGCAAT TGGGGAGGTC AGGAATCTTA TAGCCTCTGG CTGCAAGCCT CCTAAATCAT 2400
AATTTCTAAT CTTGTGGCTA ATTTGTTAGT TCTACAAAGG CAGACTGATC CCCAGGCAAG 2460
AATGGGGTTT GTTTTTGGAA AGGACTGTTA CAATCTTTGT TTCAAAGTGA AATTAGAAAT 2520
TAAATTCCTC CTGTAGTTAG TTAGGTCTTC GCCCAGGAAT GAACAAGGGC AGCTCGGAAG 2580
TGAGAAGCGT GGAGTCATTT AGGTCAGATC CCTTGCACTG TCATAACTTT CTCACTGTTA 2640
GGATTTTTGC AAAGGCAGTT TCGTGAACGT ACAGAGACAG GCCCTTGCTA TTATCCCTAT 2700
TTTTTAGATA AGGATATCCA GGCGATGAGG AAGTTTTACT TCTGGGAACA GCCTGGATAC 2760
GAAACCTTCA CACGTCAGTG TCTTTTGGGA CATTTTCTCG TCAGTACAGC CCTGTTGAAT 2820
GTTCTCACGG TGGGGAGGTA CGTGTTTAAA ATGCGGGGAA GGTGCTTTTA TTTCACCCCT 2880
GGTGAAACTA GGGGAGCTAA TTTTTTTAAA CATGATTTTT GGCCCCCTTG AACCGCCGGC 2940
CTGGACTACG TTTCCCAGCA GCCCGTGCTC AAGACTACGG GTGCCTGCAG GCGGTCAGAG 3000
TCGTTTGCGG CGGCGCAGGC GCGGTGCGGG CGGCGGACGG GCGGGCGCTT CGCCGTTTGA 3060
ATGGCTGCGG GCCCGGGCCC TCACCTCACC TGAGGTCGGC CGCCCAGGGG TGCGCTATGC 3120
CGTCGGGAGG TGACCAGTCG CCACCGCCCC CGCCTCCCCC TCCGGCGGCG GCAGCCTCGG 3180
ATGAGGAGGA GGAGGACGAC GGCGAGGCGG AAGACGCCGC GCCGCCTGCC GAGTCGCCCA 3240
CCCCTCAAAG CCGAATTCTG CAGATATCCA TCACACTGGC GGCCGCTCGA GCATGCATCT 3300
AGAGGGCCCA ATTCGCCCTA TAGTGAGTCG TATTACAATT CACTGGCCGT CGTTTTACAA 3360
CGTCGTGACT GGGAAAACCC TGGCGTTACC CAACTTAATC GCCTTGCAGC ACATCCCCCT 3420
TTCGCCAGCT GGCGTAATAG CGAAGAGGCC CGCACCGATC GCCCTTCCCA ACAGTTGCGC 3480
AGCCTGAATG GCGAATGGAC GCGCCCTGTA GCGGCGCATT AAGCGCGGCG GGTGTGGTGG 3540
TTACGCGAGC GTGACCGCTA CACTTGCCAG CGCCCTAGCG CCCGCTCCTT TCGCTTTCTT 3600
CCCTTCCTTT CTCGCCACGT TCGCCGGCTT TCCCCGTCAA GCTCTAAATC GGGGGCTCCC 3660
TTTAGGGTTC CGATTTAGTG CTTTACGGCA CCTCGACCCC AAAAAACTTG ATTAGGGTGA 3720

TGGTTCACGT ATTGGGCCAT CGCCCTGATA GACGGTTTTT CGCCCTTTGA CGTTGGGAGT 3780
CCACGTTCTT TAATAGTGGA CTCTTGTTCC AAACTGGAAC AACACTCAAC CCTATCTCGG 3840
TCTATTCTTT TGATTTATAA GGGATTTTGC CGATTTCGGC CTATTGGTTA AAAAATGAGC 3900
TGATTTAACA AAAATTTAAC GCGAATTTTA ACAAAATTCA GGGCGCAAGG GCTGCTAAAG 3960
GAAGCGGAAC ACGTAGAAAG CCAGTCCGCA GAAACGGTGC TGACCCCGGA TGAATGTCAG 4020
CTACTGGGCT ATCTGGACAA GGGAAAACGC AAGCGCAAAG AGAAAGCAGG TAGCTTGCAG 4080
TGGGCTTACA TGGCGATAGC TAGACTGGGC GGTTTTATGG ACAGCAAGCG AACCGGAATT 4140
GCCAGCTGGG GCGCCCTCTG GTAAGGTTGG GAAGCCCTGC AAAGTAAACT GGATGGCTTT 4200
CTTGCCGCCA AGGATCTGAT GGCGCAGGGG ATCAAGATCT GATCAAGAGA CAGGATGAGG 4260
ATCGTTTCGC ATGATTGAAC AAGATGGATT GCACGCAGGT TCTCCGGCCG CTTGGGTGGA 4320
GAGGCTATTC GGCTATGACT GGGCACAACA GACAATCGGC TGCTCTGATG CCGCCGTGTT 4380
CCGGCTGTCA GCGCAGGGGC GCCCGGTTCT TTTTGTCAAG ACCGACCTGT CCGGTGCCCT 4440
GAATGAACTG CAGGACGAGG CAGCGCGGCT ATCGTGGCTG GCCACGACGG GCGTTCCTTG 4500
CGCAGCTGTG CTCGACGTTG TCACTGAAGC GGGAAGGGAC TGGCTGCTAT TGGGCGAAGT 4560
GCCGGGGCAG GATCTCCTGT CATCCCACCT TGCTCCTGCC GAGAAAGTAT CCATCATGGC 4620
TGATGCAATG CGGCGGCTGC ATACGCTTGA TCCGGCTACC TGCCCATTCG ACCACCAAGC 4680
GAAACATCGC ATCGAGCGAG CACGTACTCG GATGGAAGCC GGTCTTGTCG ATCAGGATGA 4740
TCTGGACGAA GAGCATCAGG GGCTCGCGCC AGCCGAAACT GTTCGCCAGG CTCAAGGCGC 4800
GCATGCCCGA CGGCGAAGGA TCTCGTCGTG ACCCATGGCG AATGCCTGCT TGCCGAATAT 4860
CATGGGTGGA AAAATGGCCG CTTTTCTGGG ATTCATCGAA CTGGTGGCCG GGCTGGGTGT 4920
GGCGGACGCT ATCAGGACAT AGCGTTGGCT ACCCGTGATA TTGCTGAAGA GCTTGGCGGC 4980
GAATGGGCTG ACCGCTTCCT CGTGCTTTAC GGTATCGCCG CTCCCGATTC GCAGCGCATC 5040
GCCTTCTATC GCCTTCTTGA CGAGTTCTTC TGAATTGAAA AAGGAAGAGT ATGAGTATTC 5100
AACATTTCCG TGTCGCCCTT ATTCCCTTTT TTGCGGCATT TTGCCTTCCT GTTTTTGCTC 5160
ACCCAGAAAC GCTGGTGAAA GTAAAAGATG CTGAAGATCA GTTGGGTGCA CGAGTGGGTT 5220
ACATCGAACT GGATCTCAAC AGCGGTAAGA TCCTTGAGAG TTTTCGCCCC GAAGAACGTT 5280
TTCCAATGAT GAGCACTTTT AAAGTTCTGC TATGTGGCGC GGTATTATCC CGTATTGACG 5340
CCGGGCAAGA GCAACTCGGT CGCCGCATAC ACTATTCTCA GAATGACTTG GTTGAGTACT 5400
CACCAGTCAC AGAAAAAGCA TCTTACGGAT GGCATGACAG TAAGAAGAAT TATGCAGTGC 5460
TGCCATAACC ATGAGTGATA ACACTGCGGC CAACTTACTT CTGACAACGA TCGGAGGACC 5520
GAAGGAGCTA ACCGCTTTTT TGCACAACAT GGGGGATCAT GTAACTCGCC TTGATCGTTG 5580
GGAACCGGAG CTGAATGAAG CCATACCAAA CGACGAGCGT GACACCACGA TGCCTGTAGC 5640
AATGGCAACA ACGTTGCGCA AACTATTAAC TGGCGAACTA CTTACTCTAG CTTCCCGGCA 5700
ACAATTAATA GACTGGATGG AGGCGGATAA AGTTGCAGGA CCACTTCTGC GCTCGGCCCT 5760

TCCGGCTGGC TGGTTTATTG CTGATAAATC TGGAGCCGGT GAGCGTGGGT CTCGCGGTAT 5820
CATTGCAGCA CTGGGGCCAG ATGGTAAGCC CTCCCGTATC GTAGTTATCT ACACCGACGG 5880
GGAGTCAGGC AACTATGGAT GAACGAAATA GACAGATCGC TGAGATAGGT GCCTCACTGA 5940
TTAAGCATTG GTAACTGTCA GACCAAGTTT ACTCATATAT ACTTTAGATT GATTTAAAAC 6000
TTCATTTTTA ATTTAAAAGG ATCTAGGTGA AGATCCTTTT TGATAATCTC ATGACCAAAA 6060
TCCCTTAACG TGAGTATTCG TTCCACTGCA GCGTCAGACC CCGTAGAAAA GATCAAAGGA 6120
TCTTCTTGAG ATCCTTTTTT TCTGCGCGTA ATCTGCTGCT TGCAAACAAA AAAACCACCG 6180
CTACCAGCGG TGGTTTGTTT GCCGGATCAA GAGCTACCAA CTCTTTTTCC GAAGGTAACT 6240
GGCTTCAGCA GAGCGCAGAT ACCAAATACT GTTCTTCTAG TGTAGCCGTA CGTAGGCCAC 6300
CACTTCAAGA ACCTCTGTAC CACCGCCTAC ATACCTCGCT CTGCTAATCC TGTTACCAGT 6360
GGCTGCCGCC AGTGGCGATA AGTCGTGTCT TACCGGGTTG GACTCAAGAC GATAGTTACC 6420
GGATAAGGCG CAGCGGTCGG GCTGAACGGG GGGTTCGTGC ACACAGCCCA GCTTGGAGCG 6480
AACGACCTAC ACCGAACTGA GATACCTACA GCGTGAGCTA TGAGAAAGCG CCACGCTTCC 6540
CGAAGGGAGA AAGGCGGACA GGTATCCGGT AAGCGGCAGG GTCGGAACAG GAGAGCGCAC 6600
GAGGGAGCTT CCAGGGGGAA ACGCCTGGTA TCTTTATAGT CCTGTCGGGT TTCGCCACCT 6660
CTGACTTGAG CGTCGATTTT TGTGATGCTC GTCAGGGGGG CGGAGCCTAT GGAAAAACGC 6720
CAGCAACGCG GCCTTTTTAC GGTTCCTGGC CTTTTGCTGG CCTTTTGCTC ACATGTTCTT 6780
TCCTGCGTTA TCCCCTGATT CTGTGGATAA CCGTATTACC GCCTTTGAGT GAGCTGATAC 6840
CGCTCGCCGC AGCCGAACGA CCGAGCGCAG CGAGTCAGTG AGCGAGGAAG CGGAAGAGCG 6900
CCCAATACGC AAACCGCCTC TCCCCGCGCG TTGGCCGATT CATTAATGCA GCTGGCACGA 6960
CAGGTTTCCC GACTGGAAAG CGGGCAGTGA GCGCAACGCA ATTAATGTGA GTTAGCTCAC 7020
TCATTAGGCA CCCAGGCTTT ACACTTTATG CTTCCGGCTC GTATGTTGTG TGGAATTGTG 7080
AGCGGATAAC AATTTCACAC AGGAAACAGC TATGACCATG ATTACGCCAA GCTATTTAGG 7140
TGACACTATA GAATACTCAA GCTATGCATC AAGCTTGGTA CCGAGCTCGG ATCCACTAGT 7200
AACGGCCGCC AGTGTGCTGG AATTCGGCTT AAAGGTAGGC GGATCTGGGT CGACTCTAGG 7260
CCTAAATGGC CATTTAGGTG ACACTATAGA AGAGCTCGAG GACAACAGAA AATCTTAGTG 7320
AACATGTTTT ATGGGAAAAT TTTATATACA ACATCAAAAG CACAATCCGT AAAATACTGT 7380
TAAAATGGAT TTTATCAAAA TGAATAATTT CTGCTATTTG AGACACTGTT AAGAGAATTA 7440
AAAAACCAGC CATAGACTAT TAGAAAATCT GTACACGTTC CATATCTGAT GAAGCATTTG 7500
TATATCTACA GTATCTAAAG AATTCTCAAA ATTCAGTAGG AAAACCACCA AATGTAAAAG 7560
TGGGCAAAAG ATTTGAACAC ACTTCACCCA TTACATGCCT GTTAGAATGG CTAAAATCCA 7620
AAAAGTGACA AATCGTAAGT TCTGACAACA ATGTGGAACA ATTTTACATA TTGCTGGTGT 7680
GAACGCAAAA TGGCATCGCC ACTGTGGAAA GTTGTTTCTT AAACATACCA TTATACAACC 7740
AGCAATCTCA TTCCTAGGTA TTTACACAAA TGAAATGGAA ACTTATGTTT AGACAAAATC 7800

ACGTACATGA CTGTTTATAG TGACTTTCTT CCTAATTGCC AAAAAGTGGG AAACAACCCA 7860
AACGTCCTTC AGCTGGTGAA TGCATATAAA TAAGCTGTGG TGCATCCAGA CAATCGACTG 7920
CTACTTTGCA ATAAAAAGGA ACTGATATAT TCAATGTAGA TAAATCTCAA ATGCATCAAT 7980
GCTTAAGTGA AAGACACTGG ATTCAGTAGG CTACTTATGA TTCCATTTCT GTGACATTGT 8040
GGAAAAGGCA AAACTATTGG ACAAGAACAT CAGTGGTGGT TTGGGATAGG CTGACAAGGG 8100
AGTATGAGGG ATTTTTTCAG AGGAACAGTT TTATCCGACT GTAGGTATTT CTAGCACAGA 8160
ATTGGGAGTC TGTCCAGTAA AATGATAGCG ATTATTAGAC TCTTGGTTGG AGAAAGATTT 8220
GTCATCTTGA CGTAATAGGT GATAGCTGAA ACTTACGGGG AGAATATTAC AAAGCAAGGA 8280
GGGGGAGAAT ATTCCCAAGC AAGAAGTAGC TTATGTCTAG AACCAATCTA TAACGTACTA 8340
ACATTTAGAC TACTATGAGG GGATAATTAT CAAATACTAT ACAAGATCAG TTAAGATGAA 8400
GACTGATCAT TAGTGATACT TGACAGAGCA GTGTCAGTGC ACTGGTATGA CTTGTTGAGA 8460
AATAAATTAT GGTAGCATTG CTTATACACA ATTAACGATG TATACAGTAA GACAGTGTGA 8520
GAAATATTCA AGCAAATGGG AGACCGCAGA GATACCAAAT GCAGACCAGA CTCTTAGGAG 8580
GCAAGAAGGG GGCTAGAAAA AGAATTGAAG GAAAGCTTTC TTCAGATGCT TAAGATTTTG 8640
TGGCCAGGTG CAGTGGCTCA TGCCTGTTCC CAGCACATTA GGAGGCCCAA AGCAGGAGGA 8700
TTGCTTGAGC CCAGGAATTC AAGACCAGCT TGGACAACAT AGTGCAACCC CATTTCTATT 8760
GGTAATTAAA AAAAAAAAAA AATGAAAAAC ACTTGTGAAG GTACATCTGT TGATAATAAA 8820
GAACACTGAT TTTCATTAAA ACCCCCAAAA CATTTATTAC TTTAAAGAAT AAAAATAACA 8880
AGTGTCATGA TAAAATATGT CTGGGATTTG TTTTAAAATA ATCTGGGGAA TGGAAGTGAA 8940
TCAGAGTATA AATCAAGCAA GGCTGGCCAA ACATGCTGAA GTAGAGGAAT AGGTATGTGA 9000
GGATGCATTA TGCTTCTCTA CTTTTGTATG TTTACAATTT CCCTATAATA GATATCTGTG 9060
AATTTGCTTA GTATGCTTTC TGTAAGCAAA CATGGATGAA GCAGCACATG AAAAAGAATT 9120
TTAACCAACA AACTAGCAGA AATAATGTGA CAGACGACTT TTAGAGGCTT TGGAGAAACT 9180
GAATGCTAAA GGTGCTGTAC AGCCAGCCCC AGTCTTTCTG ACATTCTGGC AGTGTCTTTC 9240
TCAATTGCAG CTCCTCATCT GAGCCACTGT CCAGAAAATA ATTTGAGTAA CTTTAATCCT 9300
CAATTCTCCC AAGGATAGTA CCATTCTAGA TCTTACTAAT TTATTAGCTA CAATGGATAC 9360
CTTAGGGGGG GATTAAGGCC TACTTTTCTA GTGAAATCCC AGTTGAGAAT GGCTGCTAAA 9420
AACTGAGTAA CATTAGACTG AAAGAAAGGG AATATTGTAT AAAGTTGTAC TTTGAAAAAG 9480
AGAAAAAGAT GTGTCTAAGT GACTATCAGA TAGCAATGTA ATGCTCCCTA ATTGTAAAAA 9540
AAATCACAAA TTTGTGAACT CACGAATTAT AGACATGTAT AATTGACCTA CAGGTCAAGA 9600
AGTGCCTGTG GAAGAGCTTG TTAAAAATAG AACTACTCAG CCCCTTCTCA AATAGCCATC 9660
GGCCTCAGCC ATCTGGAAAG TAAAGTTGGC AGGTTATGTA ACTTAGTGTT TCTTTTACTC 9720
TGTAGATGTG TTCAAACTCT TCCAGGTAAA CTGCTTAACT CATTTGAGAT TCTTTGACTA 9780
ATACTGAGCT ATGTGCATTT GCATTTTGAA AAATTATGTA TCTTTTTCCC ACCATAG 9837

<210>  69
<211>  23
<212>  DNA
<213>  Artificial

<220>
<223>  CDS

<400> 69

CTCTGTAACT GCTTATAATC CTG                                                23

<210>  70
<211>  21
<212>  DNA
<213>  Artificial

<220>
<223>  CDS

<400> 70

CTAGGAAACC TGTACAACTC C                                                  21

<210>  71
<211>  21
<212>  DNA
<213>  Artificial

<220>
<223>  CDS

<400> 71

GGCTTATTGT GTGCTGATAT C                                                  21

<210>  72
<211>  21
<212>  DNA
<213>  Artificial

<220>
<223>  CDS

<400> 72

AGAGATCCTT AAGTCGTCAT G                                                  21

<210>  73
<211>  21
<212>  DNA
<213>  Artificial

<220>
<223>  CDS

<400> 73

CAGTTTCTGT GAGAGAGTAC A                                                  21

<210>  74
<211>  21
<212>  DNA
<213>  Artificial

<220>
<223>  CDS

<400> 74

GGCTTACCTG CTCCTGTATT T                                                21

<210>  75
<211>  21
<212>  DNA
<213>  Artificial

<220>
<223>  CDS

<400> 75

GAGGAGGAAT GGGCCTTTAT T                                                21

<210>  76
<211>  21
<212>  DNA
<213>  Artificial

<220>
<223>  CDS

<400> 76

AACCCACAGA ATAGGGCAGG A                                                21

<210>  77
<211>  22
<212>  DNA
<213>  Artificial

<220>
<223>  CDS

<400> 77

GGATACTGGC ATTCTGTGTA AC                                               22

<210>  78
<211>  21
<212>  DNA
<213>  Artificial

<220>
<223>  CDS

<400> 78

ATTTCCAGAT AGTAAGCCCC A                                                21

<210>  79
<211>  21
<212>  DNA
<213>  Artificial

<220>
<223>  CDS

<400> 79

AGCTTGGACG GAAGTCAGAT C                                                21

<210>  80
<211>  21
<212>  DNA
<213>  Artificial

<220>
<223>  CDS

<400> 80

TCTAGCCAAA CCTCGGGTAA C                                                21

<210>  81
<211>  18
<212>  DNA
<213>  Artificial

<220>
<223>  CDS

<400> 81

AATTGTAAAC CTCTGCCC                                                    18

<210>  82
<211>  18
<212>  DNA
<213>  Artificial

<220>
<223>  CDS

<400> 82

ATTTCCCAAG CTCATGCT                                                    18

<210>  83
<211>  18
<212>  DNA
<213>  Artificial

<220>
<223>  CDS

<400> 83

AGCATGAGCT TGGGAAAT                                                    18

<210>  84
<211>  18
<212>  DNA
<213>  Artificial

<220>
<223>  CDS

<400> 84

TGAAGACCTA TCTTTGCC                                                    18

<210>  85
<211>  21
<212>  DNA
<213>  Artificial

<220>
<223>  CDS

<400> 85

GTTCACAGAG CTCCTCACAC T                                                21

<210>  86
<211>  21
<212>  DNA
<213>  Artificial

<220>
<223>  CDS

<400> 86

AGGCCACAGA GTCAACTATG G                                                21

<210>  87
<211>  21
<212>  DNA
<213>  Artificial

<220>
<223>  CDS

<400> 87

AGGTCCTATC ACCAAGGGTG T                                                21

<210>  88
<211>  21
<212>  DNA
<213>  Artificial

<220>
<223>  CDS

<400> 88

GCTTAGTTAC TTCTTCAAGG C                                                21

<210>  89
<211>  21
<212>  DNA
<213>  Artificial

<220>
<223>  CDS

<400> 89

GTAGCTGTTC CCTTTCTCCT A                                                21

<210>  90
<211>  21
<212>  DNA
<213>  Artificial

<220>
<223>  CDS

<400> 90

CCTCAACACT CATGAGAGTG A                                                21

<210>  91
<211>  21
<212>  DNA
<213>  Artificial

<220>
<223>  CDS

<400> 91

TGGTTTAGCA CACCTCTTCA C                                                21

<210> 92
<211> 21
<212> DNA
<213> Artificial

<220>
<223> CDS

<400> 92

GCTTAGCACA AACCCTGTTT C                                                21

<210> 93
<211> 20
<212> DNA
<213> Artificial

<220>
<223> CDS

<400> 93

TTCGCCGTTT GAATTGCTGC                                                  20

<210> 94
<211> 20
<212> DNA
<213> Artificial

<220>
<223> CDS

<400> 94

ACCGGTTCAC ACCAACTAGG                                                  20

<210> 95
<211> 22
<212> DNA
<213> Artificial

<220>
<223> CDS

<400> 95

GAGATAGGGT CATCATTGAA AC                                               22

<210> 96
<211> 21
<212> DNA
<213> Artificial

<220>
<223> CDS

<400> 96

CATTAGCCAT ACTCTACTTG T                                                21

<210> 97
<211> 22
<212> DNA
<213> Artificial

<220>
<223>  CDS

<400> 97

GCTAATTTAA CTCTGTAACT GC                                              22

<210>  98
<211>  23
<212>  DNA
<213>  Artificial

<220>
<223>  CDS

<400> 98

CACTGCAGCA CAGACTAATG TGT                                             23

<210>  99
<211>  23
<212>  DNA
<213>  Artificial

<220>
<223>  CDS

<400> 23

TCTCTCCCTT TAACTGTGGG TTT                                             23

<210>  100
<211>  23
<212>  DNA
<213>  Artificial

<220>
<223>  CDS

<400> 100

GGAGTTGACG AGATTAATAC CTG                                             23

<210>  101
<211>  22
<212>  DNA
<213>  Artificial

<220>
<223>  CDS

<400> 101

CATGACGACT TAAGGATCTC TT                                              22

<210>  102
<211>  21
<212>  DNA
<213>  Artificial

<220>
<223>  CDS

<400> 21

CTCAGTTTCC AGAGTACAAA C                                               21

<210>  103
<211>  22
<212>  DNA
<213>  Artificial

<220>
<223>  CDS

<400> 103

GTGAATTAAA GTCTTTCTGG CC                                                22

<210>  104
<211>  21
<212>  DNA
<213>  Artificial

<220>
<223>  CDS

<400> 104

ATCTTAGAAA GCAGACAGGG C                                                 21

<210>  105
<211>  21
<212>  DNA
<213>  Artificial

<220>
<223>  CDS

<400> 105

GAGACATTTT ATCCCCTTGT G                                                 21

<210>  106
<211>  21
<212>  DNA
<213>  Artificial

<220>
<223>  CDS

<400> 106

TCCATGCCTC CAGTCTAAAG T                                                 21

<210>  107
<211>  20
<212>  DNA
<213>  Artificial

<220>
<223>  CDS

<400> 107

CACTTAAGTT GCACTGGGTA                                                   20

<210>  108
<211>  21
<212>  DNA
<213>  Artificial

<220>
<223>  CDS

<400> 108

CAACAGGAAG TTGGTCTCAT C                                                21

<210> 109
<211> 21
<212> DNA
<213> Artificial

<220>
<223> CDS

<400> 109

TAAAAGGAAG AGCGGCTGTT T                                                21

<210> 110
<211> 21
<212> DNA
<213> Artificial

<220>
<223> CDS

<400> 110

TTAAACCTAA CTGCCACCCT C                                                21

<210> 111
<211> 20
<212> DNA
<213> Artificial

<220>
<223> CDS

<400> 111

CTGAGCTATG TGCATTTGCA                                                  20

<210> 112
<211> 20
<212> DNA
<213> Artificial

<220>
<223> CDS

<400> 112

AAGGCTGCTG CTAAACAGAT                                                  20

<210> 113
<211> 2461
<212> DNA
<213> Artificial

<220>
<223> CDS

<400> 113

TGCCCGCCTT GGCCTCCCAA CGTGTAGGGA TTACAGGCGT GAGTCACCGC GCCTTGCCAA      60

ATTATTTATT ATTATTTTTT GGAGACAGGG TCTCTGTTGC CCAAGCTGTA GTGGTATGGC     120

CACAGTTCAC TGCAGACTCC CCAGGATTAG GCGTTCCTCC CACCTCAGTC TCCCAAGTAG     180

CTAGGATTAC AGGCGTCTAC CACCACTCTG GGTTAATTTT TCTATTTTTT GGAGAGACAG 240
GGTTTCACTA TGTCGCCCAG GCTGGACCTC GAACTCCTGT CTCAAGCAGC CCCCCCACCT 300
CGCCTCCCAA AGTGCTGGAT TTACAGGTGT GATCCACAAC GTCCAGCCTA TATACTTAAG 360
ATACTTCTAA ACCATTTGTG TTCAACTTCT GTTCTTGCCC CATAGTCACC TTGAGACTCA 420
TCACTTAGCC AACTCCAAAA GCATTGCTGA TTACTGTGAA TTTTACTAAG GTTTTCTTAA 480
GAGGGTTCCA TTGTCTCAAA ATTGTTCCTG AAATATCCTG TTACCTGTCT ACCTGATTTT 540
CTCCTATCTT CAGAGTTCCA TTTCCTGTCC TCCCGCCTGT CATTATACCT TCCATAAGCC 600
CCTACTTTTG TCCCAGCACT TTTCCCTCTG TCAGTTTACA TATCCCACCA AGCAAAACAA 660
AAATAGCAAA ACAGTAATGC CTTCTGAATC CTCAAATTGC TCAATCCTCA GATTGCTCCT 720
CAATCTGGAA AATGTTTTAT ATCAAGCCCA TTTATAAATC AAGGATTGGC AATTTAAAAA 780
ATTAAAATAA AGAAAGGAGA ATTGGAAATA AAATGAATTG GCTGGGCACG GTGGCTCACG 840
CCTGTAATCC CAGAACTTTG GGAGGCCGAG GTGGGTGGAT CACTTGAGGT CAGGAGTGCG 900
AGACCAGCCT GGCCAACATG GTGAAACCCT GCCTGTTCTG AAAATCCAAA AATCAGCTGG 960
GTGCGGCGGC GCACACCTGT AATCCCAGAT ACTCAGGAGG CTGAGGCAGG AGAATCGCTT 1020
GATCCCAGGA GGCGGAGGTT GCAGCGAGCC GAGATCGTGC CACTACACTC CAGTCTGGCC 1080
AACAGAGCCA GACTCTGTCT CACAAAAAAA AAAAAGTTTA ATTCACGGAG AGCCAGCTGA 1140
ACGGCAGACA GGAGTTTGGT TATCCAAATC AGCCTACCAG AAATTGGAGA CTGGGGTTTT 1200
TAAAAGAATG ACTTGGCGGG TAGGGGCCCA GGGATTGGCG AATGCTAATT TGTCAGGTGG 1260
GAGGTGAAAT CACAGGGGGT TGAAGTGGGC TCTTGCTGTC TTCTGTTACT GAGTGGAATT 1320
GCAGAACTTG TTGAGCCAGA TTATGGTCTG AGTGGCGCCA GCTAGTGCAT CGGAATGCGC 1380
GGTCTGAAAA GTATCTCCAG CACCAATCTT AGGTTTTACA ATAGTGATGT TATCCCTGAG 1440
AGCAATTGGG GAGGTCAGGA ATCTTATAGC CTCTGGCTGC AAGCCTCCTA AATCATAATT 1500
TCTAATCTTG TGGCTAATTT GTTAGTTCTA CAAAGGCAGA CTGATCCCCA GGCAAGAATG 1560
GGGTTTGTTT TTGGAAAGGA CTGTTACAAT CTTTGTTTCA AAGTGAAATT AGAAATTAAA 1620
TTCCTCCTGT AGTTAGTTAG GTCTTCGCCC AGGAATGAAC AAGGGCAGCT CGGAAGTGAG 1680
AAGCGTGGAG TCATTTAGGT CAGATTCCTT GCACTGTCAT AACTTTCTCA CTGTTAGGAT 1740
TTTTGCAAAG GCAGTTTCGT GAACGTACAG AGACAGGCCC TTGCTATTAT CCCTATTTTT 1800
TAGATAAGGA TATCCAGCCG ATGAGGAAGT TTTACTTCTG GAACAGCCTG GATACGAAAC 1860
CTTCACACGT CAGTGTCTTT TGGACATTTT CTCGTCAGTA CAGCCCTGTT GAATGTTCTC 1920
ACGGTGGGGA GGTACGTGTT TAAAATACGG GGAAGGTGCT TTTATTTCAC CCCTGGTGAA 1980
ACTAGGGGAG CTAATTTTTT TAAACATGAT TTTTGTCCCC CTTGAACCGC CGGCCTGGAC 2040
TACGTTTCCC AGCAGCCCGT GCTCAAGACT ACGGGTGCCT GCAGGCGGTC AGCGTCGTTT 2100
GCGACGGCGC AGACGCGGTG CGGGCGGCGG ACGGGCGGGC GCTTCGCCGT TTGAATTGCT 2160
GCGGGCCCGG GCCCTCACCT CACCTGAGGT CCGGCCGCCC AGGGGTGCGC TATGCCGTCG 2220

GGAGGTGACC AGTCGCCACC GCCCCCGCCT CCCCCTCCGG CGGCGGCAGC CTCGGATGAG 2280

GAGGAGGAGG ACGACGGCGA GGCGGAAGAC GCCGCGCCGT CTGCCGAGTC GCCCACCCCT 2340

CAGATCCAGC AGCGGTTCGA CGAGCTGTGC AGCCGCCTCA ACATGGACGA GGCGGCGCGG 2400

CCCGAGGCCT GGGACAGCTA CCGCAGCATG AGCGAAAGCT ACACGCTGGA GGTGCGCTCG 2460

C 2461

<210> 114
<211> 4853
<212> 24
<213> Artificial

<220>
<223> CDS

<400> 114

ACCTCAGGTG AGGTGAGGGC CCGG 24

<210> 115
<211> 25
<212> DNA
<213> Artificial

<220>
<223> CDS

<400> 115

GTGTGCCATT TATGTGATGG CAAAG 25

<210> 116
<211> 25
<212> DNA
<213> Artificial

<220>
<223> CDS

<400> 116

GTATACCATT TAGCAGCTGT CCGCC 25